(19) Europäisches Patentamt
European Patent Office
Office européen des brevets

(11) EP 1 019 366 B1

(12) EUROPÄISCHE PATENTSCHRIFT

(45) Veröffentlichungstag und Bekanntmachung des Hinweises auf die Patenterteilung:
**13.03.2002 Patentblatt 2002/11**

(21) Anmeldenummer: **96943969.4**

(22) Anmeldetag: **16.12.1996**

(51) Int Cl.⁷: **C07C 251/60**, A01N 37/50

(86) Internationale Anmeldenummer:
**PCT/EP96/05642**

(87) Internationale Veröffentlichungsnummer:
**WO 97/24317 (10.07.1997 Gazette 1997/30)**

(54) **IMINOOXYPHENYLESSIGSÄUREDERIVATE, VERFAHREN UND ZWISCHENPRODUKTE ZU IHRER HERSTELLUNG UND IHRE VERWENDUNG**

IMINO OXYPHENYL ACETIC ACID DERIVATIVES, METHODS AND INTERMEDIATES FOR THEIR PREPARATION AND USE THEREOF

DERIVES D'ACIDE IMINO-OXYPHENYLACETIQUE, PROCEDES ET PRODUITS INTERMEDIAIRES POUR LEUR PREPARATION, ET LEUR UTILISATION

(84) Benannte Vertragsstaaten:
**AT BE CH DE DK ES FR GB GR IE IT LI NL PT SE**

(30) Priorität: **27.12.1995 DE 19548370**
**09.02.1996 DE 19604732**
**04.06.1996 DE 19622332**

(43) Veröffentlichungstag der Anmeldung:
**19.07.2000 Patentblatt 2000/29**

(73) Patentinhaber: **BASF AKTIENGESELLSCHAFT**
**67056 Ludwigshafen (DE)**

(72) Erfinder:
- **GROTE, Thomas**
  **D-67105 Schifferstadt (DE)**
- **SAUTER, Hubert**
  **D-68167 Mannheim (DE)**
- **KIRSTGEN, Reinhard**
  **D-67434 Neustadt (DE)**
- **BAYER, Herbert**
  **D-68159 Mannheim (DE)**
- **MÜLLER, Ruth**
  **D-67159 Friedelsheim (DE)**
- **MÜLLER, Bernd**
  **D-67227 Frankenthal (DE)**
- **OBERDORF, Klaus**
  **D-69117 Heidelberg (DE)**
- **GRAMMENOS, Wassilios**
  **D-67063 Ludwigshafen (DE)**
- **GÖTZ, Norbert**
  **D-67547 Worms (DE)**
- **RACK, Michael**
  **D-69123 Heidelberg (DE)**
- **HARREUS, Albrecht**
  **D-67063 Ludwigshafen (DE)**
- **RÖHL, Franz**
  **D-67105 Schifferstadt (DE)**
- **AMMERMANN, Eberhard**
  **D-64646 Heppenheim (DE)**
- **HARRIES, Volker**
  **D-67227 Frankenthal (DE)**
- **LORENZ, Gisela**
  **D-67434 Hambach (DE)**
- **STRATHMANN, Siegfried**
  **D-67117 Limburgerhof (DE)**

(74) Vertreter: **Fitzner, Uwe, Dr. et al**
**Dres. Fitzner & Münch**
**Rechts- und Patentanwälte**
**Lintorfer Strasse 10**
**40878 Ratingen (DE)**

(56) Entgegenhaltungen:
**EP-A- 0 585 751** **WO-A-96/14305**

- **QUESTEL DARC INFORMATION SERVICE: FILE EURECAS, XP002028873**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 019 366 B1

Printed by Jouve, 75001 PARIS (FR)

## Beschreibung

Die vorliegende Erfindung betrifft 2-Iminooxyphenylessigsäurederivate der allgemeinen Formel I

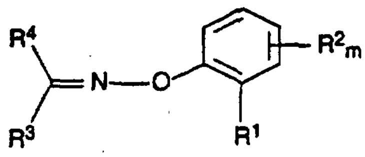

(I)

in der die Substituenten und der Index die folgende Bedeutung haben:

$R^1$ $C(CO_2CH_3)=NOCH_3$ (Ia), C $(CONHCH_3)=NOCH_3$ (Ib), $C(CONH_2)=NOCH_3$ (Ic), $C(CO_2CH_3)=CHOCH_3$ (Id) oder $C(CO_2CH_3)=CHCH_3$ (Ie);

$R^2$ Cyano, Nitro, Halogen, $C_1$-$C_4$-Alkyl, $C_1$-$C_4$-Halogenalkyl oder $C_1$-$C_4$-Alkoxy;

m 0, 1 oder 2, wobei die Reste $R^2$ verschieden sein können, wenn m für 2 steht;

$R^3$ Wasserstoff, Cyano, Hydroxy, Halogen,
$C_1$-$C_6$-Alkyl, $C_1$-$C_6$-Halogenalkyl, $C_1$-$C_6$-Alkoxy-$C_1$-$C_6$-alkyl, $C_1$-$C_6$-Alkoxy, $C_1$-$C_6$-Halogenalkoxy, $C_1$-$C_6$-Alkylthio, Cyclopropyl, $C_2$-$C_6$-Alkenyl,
Aryloxy-$C_1$-$C_6$-alkyl, Benzyl oder Benzyloxy, wobei die aromatischen Ringe in diesen Resten einen bis drei der folgenden Gruppen tragen können: Cyano, Nitro, Halogen, $C_1$-$C_6$-Alkyl, $C_1$-$C_6$-Halogenalkyl, $C_1$-$C_6$-Alkoxy, $C_1$-$C_6$-Halogenalkoxy, oder $C(CH_3)=N-Y-R^a$, wobei

$R^a$ $C_1$-$C_6$-Alkyl und

Y Sauerstoff oder Stickstoff, wobei das Stickstoffatom ein Wasserstoffatom oder eine $C_1$-$C_6$-Alkylgruppe trägt;

$R^4$ Wasserstoff, Cyano,
ggf. subst. Alkyl, Alkenyl, Alkinyl, Cycloalkyl, Heterocyclyl, Aryl und Hetaryl;
ggf. subst. Alkoxy, Alkenyloxy, Alkinyloxy, Cycloalkoxy, Heterocyclyloxy, Aryloxy und Hetaryloxy;
ggf. subst. Arylthio und Hetarylthio;

$$-Q_p-C(R^5)=N-Y^1-R^6$$

oder

$$-Q-O-N=CR^7R^8,$$

wobei

Q eine direkte Bindung, $CH_2$, $CH(CH_3)$, $CH(CH_2CH_3)$ oder 1,1-Cyclopropyl bedeutet;

p 0 oder 1 bedeutet;

$Y^1$ Sauerstoff oder Stickstoff, wobei das Stickstoffatom ein Wasserstoffatom oder eine $C_1$-$C_4$-Alkylgruppe trägt;

$R^5$ eine der für $R^3$ genannten Gruppen, oder

ggf. subst. Cycloalkoxy, Heterocyclyloxy, Aryloxy, Hetaryloxy, Arylthio und Hetarylthio;

$R^6$ ggf. subst. $C_1$-$C_{10}$-Alkyl, $C_3$-$C_6$-Cycloalkyl, $C_2$-$C_{10}$-Alkenyl, $C_2$-$C_{10}$-Alkinyl, $C_1$-$C_{10}$-Alkylcarbonyl, $C_2$-$C_{10}$-Alkenylcarbonyl, $C_2$-$C_{10}$-Alkinylcarbonyl oder $C_1$-$C_{10}$-Alkylsulfonyl;

ggf. subst. Aryl, Hetaryl, Arylcarbonyl, Hetarylcarbonyl, Arylsulfonyl oder Hetarylsulfonyl;

$R^7$,$R^8$ Methyl, Ethyl, Phenyl und Benzyl, wobei die aromatischen Ringe einen bis drei der folgenden Substituenten tragen können: Cyano, Nitro, Halogen, $C_1$-$C_6$-Alkyl, $C_1$-$C_6$-Halogenalkyl, $C_1$-$C_6$-Alkoxy und $C_1$-$C_6$-Halogenalkoxy;

$R^3$ und $R^4$ gemeinsam mit dem Rohlenstoffatom, an das sie gebunden sind, ein vier- bis achtgliedriger Ring, welcher neben Kohlenstoffatomen ein oder zwei Sauerstoff- und/oder Schwefelatome und/oder NH- und/oder N ($C_1$-$C_4$-Alkyl)-Gruppen enthalten kann, und dessen Kohlenstoffatome einen der folgenden Substituenten tragen können: Halogen, $C_1$-$C_6$-Alkyl oder $C_1$-$C_4$-Alkoxyimino; wobei

$R^3$ und $R^4$ nicht gleichzeitig über Heteroatome an das Kohlenstoffatom gebunden sind;

sowie deren Salze.

Außerdem betrifft die Erfindung Verfahren und Zwischenprodukte zur Herstellung der Verbindungen I sowie sie enthaltende Mittel und ihre Verwendung zur Bekämpfung von tierischen Schädlingen und Schadpilzen.

Aus der Literatur sind Phenylessigsäurederivate zur Bekämpfung von tierischen Schädlingen und Schadpilzen bekannt (EP-A 370 629, EP-A 463 488, EP-A 460 575, WO-A 95/21,154, WO-A 95/21,153, WO-A 95/18,789).

Demgegenüber lagen der vorliegenden Erfindung Verbindungen als Aufgabe zugrunde, welche stärker die mitochondriale Atmung hemmen und damit eine verbesserte Wirkung gegen tierische Schädlingen und Schadpilze entfalten.

Demgemäß wurden die eingangs definierten Verbindungen I gefunden. Außerdem wurden Verfahren und Zwischenprodukte zur Herstellung dieser Verbindungen sowie zur Bekämpfung von tierischen Schädlingen und Schadpilzen geeignete Mittel gefunden.

Die Verbindungen I können auf verschiedenen Wegen hergestellt werden, wobei im allgemeinen unerheblich ist, ob zunächst die Gruppe -O-N=$CR^3R^4$ (im folgenden auch als "Seitenkette" bezeichnet) oder der Rest $R^1$ (im folgenden auch als "Pharmakophor" bezeichnet) aufgebaut wird, bzw. an welcher Vorstufe des "Pharmakophors" die "Seitenkette" mit dem Gerüst gekoppelt wird.

1. Bei der Herstellung der Verbindungen I, in denen $R^1$ für $C(CO_2CH_3)=NOCH_3$ (Ia), $C(CO_2CH_3)=CHOCH_3$ (Id) oder
$C(CO_2CH_3)=CHCH_3$ (Ie) bedeutet, geht man beispielsweise so vor, daß man einen Benzoesäureester der Formel II in Gegenwart einer Base mit einem Oxim der Formel III in das entsprechende Derivat der Formel IV überführt, IV zur entsprechenden Carbonsäure IVa verseift und IVa anschließend zunächst zum Chlorid Va und danach zum Cyanid Vb umsetzt, Vb im Wege der Pinner Reaktion in den entsprechenden $\alpha$-Ketoester VI überführt und VI anschließend entweder

a) mit O-Methylhydroxylamin oder dessen Salz (VIIa) in die entsprechende Verbindung Ia [R = $C(CO_2CH_3)=NOCH_3$], oder
b) mit einem Wittig- bzw. Wittig-Horner-Reagens der Formel VIIb in die entsprechende Verbindung Id [R = $C(CO_2CH_3)=CHOCH_3$], oder
b) mit einem Wittig- bzw. Wittig-Horner-Reagens der Formel VIIc in die entsprechende Verbindung Ie [R = $C(CO_2CH_3)=CHCH_3$],

überführt.

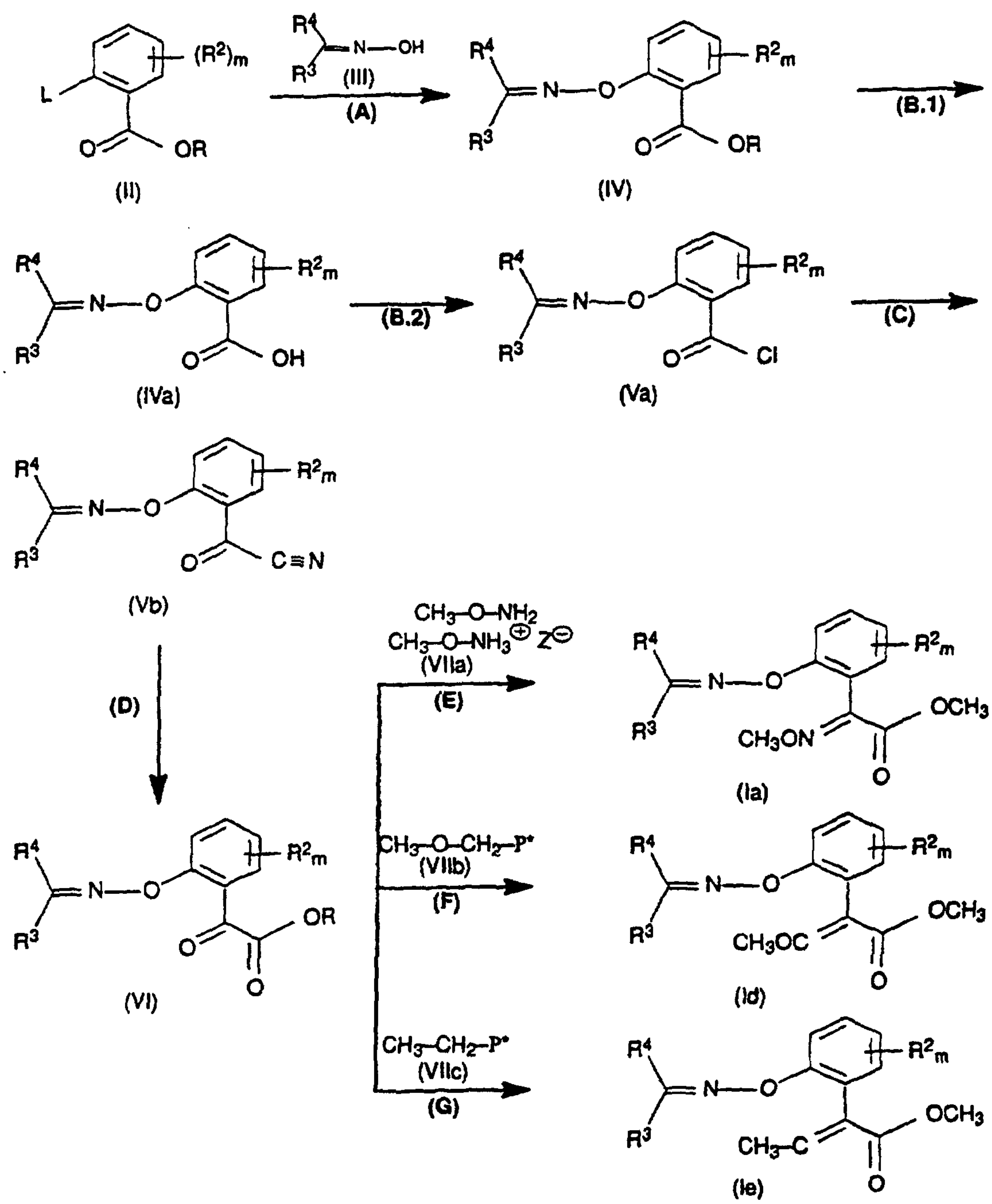


(II) (IV) (IVa) (Va) (Vb) (VI) (Ia) (Id) (Ie)

L in der Formel II steht für eine für die nucleophile aromatische Substitution übliche Abgangsgruppe wie Halogen (z.B: Fluor, Chlor, Brom), Nitro oder Sulfonat.

R in der Formel II steht für eine $C_1$-$C_4$-Alkylgruppe, insbesondere Methyl.

$Z^-$ in der Formel VIIa steht für das Anion einer anorganischen Säure, insbesondere ein Halogenidanion.

P* in den Formeln VIIb und VIIc steht für einen für die Wittig- oder Wittig-Horner-Reaktion geeigneten Phosphonat oder einen Phosphonium-halogenid Rest.

1A. Die Umsetzung des Benzoesäure-esters (II) mit dem Oxim (III) erfolgt üblicherweise bei Temperaturen von -20°C bis 170°C, vorzugsweise 0°C bis 100°C in einem inerten organischen Lösungsmittel in Gegenwart einer Base [J.Heterocycl.Chem. 4, 441 (1967): J.Org.Chem. 49, 180 (1984); Synthesis 1975, 782; J.Heterocycl.Chem.

26, 1293 (1989)].

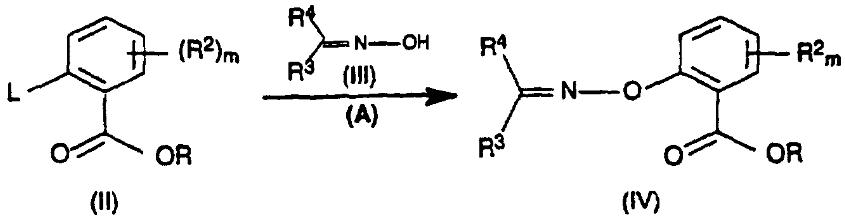


(II) (IV)

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, Dimethylacetamid, 1,3-Dimethylimidazolidin-2-on, 1,3-Dimethyltetrahydro-2(1H)-pyrimidinon und Hexamethylphosphorsäuretriamid, besonders bevorzugt Tetrahydrofuran, Dimethylsulfoxid, Dimethylacetamid, Dimethylformamid und 1,3-Dimethyltetrahydro-2(1H)-pyrimidinon. Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calziumhydrid, Alkalimetall- und Erdalkalimetallcarbonate wie Lithiumcarbonat und Calziumcarbonat, ferner Silbercarbonat, Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliummethanolat, Kaliumethanolat, Kalium- tert.-Butanolat und Dimethoxymagnesium, außerdem organische Basen, z.B. tertiäre Amine wie Trimethylamin, Triethylamin, Triisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydrid, Kaliumcarbonat, Kaliummethanolat, Kaliumethanolat, Kalium-tert.-butanolat und Natriummethylat. Die Basen werden im allgemeinen äquimolar, im Überschuß oder gegebenenfalls als Lösungsmittel verwendet.

Es kann im Hinblick auf die Vollständigkeit der Umsetzung vorteilhaft sein, die Umsetzung in Gegenwart katalytischer Mengen eines Kronenethers wie 18-Krone-6 oder 15-Krone-5 oder eines sonstigen üblichen Phasentransferkatalysators durchzuführen. Als Phasentransferkatalysatoren kommen Ammoniumhalogenide und -tetrafluoroborate wie Benzyltriethylammoniumchlorid, Benzyltributylammoniumchlorid, Tetrabutylammoniumchlorid, Hexadecyl-trimethylammoniumbromid und Tetrabutylammoniumtetrafluoroborat, sowie Phosphoniumhalogenide wie Tetrabutylphosphoniumchlorid und Tetraphenylphosphoniumbromid.

Die Edukte werden im allgemeinen in äquimolaren Mengen miteinander umgesetzt. Es kann für die Ausbeute vorteilhaft sein, III in einem Überschuß bezogen auf II einzusetzen.

Es kann im Hinblick auf die Geschwindigkeit der Umsetzung und deren Vollständigkeit vorteilhaft sein, zunächst die Verbindungen III mit Base zu behandeln und das resultierende Salz mit der Verbindung II umzusetzen.

Die für die Herstellung der Verbindungen I benötigten Ausgangsstoffe II sind in der Literatur bekannt. Die Ausgangsstoffe III sind aus der eingangs zitierten Literatur bekannt oder können nach den dort beschriebenen Methoden erhalten werden (vgl. WO-A 95/21,153).

1B. Die Umsetzung des Esters IV zur entsprechenden Säure IVa erfolgt in an sich bekannter Weise durch saure oder alkalische Verseifung bei Temperaturen von 0°C bis 100°C, vorzugsweise von 0°C bis 50°C, in einem inerten organischen Lösungsmittel oder wäßrig/organischen Lösungsmitteln in Gegenwart einer Base oder einer Säure [basische Verseifung vgl.: J.Org.Chem. 30, 3676 (1965); saure Verseifung vgl.: Chem.Ind. (London) 1964, 193].

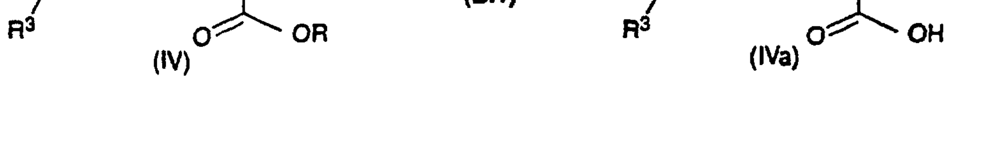


(IV) (IVa)

Die so erhaltene Carbonsäure IVa wird anschließend in an sich bekannter Weise mittels üblicher Chlorierungsmittel bei Temperaturen von 0°C bis 150°C, vorzugsweise 0°C bis 100°C, ggf. in Gegenwart eines inerten organiscgen Lösungsmittels [Houben-Weyl, Ergänzungsband 5, S. 59ff, 225ff und 664ff].

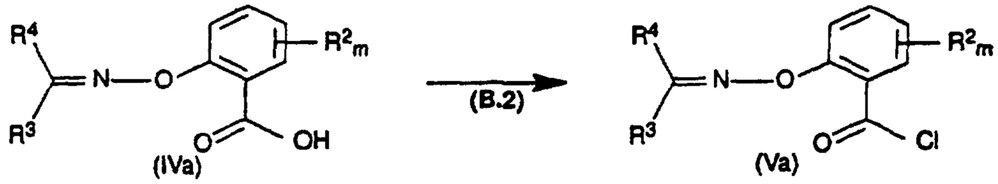


(IVa) (Va)

Als Chlorierungsmittel sind alle hierfür üblichen Reagentien geeignet, insbesondere $SOCl_2$, $(COCl)_2$, $POCl_3$, $AlCl_3$ und $PCl_5$. Die Chlorierungsmittel werden im allgemeinen im Überschuß oder ggf. als Lösungsmittel verwendet.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol sowie halogenierte Kohlenwasserstoffe wie Methylenchlorid, Chloroform und Chlorbenzol. Es können auch Gemische der genannten Lösungsmittel verwendet werden.

Die Edukte werden im allgemeinen in mindestens äquimolaren Mengen miteinander umgesetzt. Es kann für die Ausbeute vorteilhaft sein, das Chlorierungsmittel in einem Überschuß bezogen auf IVa einzusetzen.

1C. Die Umsetzung des Benzoesäurechlorids Va in das entsprechende Cyanid Vb erfolgt in an sich bekannter Weise [DE Anm. Nr. 19 603 990.8; Bull.Chem.Soc.Jpn. 60, 1085 (1987); Synthesis 1983, 636; J.Org.Chem. 43, 2280 (1978); Tetrahedron.Lett. 1974, 2275] bei Temperaturen von 0°C bis 150°C, vorzugsweise 10°C bis 100°C mit einem anorganischen Cyanid in einem inerten organischen Lösungsmittel ggf. in Gegenwart eines Katalysators.

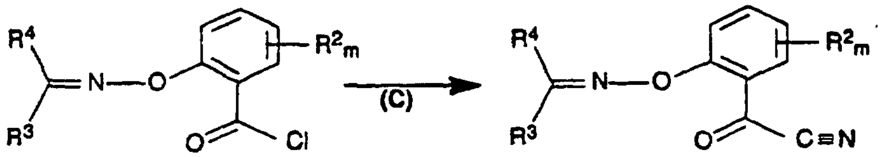


(Va) (Vb)

Als anoganische Cyanide eignen sich Cyanide von Metallen der ersten Hauptgruppe oder der Nebengruppen des Periodensystems, beisielsweise Lithium, Natrium, Kalium, Kupfer und Silber, insbesondere Kupfer, sowie organische Cyanide wie Trimethylsilylcyanid.

Geeignete Lösungsmittel sind aliphatische Kohlenwasserstoffe wie Pentan, Hexan, Cyclohexan und Petrolether, aromatische Rohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert. -Butanol sowie Dimethylsulfoxid und Dimethylformamid, besonders bevorzugt Toluol, Methylenchlorid und Tetrahydrofuran. Es können auch Gemische der genannten Lösungsmittel verwendet werden.

Bei der Umsetzung mit den genannten Cyanid-Verbindungen mit Ausnahme von CuCN empfiehlt sich die Verwendung eines Katalysators. Als Katalysatoren finden übliche Phasentransferkatalysatoren, insbesondere Ammoniumsalze wie Tetrabutylammoniumbromid, Verwendung.

Die Edukte werden im allgemeinen in äquimolaren Mengen miteinander umgesetzt. Es kann für die Ausbeute vorteilhaft sein, das Cyanid in einem Überschuß bezogen auf Va einzusetzen.

1D. Die Umsetzung des Cyanids Vb zum α-Ketoester VI erfolgt in an sich bekannter Weise im Sinne einer Pinner-Reaktion bei Temperaturen von 0°C bis 150°C, vorzugsweise 30°C bis 100°C in einem Alkohol (R-OH) in Gegenwart einer Säure und eines Katalysators [Tetrahedron Lett. 21, 3539 (1980): J.Org.Chem. 47, 2342 (1982)].

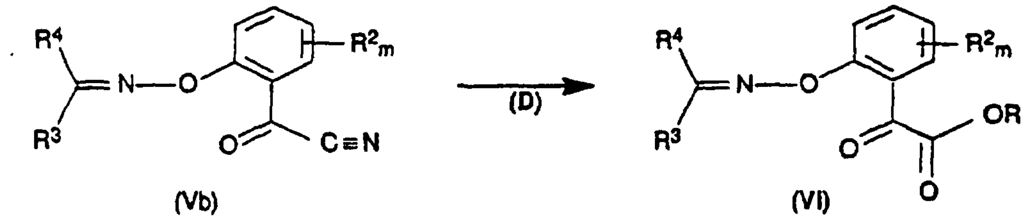


(Vb) (VI)

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung.

Die Säuren werden im allgemeinen mindestens äquimolar, bevorzugt jedoch im Überschuß verwendet.

1E. Die Umsetzung des α-Ketoesters VI mit O-Methylhydroxylamin bzw. dessen Salz (VIIa) zur Verbindung Ia erfolgt in an sich bekannter Weise bei Temperaturen von 0°C bis 100°C, vorzugsweise 20°C bis 70°C in einem inerten organischen Lösungsmittel, ggf. in Gegenwart einer Base [US-A 5,221,762].

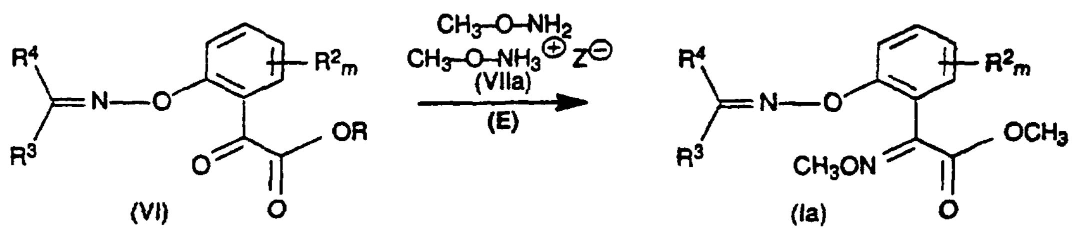


(VI) (Ia)

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert. -Butanol sowie Dimethylsulfoxid und Dimethylformamid, besonders bevorzugt Dimethylformamid oder Alkohole. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kaliumtert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden tertiäre Amine.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, VIIa in einem Überschuß bezogen auf VI einzusetzen.

1F. Die Umsetzung des α-Ketoesters VI mit zur Verbindung Id erfolgt in an sich bekannter Weise im Sinne einer Wittig- bzw. Wittig-Horner-Reaktion bei Temperaturen von 0°C bis 100°C, vorzugsweise 0°C bis 50°C in einem inerten organischen Lösungsmittel in Gegenwart einer Base und eines Katalysators [Tetrahedron 44, 3727 (1988)].

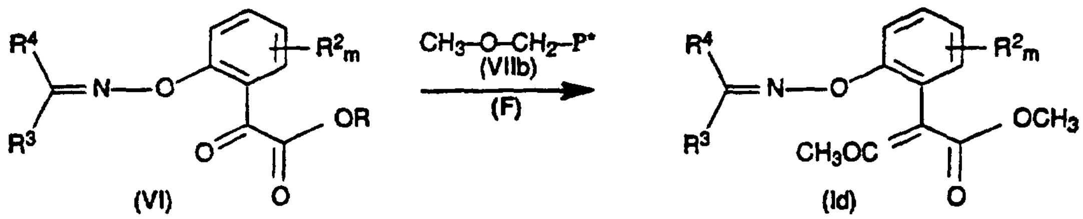


Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert. -Butanol sowie Dimethylsulfoxid und Dimethylformamid, besonders bevorzugt Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Alkoholate.

Die Basen werden im allgemeinen in katalytischen Mengen eingesetzt, sie können aber auch äquimolar, im Überschuß oder gegebenenfalls als Lösungsmittel verwendet werden. Die Edukte werden im allgemeinen in äquimolaren Mengen miteinander umgesetzt. Es kann für die Ausbeute vorteilhaft sein, VIIb in einem Überschuß bezogen auf VI einzusetzen.

1G. Die Umsetzung des α-Ketoesters VI mit zur Verbindung Ie erfolgt in an sich bekannter Weise im Sinne einer Wittig- bzw. Wittig-Horner-Reaktion bei Temperaturen von 0°C bis 100°C, vorzugsweise 0°C bis 50°C in einem inerten organischen Lösungsmittel in Gegenwart einer Base und eines Katalysators [Austr.J.Chem. 34, 2363 (1981); Can.J.Chem. 49, 2143 (1971)].

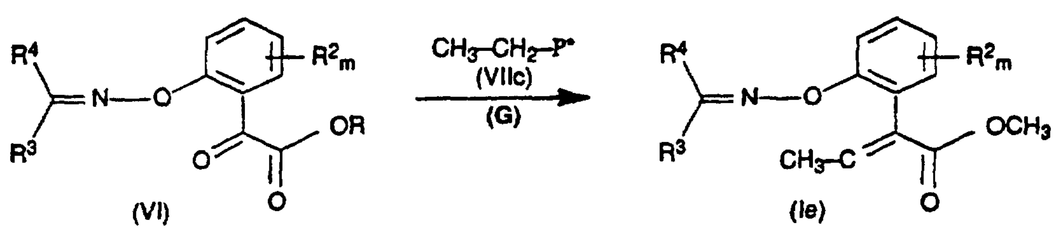


Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert. -Butanol sowie Dimethylsulfoxid und Dimethylformamid, besonders bevorzugt Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide

wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Caiziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Alkoholate.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, VIIc in einem Überschuß bezogen auf VI einzusetzen.

2. Besonders bevorzugt erhält man die Verbindungen Vb auch dadurch, daß man ein Cyanid der Formel Vc wie unter Punkt 1A. beschrieben mit einem Oxim der Formel III umsetzt.

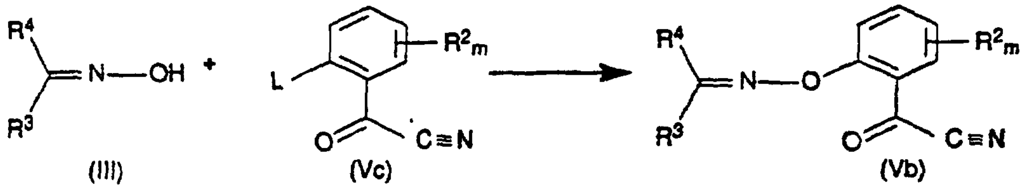

(III) (Vc) (Vb)

3. Die Verbindungen der Formel VI erhält man vorteilhaft auch dadurch, daß man einen α-Ketoester der Formel VIa in die Verbindung VIb überführt und VIb in situ mit einem Keton bzw. Aldehyd IX zu VI umsetzt.

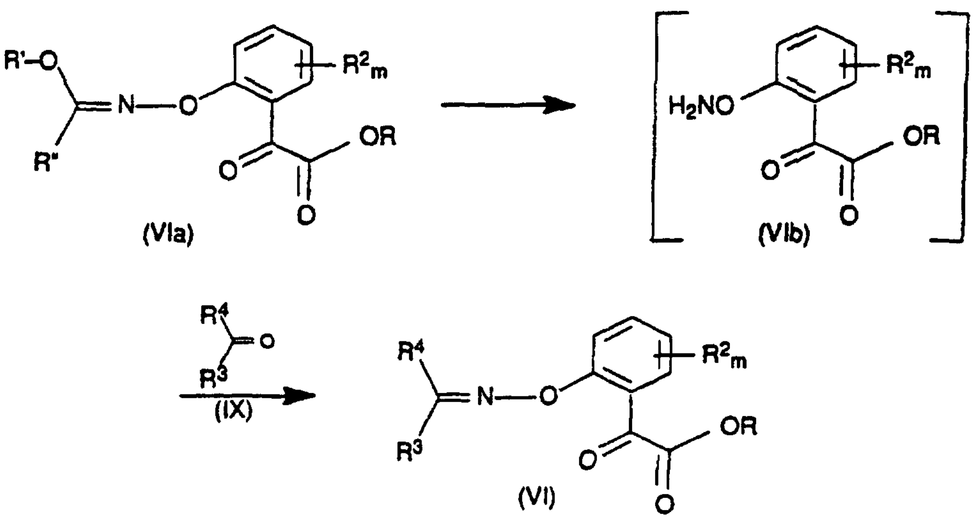

(VIa) (VIb) (IX) (VI)

Die Gruppen R' und R" in der Formel VIa stehen unabhängig voneinander für $C_1$-$C_6$-Alkyl oder Aryl.

Die Freisetzung des Hydroxylamins VIb aus dem Oxim VIa und die weitere Umsetzung der so erhaltenen Reaktionsmischung mit dem Keton IX erfolgt in an sich bekannter Weise bei Temperaturen von 0°C bis 150°C, vorzugsweise 0°C bis 50°C in einem inerten organischen Lösungsmittel in Gegenwart einer Säure bzw. eines Katalysators.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.

-Butanol sowie Dimethylsulfoxid und Dimethylformamid, besonders bevorzugt Alkohole. Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, IX in einem Überschuß bezogen auf VIIb einzusetzen.

4. Verbindungen I, in denen $R^1$ für $C(CONHCH_3)=NOCH_3$ (Ib) oder $C(CONH_2)=NOCH_3$ (Ic) steht, erhält man beispielsweise dadurch, daß man eine Verbindung der Formel Ia in an sich bekannter Weise mit Methylamin oder dessen Salz (XIII) oder mit Ammoniak oder einem Ammoniumsalz umsetzt, z.B.

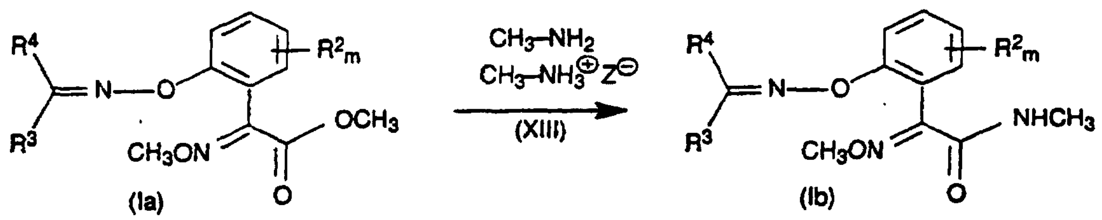


$Z^-$ in der Formel XIII steht für das Anion einer anorganischen Säure, insbesondere für ein Halogenidanion wie Chlor und Brom.

Diese Umsetzung erfolgt üblicherweise bei Temperaturen von 0°C bis 150°C, vorzugsweise 0°C bis 70°C in einem inerten organischen Lösungsmittel, ggf. in Gegenwart einer Base. Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Tetrahydrofuran. Es können auch Gemische der genannten Lösungsmittel verwendet werden.

Für den Fall, daß als Ausgangsstoffe die Ammoniumsalze eingesetzt werden, wird die Umsetzung vorteilhaft in Gegenwart einer Base durchgeführt. Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden tertiäre Amine bzw. Kaliumcarbonat.

Die Basen werden mindestens äquimolar, im Überschuß oder gegebenenfalls als Lösungsmittel verwendet.

Die Edukte werden im allgemeinen in äquimolaren Mengen miteinander umgesetzt. Es kann für die Ausbeute vorteilhaft sein, XIII bzw. Ammoniak in einem Überschuß bezogen auf Ia einzusetzen.

5. Nach einem besonders bevorzugten Verfahren erhält man die Verbindungen Ib vorteilhaft dadurch, daß man ein a-Ketoamid der Formel VIc zunächst gemäß den unter 1A. beschriebenen Bedingungen mit einem Oxim der Formel III in das entsprechende Amid der Formel VId überführt und VId anschließend gemäß den unter 4. beschriebenen Bedingungen mit O-Methylhydroxylamin oder dessen Salz (VIIa) umsetzt.

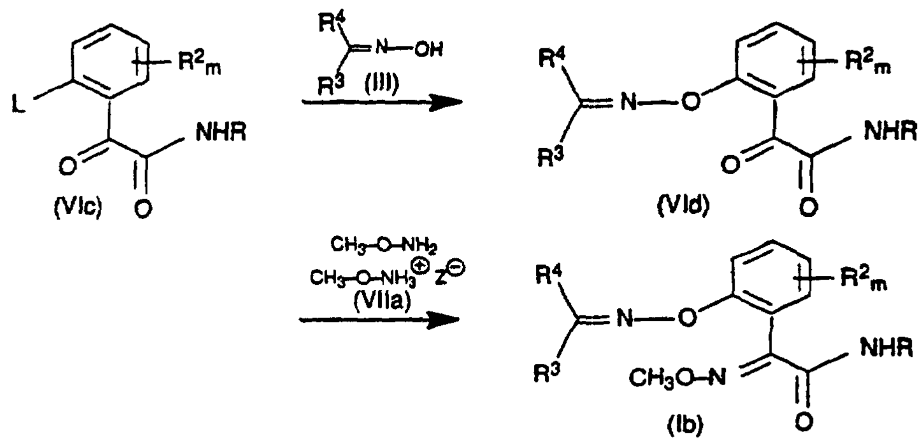


6. Nach einem weiteren Verfahren erhält man die Verbindungen I auch dadurch, daß man einen α-Ketoester VIe mit N-Hydroxyphthalimid (XI) in das entsprechende geschützte Oxim der Formel VIf überführt. Die α-Ketoester-Funktion des Oxims VIf kann anschließend in an sich bekannter Weise gemäß den vorstehend beschriebenen Verfahren in die verschiedenen Gruppen $R^1$ überführt werden (vgl. Formeln XIIa und XIIb). Die Phthalimidschutzgruppe wird anschließend in an sich bekannter Weise mit Hydrazin in Ethanol [Synthesis 23, 682 (1979)] oder mit Bromwasserstoff in Essigsäure [J.Org.Chem. 28, 1604 (1963)] abgespalten und das erhaltenen O-substituierte Hydroxylamin (vgl. Formeln XIIIa und XIIIb) wird in situ mit dem Keton der Formel IX zu I umgesetzt.

Die Reaktionsabfolge ist im folgenden Reaktionsschema skizziert.

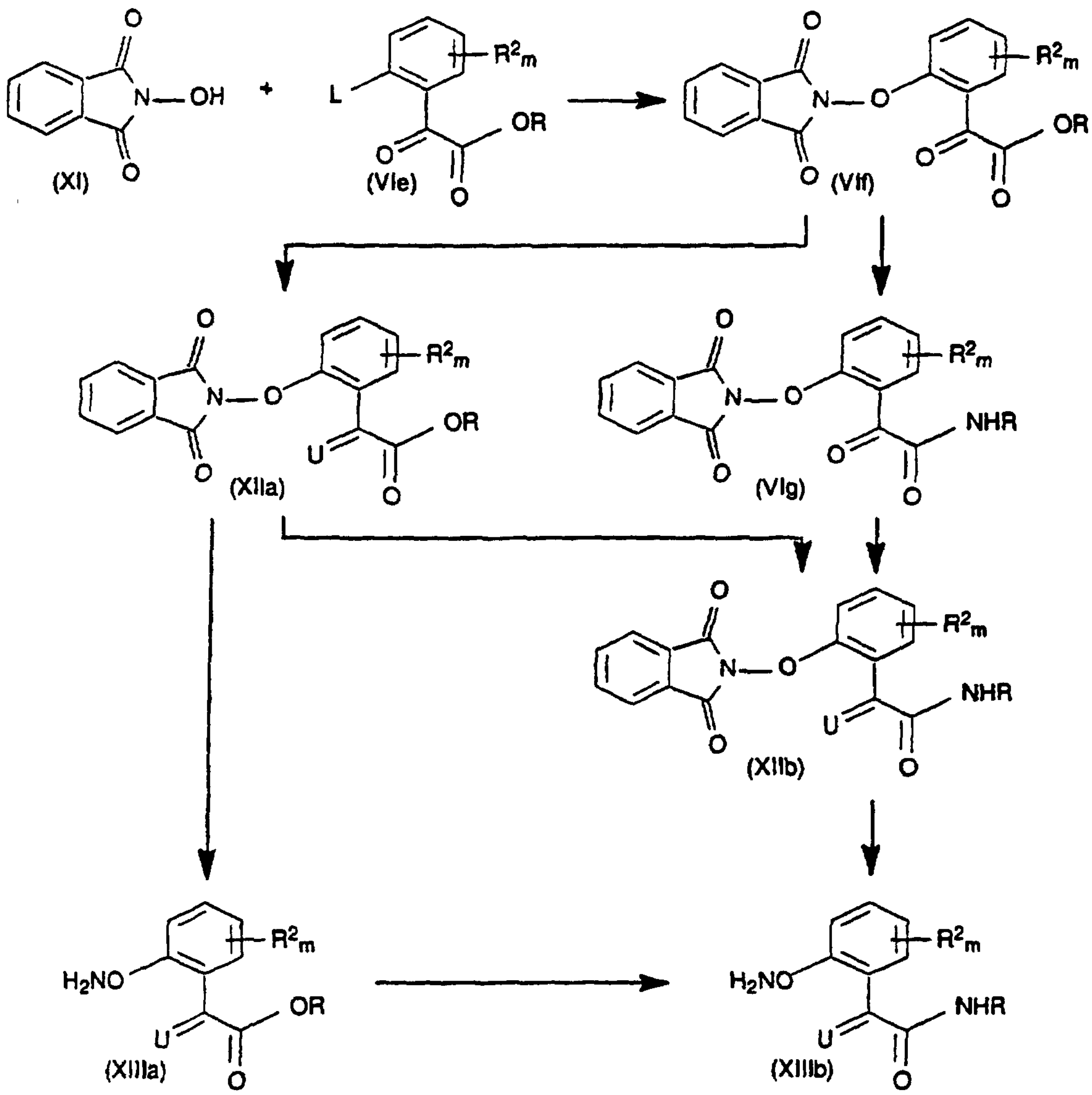

(XI) (VIe) (VIf) (XIIa) (VIg) (XIIb) (XIIIa) (XIIIb)

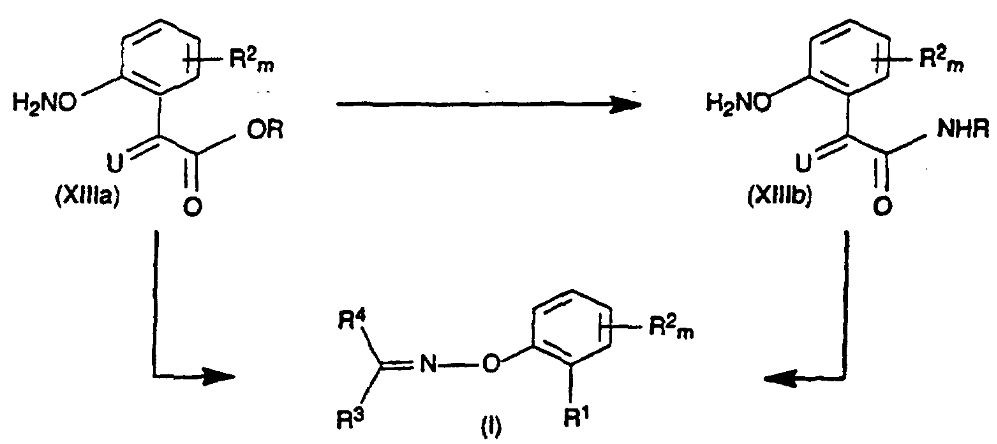

(XIIIa) (XIIIb) (I)

Die einzelnen Umsetzungen erfolgen im allgemeinen und besonderen unter den eingangs beschriebenen Verfahrensbedingungen.

7. Nach einem weiteren besonders bevorzugten Verfahren erhät man die Verbindungen I auch dadurch, daß man den α-Ketoester VIe zunächst durch Umsetzung mit dem Oxim III in den entsprechenden α-Ketoester VI überführt. Die α-Ketoester-Funktion des Oxims VI kann anschließend in an sich bekannter Weise gemäß den vorstehend beschriebenen Verfahren in die verschiedenen Gruppen $R^1$ überführt werden.

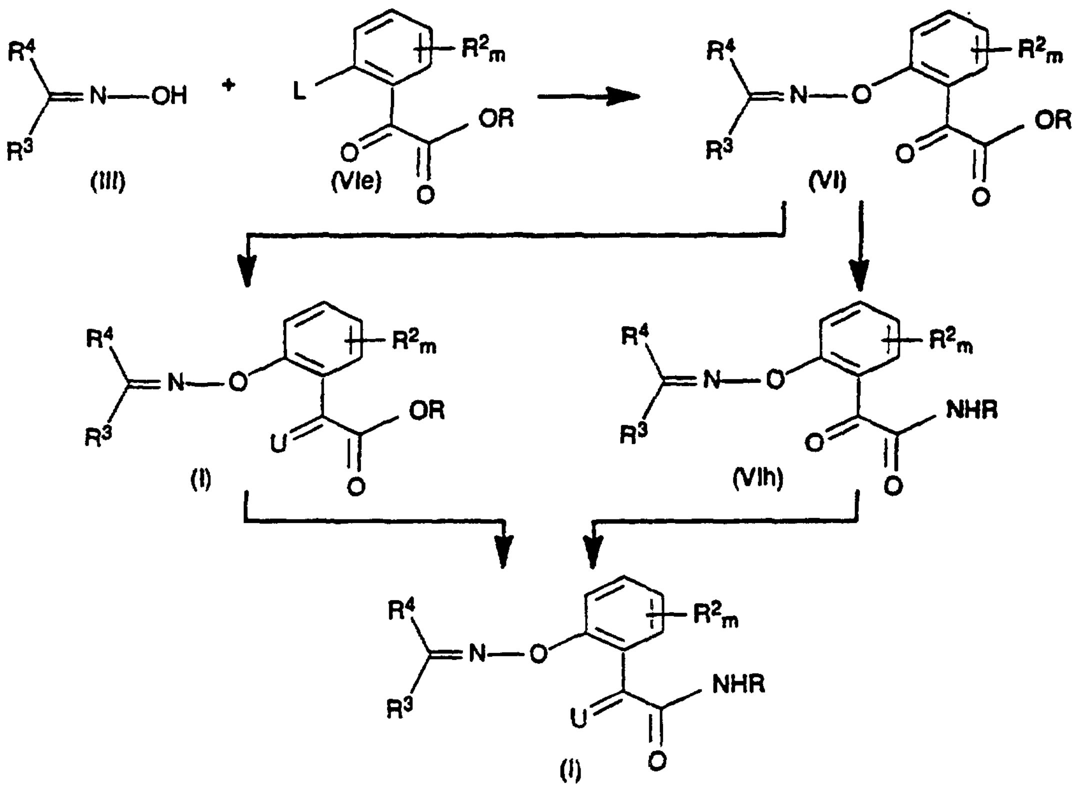

Die einzelnen Umsetzungen erfolgen im allgemeinen und besonderen unter den eingangs beschriebenen Verfahrensbedingungen.

Die für die vorstehenden Umsetzungen als Ausgangsstoff benötigten Verbindungen VIe erhält man beispielsweise gemäß dem folgenden Reaktionsschema:

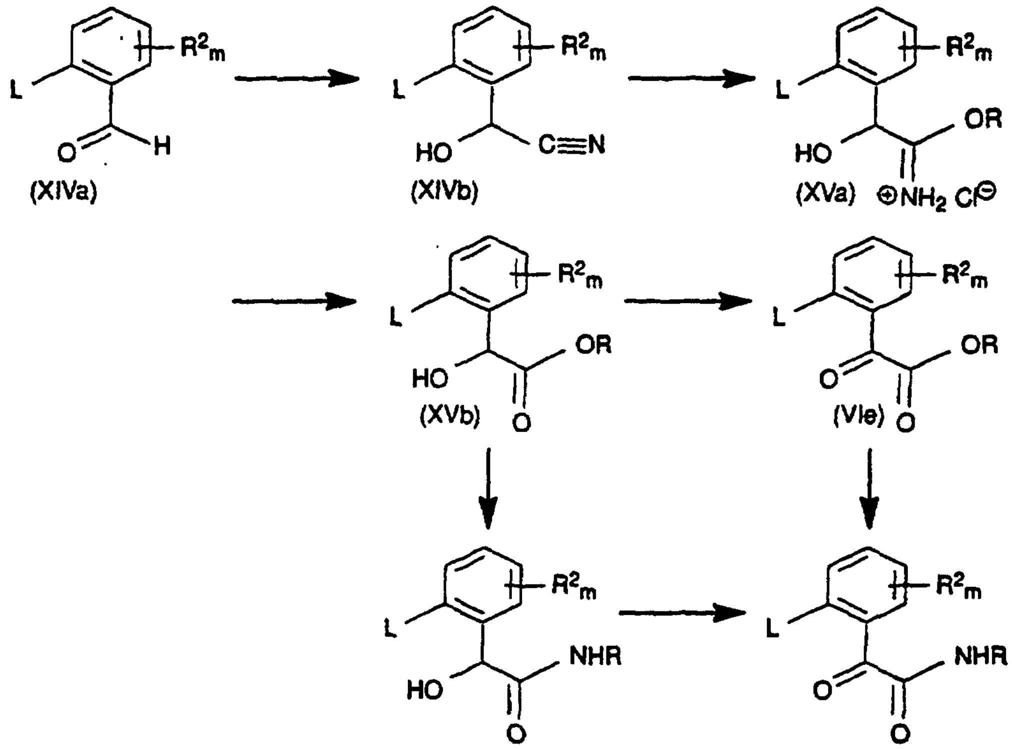

(XIVa) (XIVb) (XVa) (XVb) (VIe)

Die entsprechende Reaktionssequenz liefert den α-Ketoester der Formel VI bzw. das entsprechende α-Ketoamid, wenn man die Gruppe L gemäß den eingangs beschriebenen Bedingungen durch Umsetzung mit dem Oxim III (auf der Stufe der Verbindungen XIVa, XIVb, XVb oder VIe) durch die Gruppe $-ON{=}CR^3R^4$ substituiert.

Das Verfahren wird entsprechend den in *Coll. Czech. Chem. Commun.* 29, *97/119 (1964)* beschriebenen Methoden durchgeführt.

Nach einem weiteren Verfahren erhält man die Verbindungen I auch ausgehend von Phenylketonen der Formel XVI gemäß den in EP-A 178 826, EP-A 256 667 und EP-A 468 775 beschriebenen Methoden gemäß dem folgenden Reaktionsschema:

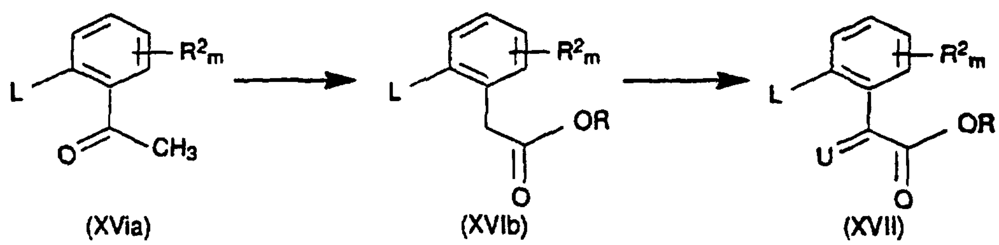

(XVIa) (XVIb) (XVII)

Die Gruppe L kann bei dieser Umsetzungssequenz in der vorstehend beschriebenen Weise auf den Stufen der Verbindungen XVIa, XVIb und XVII durch den Rest $-ON{=}CR^3R^4$ ersetzt werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Salze der Verbindungen I können in an sich bekannter Weise erhalten werden, indem man eine Verbindung I in Wasser oder einem inerten organischen Lösungsmittel bei Temperaturen von -80°C bis 120°C, vorzugsweise 0°C bis 60°C, mit einer zur Salzbildung geeigneten Säure versetzt.

Als Säuren eignen sich beispielsweise Mineralsäuren wie Schwefelsäure und Phosphorsäure oder Lewis-Säuren wie Zinkchlorid.

Im allgemeinen ist die Art des Salzes im Hinblick auf die Verwendung der Verbindungen I gegen tierische Schädlinge und Schadpilze unkritisch. Im Sinne der Erfidnung sind solche Salze bevorzugt, die die von tierischen Schädlingen oder Schadpilzen freizuhaltenden Pflanzen, Flächen, Materialien, Saatgüter und Räume nicht beeinträchtigen.

Die Verbindungen I können hinsichtlich ihrer C=N- bzw. C=C-Doppelbindungen als E- und Z-Isomere auftreten, wobei bezüglich der Doppelbindung des Restes $R^1$ grundsätzlich die E-Isomere biologisch aktiver und damit bevorzugt sind.

Bezüglich der Doppelbindung der O-N=$CR^3R^4$-Gruppierung und weiterer, in den Resten $R^3$ und $R^4$ auftretenden Doppelbindungen können ebenfalls E- und Z-Isomere auftreten. Im allgemeinen werden Verbindungen I bevorzugt, in denen diese Doppelbindungen in der E-Konfiguration vorliegen.

Üblicherweise werden diese Doppelbindungen bei der Synthese vorwiedend in der E-Konfiguration gebildet. Die Isomerengemische können entweder in üblicher Weise in die einzelnen Isomeren getrennt werden, z.B. durch fraktionierte Kristallisation oder Chromatographie, oder sie können unter der Einwirkung von Protonensäuren oder Lewis-Säuren oder von Licht in andere Isomere überführt werden. Sofern bei der Synthese Isomerengemische asnfallen, ist eine Trennung im allgemeinen nicht erforderlich, da sich die Isomeren teilweise bei der erfindungsgemäßen Anwendung, durch den Einfluß von Licht, Formulierungsmedium oder auch in vivo, ineinander umwandeln können.

Bei den in den vorstehenden Formeln angegebenen Definitionen der Symbole wurden Sammelbegriffe verwendet, die allgemein repräsentativ für die folgenden Substituenten stehen:

**Halogen:** Fluor, Chlor, Brom und Jod;

**Alkyl:** gesättigte, geradkettige oder verzweigte Kohlenwasserstoffreste mit 1 bis 4, 6 oder 10 Kohlenstoffatomen, z.B. $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

**Halogenalkyl:** geradkettige oder verzweigte Alkylgruppen mit 1 bis 4, 6 oder 10 Kohlenstoffatomen (wie vorstehend genannt), wobei in diesen Gruppen teilweise oder vollständig die Wasserstoffatome durch Halogenatome wie vorstehend genannt ersetzt sein können, z.B. $C_1$-$C_2$-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;

**Alkylcarbonyl:** eine Alkylgruppe mit 1 bis 6 oder 10 Kohlenstoffatomen (wie vorstehend genannt), welche über eine Carbonylgruppe (-CO-) an das Gerüst gebunden ist;

**Alkylsulfonyl:** eine geradkettige oder verzweigte Alkylgruppe mit 1 bis 6 oder 10 Kohlenstoffatomen (wie vorstehend genannt), welche über eine Sulfonylgruppe (-$SO_2$-) an das Gerüst gebunden ist; **AlkylsulfoXyl:** eine geradkettige oder verzweigte Alkylgruppe mit 1 bis 6 Kohlenstoffatomen (wie vorstehend genannt), welche über eine Sulfoxylgruppe (-$SO_3$-) an das Gerüst gebunden ist;

**Alkoxy:** geradkettige oder verzweigte Alkylgruppen mit 1 bis 4 oder 6 Kohlenstoffatomen (wie vorstehend genannt), welche über ein Sauerstoffatom (-O-) an das Gerüst gebunden sind;

**Halogenalkoxy:** geradkettige oder verzweigte Halogenalkylgruppen mit 1 bis 4, 6 oder 10 Kohlenstoffatomen (wie vorstehend genannt), welche über ein Sauerstoffatom (-O-) an das Gerüst gebunden sind;

**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;

**Alkoxyimino:** geradkettige oder verzweigte Alkylgruppen mit 1 bis 4 Kohlenstoffatomen (wie vorstehend genannt), welche über eine Oxyiminogruppe (-ON=) an das Gerüst gebunden sind;

**Alkylthio:** geradkettige oder verzweigte Alkylgruppen mit 1 bis 6 Kohlenstoffatomen (wie vorstehend genannt), welche über ein Schwefelatom (-S-) an das Gerüst gebunden sind;

**Alkylamino:** eine geradkettige oder verzweigte Alkylgruppe mit 1 bis 6 Kohlenstoffatomen (wie vorstehend genannt), welche über eine Aminogruppe (-NH-) an das Gerüst gebunden ist;

**Dialkylamino:** zwei voneinander unabhängige geradkettige oder verzweigte Alkylgruppen mit jeweils 1 bis 6 Kohlenstoffatomen (wie vorstehend genannt), welche über ein Stickstoffatom an das Gerüst gebunden sind;

**Alkylaminocarbonyl:** eine Alkylaminogruppe mit 1 bis 6 Kohlenstoffatomen (wie vorstehend genannt), welche über eine Carbonylgruppe (-CO-) an das Gerüst gebunden ist;

**Dialkylaminocarbonyl:** eine Dialkylaminogruppe mit zwei voneinander unabhängigen $C_1$-$C_6$-Alkylgruppen (wie vorstehend genannt), welche über eine Carbonylgruppe (-CO-) an das Gerüst gebunden ist;

**Alkylaminothiocarbonyl:** eine Alkylaminogruppe mit 1 bis 6 Kohlenstoffatomen (wie vorstehend genannt), welche über eine Thiocarbonylgruppe (-CS-) an das Gerüst gebunden ist;

**Dialkylaminothiocarbonyl**: eine Dialkylaminogruppe mit zwei voneinander unabhängigen $C_1$-$C_6$-Alkylgruppen (wie vorstehend genannt), welche über eine Thiocarbonylgruppe (-CS-) an das Gerüst gebunden ist;

**Alkenyl:** ungesättigte, geradkettige oder verzweigte Kohlenwasserstoffreste mit 2 bis 6 oder 10 Kohlenstoffatomen und einer Doppelbindung in einer beliebigen Position, z.B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;

**Alkenyloxy:** ungesättigte, geradkettige oder verzweigte Kohlenwasserstoffreste mit 3 bis 6 oder 10 Kohlenstoffatomen und einer Doppelbindung in einer beliebigen, nicht zum Heteroatom benachbarten, Position (wie vorstehend genannt), welche über ein Sauerstoffatom (-O-) an das Gerüst gebunden sind;

**Alkenylcarbonyl:** ungesättigte, geradkettige oder verzweigte Kohlenwasserstoffreste mit 2 bis 10 Kohlenstoffatomen und einer Doppelbindung in einer beliebigen Position (wie vorstehend genannt), welche über eine Carbonylgruppe (-CO-) an das Gerüst gebunden sind;

**Alkinyl:** geradkettige oder verzweigte Kohlenwasserstoffgruppen mit 2 bis 4, 6, 8 oder 10 Kohlenstoffatomen und einer Dreifachbindung in einer beliebigen Position, z.B. $C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;

**Alkinyloxy:** ungesättigte, geradkettige oder verzweigte Kohlenwasserstoffreste mit 3 bis 10 Kohlenstoffatomen und einer Dreifachbindung in einer beliebigen, nicht zum Heteroatom benachbarten, Position (wie vorstehend genannt), welche über ein Sauerstoffatom (-O-) an das Gerüst gebunden sind;

**Alkinylcarbonyl:** ungesättigte, geradkettige oder verzweigte Kohlenwasserstoffreste mit 2 bis 10 Kohlenstoffatomen und einer Dreifachbindung in einer beliebigen Position (wie vorstehend genannt), welche über eine Carbonylgruppe (-CO-) an das Gerüst gebunden sind;

**Cycloalkyl:** monocyclische, gesättigte Kohlenwasserstoffgruppen mit 3 bis 6 oder 10 Kohlenstoffringgliedern, z. B. $C_3$-$C_8$-Cycloalkyl wie Cyclopropyl, Cyclobutyl, Cyclopentyl, Cyclohexyl, Cycloheptyl und Cyclooctyl;

**Cycloalkoxy:** monocyclische, gesättigte Kohlenwasserstoffgruppen mit 3 bis 6 oder 10 Kohlenstoffringgliedern (wie vorstehend genannt), welche über ein Sauerstoffatom (-O-) an das Gerüst gebunden sind;

**Cycloalkenyl:** monocyclische, einfach ungesättigte Kohlenwasserstoffgruppen mit 3 bis 6 oder 10 Kohlenstoffringgliedern und einer Doppelbindung in einer beliebigen Position des Ringes, z.B. $C_5$-$C_8$-Cycloalkyl wie Cyclopentenyl, Cyclohexenyl, Cycloheptenyl und Cyclooctenyl;

**Heterocyclyl:** 5- oder 6-gliedrige Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Didydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl;

**Heterocyclyloxy:** 5- oder 6-gliedrige Heterocyclen (wie vorstehend genannt), welche über ein Sauerstoffatom (-O-) an das Gerüst gebunden sind;

**Aryl:** ein ein- bis dreikerniges aromatisches Ringsystem enthaltend 6 bis 14 Kohlenstoffringglieder, z.B. Phenyl, Naphthyl und Anthracenyl;

**Aryloxy:** ein ein- bis dreikerniges aromatisches Ringsystem (wie vorstehend genannt), welches über ein Sauerstoffatom (-O-) an das Gerüst gebunden ist;

**Arylthio:** ein ein- bis dreikerniges aromatisches Ringsystem (wie vorstehend genannt), welches über ein Schwefelatom (-S-) an das Gerüst gebunden ist;

**Arylcarbonyl:** ein ein- bis dreikerniges aromatisches Ringsystem (wie vorstehend genannt), welches über eine Carbonylgruppe (-CO-) an das Gerüst gebunden ist;

**Arylsulfonyl:** ein ein- bis dreikerniges aromatisches Ringsystem (wie vorstehend genannt), welches über eine Sulfonylgruppe (-$SO_2$-) an das Gerüst gebunden ist;

**Hetaryl:** ein ein- oder zweikerniges, 5-, 6-, 9- oder 10-gliedriges aromatisches Ringsystem, welches neben Kohlenstoffringgliedern Heteroatome aus der Gruppe Sauerstoff, Schwefel und Stickstoff enthält: z.B.

- 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-oxadiazol-2-yl, 1,3,4-Thiadiazol-2-yl und 1,3,4-Triazol-2-yl;

- benzokondensiertes 5-gliedriges Heteroaryl, enthaltend ein bis drei Stickstoffatome oder ein Stickstoffatom und ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
- über Stickstoff gebundenes 5-qliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoff ringglieder an das Gerüst gebunden sind;
- 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;

**Hetaryloxy:** ein ein- oder zweikerniges heteroaromatisches Ringsystem (wie vorstehend genannt), welches über ein Sauerstoffatom (-O-) an das Gerüst gebunden ist;

**Hetarylthio:** ein ein- oder zweikerniges heteroaromatisches Ringsystem (wie vorstehend genannt), welches über ein Schwefelatom (-S-) an das Gerüst gebunden ist;

**Hetarylcarbonyl:** ein ein- oder zweikerniges heteroaromatisches Ringsystem (wie vorstehend genannt), welches über eine Carbonylgruppe (-CO-) an das Gerüst gebunden ist;

**Hetarylsulfonyl:** ein ein- oder zweikerniges heteroaromatisches Ringsystem (wie vorstehend genannt), welches über eine Sulfonylgruppe ($-SO_2-$) an das Gerüst gebunden ist.

Der Zusatz *"ggf. subst."* in Bezug auf *Alkyl-, Alkenyl- und Alkinylgruppen* soll zum Ausdruck bringen, daß diese Gruppen partiell oder vollständig halogeniert sein können *[d.h. die Wasserstoffatome dieser Gruppen können teilweise* oder *vollständig durch gleiche* oder *verschiedene Halogenatome wie vorstehend genannt (vorzugsweise Fluor, Chlor oder Brom) ersetzt sein]* und/ oder einen bis drei (vorzugsweise einen) der folgenden Reste tragen können:

- Cyano, Nitro, Hydroxy, Amino, Formyl, Carboxyl. Aminocarbonyl, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten;
- unsubstituiertes oder durch übliche Gruppen substituiertes Cycloalkyl, Cycloalkoxy, Cycloalkylthio, Cycloalkylamino, Cycloalkyl-N-alkylamino, Heterocyclyl, Heterocyclyloxy, Heterocyclylthio, Heterocyclylamino oder Heterocyclyl-N-alkylamino, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
- unsubstituiertes oder durch übliche Gruppen substituiertes Aryl, Aryloxy, Arylthio, Arylamino, Aryl-N-alkylamino, Arylalkoxy, Arylalkylthio, Arylalkylamino, Arylalkyl-N-alkylamino, Hetaryl, Hetaryloxy, Hetarylthio, Hetarylamino, Hetaryl-N-alkylamino, Hetarylalkoxy, Hetarylalkylthio, Hetarylalkylamino und Hetarylalkyl-N-alkylamino, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten.

Der Zusatz *"ggf. subst"* in Bezug auf die *cyclischen (gesättigten, ungesättigten oder aromatischen) Gruppen* soll zum Ausdruck bringen, daß diese Gruppen partiell oder vollständig halogeniert sein können *[d.h. die Wasserstoffa-*

*tome dieser Gruppen können teilweise oder vollständig durch gleiche oder verschiedene Halogenatome wie vorstenend genannt (vorzugsweise Fluor, Chlor oder Brom, insbesondere Fluor oder Chlor) ersetzt sein]* und/oder einen bis vier (insbesondere einen bis drei) der folgenden Reste tragen können:

- Cyano, Nitro, Hydroxy, Amino, Carboxyl, Aminocarbonyl, Alkyl, Haloalkyl, Alkenyl, Haloalkenyl, Alkenyloxy, Haloalkenyloxy, Alkinyl, Haloalkinyl, Alkinyloxy, Haloalkinyloxy, Alkoxy, Halogenalkoxy, Alkylthio, Halogenalkylthio, Alkylamino, Dialkylamino, Alkylcarbonyl, Alkoxycarbonyl, Alkylcarbonyloxy, Alkylaminocarbonyl, Dialkylaminocarbonyl, Alkylcarbonylamino, Alkoxycarbonylamino, Alkylcarbonyl-N-alkylamino und Alkylcarbonyl-N-alkylamino, wobei die Alkylgruppen in diesen Resten vorzugsweise 1 bis 6 Kohlenstoffatome, insbesondere 1 bis 4 Kohlenstoffatome enthalten und die genannten Alkenyl- oder Alkinylgruppen in diesen Resten 2 bis 8, vorzugsweise 2 bis 6, insbesondere 2 bis 4 Kohlenstoffatome enthalten;

und/oder einen bis drei (insbesondere einen) der folgenden Reste:

- unsubstituiertes oder durch übliche Gruppen substituiertes Cycloalkyl, Cycloalkoxy, Cycloalkylthio, Cycloalkylamino, Cycloalkyl-N-alkylamino, Heterocyclyl, Heterocyclyloxy, Heterocyclylthio, Heterocyclylamino oder Heterocyclyl-N-alkylamino, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
- unsubstituiertes oder durch übliche Gruppen substituiertes Aryl, Aryloxy, Arylthio, Arylamino, Aryl-N-alkylamino, Arylalkoxy, Arylalkylthio, Arylalkylamino, Arylalkyl-N-alkylamino, Hetaryl, Hetaryloxy, Hetarylthio, Hetarylamino, Hetaryl-N-alkylamino, Hetarylalkoxy, Hetarylalkylthio, Hetarylalkylamino und Hetarylalkyl-N-alkylamino, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten

und/oder einen oder zwei (insbesondere einen) der folgenden Reste trage kann:

- Formyl,
- $CR^v$=$NOR^{vi}$ [wobei $R^v$ Wasserstoff, Alkyl, Cycloalkyl und Aryl und $R^{vi}$ Alkyl, Alkenyl, Halogenalkenyl, Alkinyl und Arylalkyl bedeutet (wobei die genannten Alkylgruppen vorzugsweise 1 bis 6 Kohlenstoffatome, insbesondere 1 bis 4 Kohlenstoffatome, enthalten, die genannten Cycloalkylgruppen, Alkenylgruppen und Alkinylgruppen vorzugsweise 3 bis 8, insbesondere 3 bis 6, Kohlenstoffatome enthalten) und Aryl insbesondere Phenyl bedeutet, welches unsubstituiert ist oder durch übliche Gruppen substituiert sein kann] oder
- $NR^{vii}$-CO-D-$R^{viii}$ [wobei $R^{vii}$ für Wasserstoff, Hydroxy, $C_1$-$C_6$-Alkyl, $C_2$-$C_6$-Alkenyl, $C_2$-$C_6$-Alkinyl, $C_1$-$C_6$-Alkoxy, $C_2$-$C_6$-Alkenyloxy, $C_2$-$C_6$-Alkinyloxy, $C_1$-$C_6$-Alkoxy-$C_1$-$C_6$-alkyl, $C_1$-$C_6$-Alkoxy-$C_1$-$C_6$-alkoxy und $C_1$-$C_6$-Alkoxycarbonyl steht, $R^{viii}$ für Wasserstoff, $C_1$-$C_6$-Alkyl, $C_2$-$C_6$-Alkenyl, $C_2$-$C_6$-Alkinyl, $C_3$-$C_6$-Cycloalkyl, $C_3$-$C_6$-Cycloalkenyl, Aryl, Aryl-$C_1$-$C_6$-alkyl, Hetaryl und Hetaryl-$C_1$-$C_6$-alkyl steht und D eine direkte Bindung, Sauerstoff oder Stickstoff bedeutet, wobei der Stickstoff eine der bei $R^{vi}$ genannten Gruppen tragen kann],

und/oder bei denen zwei benachbarte C-Atome der cyclischen Systeme eine $C_3$-$C_5$-Alkylen-, $C_3$-$C_5$-Alkenylen-, Oxy-$C_2$-$C_4$-alkylen-, Oxy-$C_1$-$C_3$-alkylenoxy, oxy-$C_2$-$C_4$-alkenylen-, Oxy-$C_2$-$C_4$-alkenylenoxy- oder Butadiendiylgruppe tragen können, wobei diese Brücken ihrerseits partiell oder vollständig halogeniert sein können und/oder einen bis drei, insbesondere einen oder zwei der folgenden Reste tragen können:

- $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio.

Unter üblichen Gruppen sind insbesondere die folgenden Substituenten zu verstehen: Cyano, Nitro, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, Di-$C_1$-$C_4$-Alkylamino und $C_1$-$C_4$-Aakylthio.

Im Hinblick auf ihre biologische Wirkungen werden Verbindungen der Formel I bevorzugt, in denen $R^4$ die folgende Bedeutung hat:

Alkyl, Alkenyl, Alkinyl, Alkoxy, Alkenyloxy oder Alkinyloxy, wobei die Kohlenwasserstoffgruppen ihrerseits partiell oder vollständig halogeniert sein können und/oder eine bis drei der folgenden Reste tragen können:

- Cyano, Nitro, Hydroxy, Mercapto, Amino, Carboxyl, Aminocarbonyl, Aminothiocarbonyl,
- $C_1$-$C_6$-Alkylsulfonyl, $C_1$-$C_6$-Alkylsulfoxyl, $C_1$-$C_6$-Alkylcarbonyl, $C_3$-$C_6$-Cycloalkyl, $C_1$-$C_6$-Alkoxy, $C_1$-$C_6$-Halo-

genalkoxy, $C_1$-$C_6$-Alkoxycarbonyl, $C_1$-$C_6$-Alkylthio, $C_1$-$C_6$-Alkylamino, Di-$C_1$-$C_6$-alkylamino, $C_1$-$C_6$-Alkylaminocarbonyl, Di-$C_1$-$C_6$-alkylaminocarbonyl, $C_1$-$C_6$-Alkylaminothiocarbonyl, Di-$C_1$-$C_6$-alkylaminothiocarbonyl, $C_2$-$C_6$-Alkenyloxy,
- Benzyloxy, Aryl, Aryloxy, Hetaryl und Hetaryloxy, wobei die aromatischen Ringe durch übliche Gruppen substituiert sein können;

Cycloalkyl, Cycloalkoxy, Heterocyclyl oder Heterocyclyloxy, wobei die cyclischen Gruppen ihrerseits partiell oder vollständig halogeniert sein können und/oder eine bis drei der folgenden Reste tragen können:

- Cyano, Nitro, Hydroxy, Mercapto, Amino, Carboxyl, Aminocarbonyl, Aminothiocarbonyl,
- $C_1$-$C_6$-Alkyl, $C_1$-$C_6$-Halogenalkyl, $C_1$-$C_6$-Alkylsulfonyl, $C_1$-$C_6$-Alkylsulfoxyl, $C_1$-$C_6$-Alkylcarbonyl, $C_3$-$C_6$-Cycloalkyl, $C_1$-$C_6$-Alkoxy, $C_1$-$C_6$-Halogenalkoxy, $C_1$-$C_6$-Alkoxycarbonyl, $C_1$-$C_6$-Alkylthio, $C_1$-$C_6$-Alkylamino, Di-$C_1$-$C_6$-alkylamino, $C_1$-$C_6$-Alkylaminocarbonyl, Di-$C_1$-$C_6$-alkylaminocarbonyl, $C_1$-$C_6$-Alkylaminothiocarbonyl, Di-$C_1$-$C_6$-alkylaminothiocarbonyl, $C_2$-$C_6$-Alkenyl, $C_2$-$C_6$-Alkenyloxy,
- Benzyl, Benzyloxy, Aryl, Aryloxy, Hetaryl und Hetaryloxy, wobei die aromatischen Ringe durch übliche Gruppen substituiert sein können;

Aryl, Hetaryl, Aryloxy oder Hetaryloxy, wobei die cyclischen Gruppen ihrerseits partiell oder vollständig halogeniert sein können und/oder eine bis drei der folgenden Reste tragen können:

- Cyano, Nitro, Hydroxy, Mercapto, Amino, Carboxyl, Aminocarbonyl, Aminothiocarbonyl,
- $C_1$-$C_6$-Alkyl, $C_1$-$C_6$-Halogenalkyl, $C_1$-$C_6$-Alkylsulfonyl, $C_1$-$C_6$-Alkylsulfoxyl, $C_1$-$C_6$-Alkylcarbonyl, $C_3$-$C_6$-Cycloalkyl, $C_1$-$C_6$-Alkoxy, $C_1$-$C_6$-Halogenalkoxy, $C_1$-$C_6$-Alkoxycarbonyl, $C_1$-$C_6$-Alkylthio, $C_1$-$C_6$-Alkylamino, Di-$C_1$-$C_6$-alkylamino, $C_1$-$C_6$-Alkylaminocarbonyl, Di-$C_1$-$C_6$-alkylaminocarbonyl, $C_1$-$C_6$-Alkylaminothiocarbonyl, Di-$C_1$-$C_6$-alkylaminothiocarbonyl, $C_2$-$C_6$-Alkenyl, $C_2$-$C_6$-Alkenyloxy,
- Benzyl, Benzyloxy, Aryl, Aryloxy, Hetaryl, Hetaryloxy, wobei die aromatischen Ringe durch übliche Gruppen substituiert sein können,
- C(=NOR$^i$)-$A_n$-R$^{ii}$ oder NR$^{iii}$-CO-D-R$^{iv}$;

A Sauerstoff, Schwefel oder Stickstoff, wobei der Stickstoff Wasserstoff oder $C_1$-$C_6$-Alkyl trägt;

n 0 oder 1;

R$^i$ Wasserstoff, $C_1$-$C_6$-Alkyl, $C_3$-$C_6$-Alkenyl oder $C_3$-$C_6$-Alkinyl;

R$^{ii}$ Wasserstoff oder $C_1$-$C_6$-Alkyl;

D eine direkte Bindung, Sauerstoff oder NR$^b$ (R$^b$ = Wasserstoff, $C_1$-$C_6$-Alkyl, $C_2$-$C_6$-Alkenyl, $C_2$-$C_6$-Alkinyl, $C_3$-$C_6$-Cycloalkyl, $C_3$-$C_6$-Cycloalkenyl, Aryl, Aryl-$C_1$-$C_6$-alkyl, Hetaryl und Hetaryl-$C_1$-$C_6$-alkyl);

R$^{iii}$ Wasserstoff, Hydroxy, $C_1$-$C_6$-Alkyl, $C_2$-$C_6$-Alkenyl, $C_2$-$C_6$-Alkinyl, $C_1$-$C_6$-Alkoxy, $C_2$-$C_6$-Alkenyloxy, $C_2$-$C_6$-Alkinyloxy, $C_1$-$C_6$-Alkoxy-$C_1$-$C_6$-alkyl, $C_1$-$C_6$-Alkoxy-$C_1$-$C_6$-alkoxy und $C_1$-$C_6$-Alkoxycarbonyl;

R$^{iv}$ Wasserstoff, $C_1$-$C_6$-Alkyl, $C_2$-$C_6$-Alkenyl, $C_2$-$C_6$-Alkinyl, $C_3$-$C_6$-Cycloalkyl, $C_3$-$C_6$-Cycloalkenyl, Aryl, Aryl-$C_1$-$C_6$-alkyl, Hetaryl und Hetaryl-$C_1$-$C_6$-alkyl.

Besonders werden Phenylderivate der Formel I bevorzugt, in denen die Substituenten die folgende Bedeutung haben:

R$^3$ Wasserstoff, Cyano,
$C_1$-$C_6$-Alkyl, $C_1$-$C_6$-Halogenalkyl, $C_3$-$C_6$-Cycloalkyl, $C_3$-$C_6$-Cycloalkyl-$C_1$-$C_4$-alkyl,
Aryl, Aryl-$C_1$-$C_4$-alkyl und Aryloxy-$C_1$-$C_4$-alkyl, wobei die aromatischen Ringe partiell oder vollständig halogeniert sein können und/oder einen bis drei der folgenden Reste tragen können: Cyano, Nitro, $C_1$-$C_6$-Alkyl,
$C_1$-$C_6$-Halogenalkyl $C_1$-$C_6$-Alkoxy, $C_1$-$C_6$-Halogenalkoxy, und C(=NOR$^i$)-$A_n$-R$^{ii}$;

R$^4$ ggf. subst. Aryl, Hetaryl, Aryloxy, Hetaryloxy, Arylthio und Hetarylthio:

$$-Qp-C(R^5)=N-Y^1-R^6$$

oder

$$-Q-O-N=CR^7R^8,$$

$R^3$ und $R^4$ gemeinsam mit dem Kohlenstoffatom, an das sie gebunden sind, ein vier- bis achtgliedriger Ring, welcher neben Kohlenstoffatomen ein oder zwei Sauerstoff- und/ oder Schwefelatome und/oder NH- und/oder N ($C_1$-$C_4$-Alkyl)-Gruppen enthalten kann, und dessen Kohlenstoffatome einen der folgenden Substituenten tragen können: Habgen, $C_1$-$C_6$-Alkyl oder $C_1$-$C_4$-Alkoxyimino; wobei

$R^3$ und $R^4$ nicht gleichzeitig über Heteroatome an das Kohlenstoffatom gebunden sind.

Außerdem werden Phenylderivate der Formel I bevorzugt, in der die Substituenten die folgende Bedeutung haben:

$R^3$ Wasserstoff, Cyano, Halogen,
$C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_3$-$C_6$-Cycloalkyl $C_3$-$C_6$-Cycloalkyl-$C_1$-$C_4$-alkyl,
Aryl, Aryl-$C_1$-$C_4$-alkyl, Aryloxy-$C_1$-$C_4$-alkyl und Aryl-$C_1$-$C_4$-alkoxy, wobei die aromatischen Ringe partiell oder vollständig halogeniert sein können und/oder einen bis drei der folgenden Reste tragen können: Cyano, Nitro, $C_1$-$C_6$-Alkyl, $C_1$-$C_6$-Halogenalkyl, $C_1$-$C_6$-Alkoxy, $C_1$-$C_6$-Halogenalkoxy, und $C(=NOR^i)-A_n-R^{ii}$;

$R^4$ ggf. subst. Aryl und Hetaryl;

$$-Q_p-C(R^5)=N-Y^1-R^6$$

oder

$$-Q-O-N=CR^7R^8,$$

$R^3$und $R^4$ nicht gleichzeitig über Heteroatome an das Kohlenstoffatom gebunden sind.

Bevorzugt sind weiterhin Verbindungen der Formel I, in denen

- m für 0 steht,
- $R^1$ für die Gruppen (Ia), (Ib), (Ic) und (Id) steht,
- $R^1$ für die Gruppe (Ib) steht,
- $R^1$ für die Gruppe (Id) steht,
- $R^3$ für $C_1$-$C_4$-Alkyl (insbesondere Methyl) steht,
- $R^4$ für ggf. subst. $C_1$-$C_6$-Alkyl steht,
- $R^4$ für ggf. subst. $C_2$-$C_6$-Alkenyl steht,
- $R^4$ für ggf. subst. Aryl (insbesondere 3-substituiertes Phenyl) steht,
- $R^3$ für Methyl und $R^4$ für ggf. subst. $C_1$-$C_6$-Alkyl steht,
- $R^3$ für Methyl und $R^4$ für ggf. subst. Aryl (insbesondere 3-substituiertes Phenyl) steht,
- $R^4$ für $C(R^5)=N-Y^1-R^6$ steht, wobei $Y^1$ insbesondere Sauerstoff bedeutet und $R^5$ insbesondere Methyl, Ethyl, Allyl oder Propargyl bedeutet.

Bevorzugt sind weiterhin Verbindungen der Formel I, in denen

$R^3$ für $C_1$-$C_6$-Alkyl, $C_1$-$C_6$-Halogenalkyl, $C_1$-$C_6$-Alkoxy, $C_1$-$C_6$-Halogenalkoxy, Cyclopropyl oder $C(CH_3)=NO-C_1-C_6$-Alkyl steht, und
$R^4$ Aryl, Hetaryl oder Benzyl bedeutet, wobei die aromatischen Ringe in diesen Resten teilweise oder vollständig

halogeniert sein können und/oder einen bis drei der folgenden Reste tragen können: Cyano, Nitro, $C_1$-$C_6$-Alkyl, $C_1$-$C_6$-Halogenalkyl, $C_1$-$C_6$-Alkoxy, $C_1$-$C_6$-Halogenalkoxy und C(=NOR$^i$)-$A_n$-R$^{ii}$, oder

$R^4$ für CR$^5$=N-Y$^1$-R$^6$ steht.

Bevorzugt sind weiterhin Verbindungen der Formel I, in denen

$R^3$ für $C_1$-$C_6$-Alkyl, $C_1$-$C_6$-Halogenalkyl, $C_1$-$C_6$-Alkoxy, $C_1$-$C_6$-Halogenalkoxy, Cyclopropyl oder C($CH_3$)=NO-$C_1$-$C_6$-Alkyl steht, und

R4 $C_1$-$C_6$-Alkyl, $C_2$-$C_6$-Alkenyl, $C_2$-$C_6$-Alkenyl oder Cyclopropyl steht, wobei diese Reste teilweise oder vollständig halogeniert sein können und/oder eine bis drei der folgenden Gruppen tragen können: Aryl und Hetaryl, wobei die aromatischen Gruppen iherseits einen bis drei der folgenden Substituenten tragen können: Cyano, Halogen, $C_1$-$C_6$-Alkyl, $C_1$-$C_6$-Halogenalkyl, $C_1$-$C_6$-Alkoxy und $C_1$-$C_6$-Halogenalkoxy.

Bevorzugt sind weiterhin Verbindungen der Formel I, in denen

$R^3$ für $C_1$-$C_6$-Alkyl, $C_1$-$C_6$-Halogenalkyl, Cyclopropyl oder C($CH_3$)=NO-$C_1$-$C_6$-Alkyl steht, und

$R^4$ $C_1$-$C_6$-Alkoxy bedeutet, wobei dieser Rest teilweise oder vollständig halogeniert sein kann und/oder eine bis drei der folgenden Gruppen tragen kann: Aryl und Hetaryl, wobei die aromatischen Gruppen iherseits einen bis drei der folgenden Substituenten tragen können: Cyano, Halogen, $C_1$-$C_6$-Alkyl, $C_1$-$C_6$-Halogenalkyl, $C_1$-$C_6$-Alkoxy und $C_1$-$C_6$-Halogenalkoxy.

Bevorzugt sind weiterhin Verbindungen der Formel I, in denen

$R^3$ für $C_1$-$C_6$-Alkyl, $C_1$-$C_6$-Halogenalkyl, $C_1$-$C_6$-Alkoxy, $C_1$-$C_6$-Halogenalkoxy, Cyclopropyl oder C($CH_3$)=NO-$C_1$-$C_6$-Alkyl steht, und

$R^4$ für $C_1$-$C_6$-Alkyl, $C_2$-$C_6$-Alkenyl, $C_2$-$C_6$-Alkenyl oder Cyclopropyl steht, wobei diese Reste teilweise oder vollständig halogeniert sein können und/oder eine bis drei der folgenden Gruppen tragen können: Aryl und Hetaryl, wobei die aromatischen Gruppen iherseits einen bis drei der folgenden Substituenten tragen können: Cyano, Halogen, $C_1$-$C_6$-Alkyl, $C_1$-$C_6$-Halogenalkyl, $C_1$-$C_6$-Alkoxy und $C_1$-$C_6$-Halogenalkoxy, oder

$R^4$ Aryl, Hetaryl oder Benzyl bedeutet, wobei die aromatischen Ringe in diesen Resten teilweise oder vollständig halogeniert sein können und/oder einen bis drei der folgenden Reste tragen können: Cyano, Nitro, $C_1$-$C_6$-Alkyl, $C_1$-$C_6$-Halogenalkyl, $C_1$-$C_6$-Alkoxy, $C_1$-$C_6$-Halogenalkoxy und C(=NOR$^i$)-$A_n$-R$^{ii}$, oder

$R^4$ für CR$^5$=N-Y$^1$-R$^6$ steht.

Bevorzugt sind weiterhin Verbindungen der Formel I, in denen

$R^3$ für $C_1$-$C_6$-Alkyl, $C_1$-$C_6$-Halogenalkoxy, Cyclopropyl oder C($CH_3$)=NO-$C_1$-$C_6$-Alkyl steht, und

$R^4$ für $C_1$-$C_6$-Alkyloxy steht, wobei dieser Rest teilweise oder vollständig halogeniert sein kann und/oder eine bis drei der folgenden Gruppen tragen kann: Aryl und Hetaryl, wobei die aromatischen Gruppen iherseits einen bis drei der folgenden Substituenten tragen können: Cyano, Halogen, $C_1$-$C_6$-Alkyl, $C_1$-$C_6$-Halogenalkyl, $C_1$-$C_6$-Alkoxy und $C_1$-$C_6$-Halogenalkoxy, oder

$R^4$ Aryloxy, Hetaryloxy oder N-Aryl-N-methylamino bedeutet, wobei die aromatischen Ringe in diesen Resten teilweise oder vollständig halogeniert sein können und/oder einen bis drei der folgenden Reste tragen können: Cyano, Nitro, $C_1$-$C_6$-Alkyl, $C_1$-$C_6$-Halogenalkyl, $C_1$-$C_6$-Alkoxy, $C_1$-$C_6$-Halogenalkoxy und C(=NOR$^i$)-$A_n$-R$^{ii}$, oder

$R^4$ für CR$^5$=N-Y$^1$-R$^6$ steht.

Insbesondere sind im Hinblick auf die Verwendung gegen tierische Schädlinge und Schadpilze Verbindungen der Formel I.1 bevorzugt,

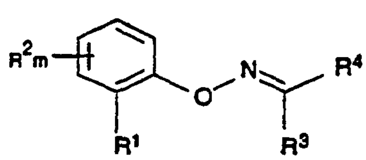

(I.1)

in der die Substituenten und der Index die folgende Bedeutung haben:

$R^1$ — C $(CO_2CH_3)=CHCH_3$, C $(CO_2CH_3)=CHOCH_3$, C $(CO_2CH_3)=NOCH_3$ oder C $(CONHCH_3)=NOCH_3$;

$R^2$ — Cyano, Halogen, $C_1$-$C_4$-Alkyl, $C_1$-$C_2$-Halogenalkyl, $C_1$-$C_4$-Alkoxy, $C_1$-$C_2$-Halogenalkoxy und $C_3$-$C_6$-Cycloalkyl;

m — 0 oder 1;

$R^3$ — Wasserstoff, Hydroxy, Cyano,
$C_1$-$C_4$-Alkyl, $C_1$-$C_2$-Halogenalkyl, $C_1$-$C_4$-Alkoxy, $C_1$-$C_2$-Halogenalkoxy, $C_3$-$C_6$-Cycloalkyl, $C_3$-$C_6$-Cycloalkyl-$C_1$-$C_4$-alkyl, oder Benzyl, welches durch übliche Gruppen substituiert sein kann;

$R^4$ — Wasserstoff, $C_3$-$C_6$-Cycloalkyl,
$C_1$-$C_6$-Alkyl oder $C_1$-$C_6$-Alkoxy, welches partiell oder vollständig halogeniert sein kann und/oder eine bis drei (insbesondere eine) der folgenden Gruppen tragen kann: Cyano, $C_1$-$C_4$-Alkoxy, $C_3$-$C_6$-Cycloalkyl, unsubstituiertes oder durch übliche Gruppen substituiertes Phenyl, Naphthyl, Pyridinyl, Pyrazinyl, Thienyl, Pyrazolyl oder Isoxazolyl;
unsubstituiertes oder durch übliche Gruppen substituiertes Phenyl, Naphthyl, Pyridinyl, Pyrazinyl, Thienyl, Pyrazolyl oder Isoxazolyl;
unsubstituiertes oder durch übliche Gruppen substituiertes Phenoxy, Naphthyloxy, Pyridinyloxy, Pyrazinyloxy, Thienyloxy, Pyrazolyloxy oder Isoxazolyloxy; oder

$R^3$ und $R^4$ — gemeinsam mit dem Kohlenstoffatom, an das sie gebunden sind, ein gesättigter oder partiell ungesättigter, vier- bis achtgliedriger Ring, welcher neben Kohlenwasserstoff-Ringgliedern ein Heteroatom aus der Gruppe Sauerstoff, Schwefel und Stickstoff enthalten kann und welcher partiell halogeniert sein kann und/oder eine bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, Oxo (=O) und $C_1$-$C_4$-Alkoxyimino (=N-Alkoxy) wobei

$R^3$ und $R^4$ — nicht gleichzeitig über Heteroatome an das Kohlenstoffatom gebunden sind;

Insbesondere sind im Hinblick auf die Verwendung gegen tierische Schädlinge und Schadpilze außerdem Verbindungen der Formel I.3 bevorzugt,

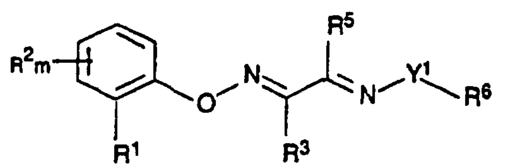

(I.3)

in der die Substituenten und der Index die folgende Bedeutung haben:

$R^1$ — C $(CO_2CH_3)=CHCH_3$, C $(CO_2CH_3)=CHOCH_3$, C $(CO_2CH_3)=NOCH_3$ oder C $(CONHCH_3)=NOCH_3$;

$R^2$ — Cyano, Halogen, $C_1$-$C_4$-Alkyl, $C_1$-$C_2$-Halogenalkyl, $C_1$-$C_4$-Alkoxy, $C_1$-$C_2$-Halogenalkoxy und $C_3$-$C_6$-Cycloalkyl;

m — 0 oder 1;

$R^3$ — Wasserstoff, Cyano, Halogen, $C_1$-$C_4$-Alkyl, $C_1$-$C_2$-Halogenalkyl, $C_1$-$C_4$-Alkoxy, $C_1$-$C_2$-Halogenalkoxy und $C_3$-$C_6$-Cycloalkyl;

$R^5$ — Wasserstoff, Cyano, Halogen, $C_1$-$C_4$-Alkyl, $C_1$-$C_2$-Halogenalkyl, $C_1$-$C_4$-Alkoxy, $C_1$-$C_2$-Halogenalkoxy und $C_3$-$C_6$-Cycloalkyl;

$Y^1$ — O, NH oder $N(CH_3)$;

$R^6$ — Wasserstoff,
$C_1$-$C_6$-Alkyl, welches eine der folgenden Gruppen tragen kann: $C_1$-$C_4$-Alkoxy, unsubstituiertes oder durch übliche

Gruppen substituiertes $C_3$-$C_6$-Cycloalkyl oder unsubstituiertes oder durch übliche Gruppen substituiertes Phenyl;
$C_3$-$C_6$-Alkenyl, $C_3$-$C_6$-Halogenalkenyl, $C_3$-$C_6$-Alkinyl, $C_3$-$C_6$-Halogenalkinyl oder unsubstituiertes oder durch übliche Gruppen substituiertes $C_3$-$C_6$-Cycloalkyl.

Insbesondere sind im Hinblick auf die Verwendung gegen tierische Schädlinge und Schadpilze desweiteren Verbindungen der Formel I.5 bevorzugt,

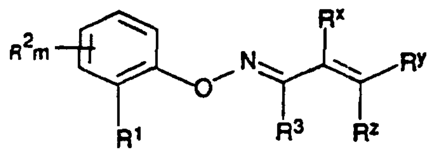

(I.5)

in der die Substituenten und der Index die folgende Bedeutung haben:

| | |
|---|---|
| $R^1$ | C $(CO_2CH_3)$=$CHCH_3$, C $(CO_2CH_3)$=$CHOCH_3$, C $(CO_2CH_3)$=$NOCH_3$ oder C $(CONHCH_3)$=$NOCH_3$; |
| $R^2$ | Cyano, Halogen, $C_1$-$C_4$-Alkyl, $C_1$-$C_2$-Halogenalkyl, $C_1$-$C_4$-Alkoxy, $C_1$-$C_2$-Halogenalkoxy und $C_3$-$C_6$-Cycloalkyl; |
| m | 0 oder 1; |
| $R^3$ | Wasserstoff, Cyano, Halogen, $C_1$-$C_4$-Alkyl, $C_1$-$C_2$-Halogenalkyl, $C_1$-$C_4$-Alkoxy, $C_1$-$C_2$-Halogenalkoxy und $C_3$-$C_6$-Cycloalkyl; |
| $R^x$ | Wasserstoff, Halogen, $C_1$-$C_4$-Alkyl, $C_1$-$C_2$-Halogenalkyl, $C_1$-$C_4$-Alkoxy, $C_1$-$C_2$-Halogenalkoxy und $C_3$-$C_6$-Cycloalkyl; |
| $R^y$ | Wasserstoff,<br>$C_1$-$C_6$-Alkyl, welches partiell oder vollständig halogeniert sein kann und/oder eine bis drei (insbesondere eine) der folgenden Gruppen tragen kann: $C_1$-$C_4$-Alkoxy, unsubstituiertes oder durch übliche Gruppen substituiertes $C_3$-$C_6$-Cycloalkyl oder unsubstituiertes oder durch übliche Gruppen substituiertes Phenyl;<br>$C_1$-$C_6$-Alkoxyl $C_1$-$C_6$-Halogenalkoxy,<br>unsubstituiertes oder durch übliche Gruppen substituiertes $C_3$-$C_6$-Cycloalkyl; unsubstituiertes oder durch übliche Gruppen substituiertes Phenyl, unsubstituiertes oder durch übliche Gruppen substituiertes Pyridyl oder unsubstituiertes oder durch übliche Gruppen substituiertes Pyrimidyl; |
| $R^z$ | Wasserstoff, Cyano, Halogen, $C_1$-$C_4$-Alkyl, $C_1$-$C_2$-Halogenalkyl und $C_3$-$C_6$-Cycloalkyl, oder |
| $R^y$ und $R^x$ | gemeinsam mit der Doppelbindung, an die sie gebunden sind, $C_4$-$C_6$-Cycloalkenyl. |

Es ist bekannt, daß Inhibitoren der motochondrialen Atmung von Pilzen als Fungizide eingesetzt werden können, z.B. Antimycin (vgl. "Inhibitors of Mitochondrial Function", Hrsg.: Rieske et al.; Pergamon Press, Oxford 1981, S. 110). Der praktischen Anwendung von Antimycin stehen jedoch verschiedene Nachteile im Wege, beispielsweise die schwierige und aufwendige Herstellung und die mangelnde Stabilität unter den Bedingungen der Anwendung, z. B. im Pflanzenschutz.

Die mitochondriale Atmung ist für den Stoffwechsel essentiell. In den Mitochondrien wird mit Hilfe der Atmung Energie in Form von Adenosintriphosphat (ATP) gespeichert. Die Hemmung der mitochondrialen Atmung führt letztlich zur Hemmung der ATP-Bildung.

Die erfindungsgemäßen Verbindungen I hemmen beispielsweise die mitochondriale Atmung von Schadpilzen. Bei den mit den erfindungsgemäßen Verbindugen I behandelten Pilzen kommen der ATP-Stoffwechsel bzw. energieabhängige Stoffwechselprozesse zum Stillstand, wodurch das Wachstum der Pilze zum erliegen kommt und die Pilze absterben.

In entsprechender Weise wird die mitochondriale Atmung von tierischen Schädlingen gehemmt.

Die Verbindungen I eignen sich daher zur Bekämpfung von tierischen Schädlingen, insbesondere aus der Klasse der Insekten, Spinnentiere und Nematoden, und von Schadpilzen.

Die Verbindungen I zeichnen sich durch eine hervorragende Wirksamkeit gegen ein breites Spektrum von pflanzenpathogenen Pilzen, insbesondere aus der Klasse der Ascomyceten, Deuteromyceten, Phycomyceten und Basidiomyceten, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Gemüse, Zierpflanzen und Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Pseudocercosporella-Arten in Hopfen und Gurken, Alternaria-Arten an Gemüse und Obst, sowie Mycosphaerella-Arten in Bananen.

Die Verbindungen I eignen sich außerdem zur Bekämpfung von Schadpilzen im Materialschutz (z.B. Holz, Papier, Dispersionen für den Ansprich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:

Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;

Nitroderivate, wie Dinitro-(1-methylheptyl)-phenylcrotonat, 2-sec-Butyl-4,6-dinitrophenyl-3,3-dimethylacrylat,

2-sec-Butyl-4,6-dinitrophenyl-isopropylcarbonat, 5-Nitro-isophthalsäure-diisopropylester;

heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl(4))-benzimidazol, N-(1,1,2,2-Tetrachlorethylthio)-tetrahydrophthalimid, N-Trichlormethylthio-tetrahydrophthalimid, N-Trichlormethylthio-phthalimid,

N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw, dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-di-ylbis-(1-(2,2,2-trichlor-ethyl)-formamid, 1-(3,4-Dichloranilino)-1-formylamino-2,2,2-trichlorethan, 2,6-Dimethyl-N-tridecylmorpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol,1-[2-(2,4-Dichlorphenyl)-4-n-propyl-1,3-dioxolan-2-yl-ethyl]-1H-1,2,4-triazol,N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,

Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,

Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,

Phenylpyrrole wie 4-(2,2-Difluor-1,3-benzodioxol-4-yl)-pyrrol-3-carbonitril,

Zimtsäureamide wie 3-(4-Chlorphenyl)-3-(3,4-dimethoxyphenyl)-acrylsäuremorpholid,

sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria,

Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus. Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femurrubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus

similis, Rotylenchus robustus, Trichodorus primitives, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff zur Bekämpfung von Scbädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:

I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.

II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).

III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).

IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl

besteht (Wirkstoffgehalt 16 Gew.-%).

V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).

VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).

VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

Die chemischen Verschiebungen (in ppm) der $^1$H-NMR-Spektren wurde gemessen gegen Tetramethylsilan (br = breites Signal, s = Singulett, d = Dublett, m = Multiplett).

1. Herstellung der Zwischenprodukte:

1.a Synthese von 2-Fluorbenzaldehydcyanhydrin

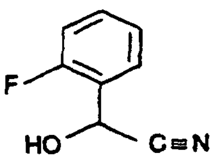

Zur Suspension von 179 g Kaliumcyanid in 500 ml Diethylether tropft man unter Rühren bei Raumtemperatur zunächst 171 g 2-Fluorbenzaldehyd zu, sodann unter Eiskühlung innerhalb von 5 min eine Lösung von 148 g Ammoniumchlorid in 600 ml Wasser. Nach 2 h Rühren bei Raumtemperatur (ca. 25°C) werden die Phasen getrennt, die Etherphase einmal mit verdünnter NaCl-Lösung und dreimal mit $H_2O$ gewaschen, getrocknet und eingeengt.

Man erhält 191 g (91 %) der Titelverbindung als Öl.
$^1$H-NMR ($CDCl_3$): 4,20 (s, br, 1H, OH); 5,75 (s, 1H, CHOH); 7,05-7,75 (m, 4H, Phenyl-H).
1.b Synthese von 2-Fluormandelsäuremethylester-imid-hydrochlorid

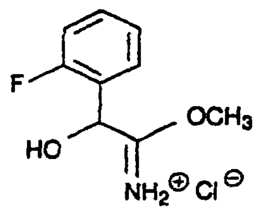

235 g des Cyanhydrins aus **1.a** werden in 1000 ml Diethylether und 100 g Methanol gelöst. Dazu werden unter Eiskühlung innerhalb von 15 min 126 g einer 55 proz. Lösung von HCl in Ether ($\hat{=}$ 69 g HCl) getropft. Nach 20 h Stehen bei Raumtemperatur (ca. 25°C) wird der Niederschlag abfiltriert und im Stickstoffstrom trockengeblasen. Ausbeute 108 g der Titelverbindung.
$^1$H-NMR [$D_6$-Dimethylsulfoxid ($D_6$-DMSO)]: 3,05 (s, 3H, $OCH_3$); 5,12 (s, 1H, CHOH); 6,25 (s, br, 1H, CHOH); 7,10-7,60 (m, 4H, Phenyl-H).
1.c Synthese von 2-Fluormandelsäuremethylester

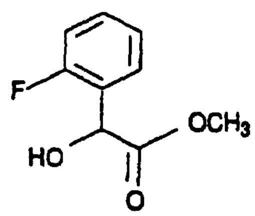

Das Produkt aus **1.b** (108 g) wird eine halbe Stunde in 450 ml $H_2O$ unter Rühren auf Rückfluß erhitzt. Nach Abkühlen wird dreimal mit Ether extrahiert, die Etherphase getrocknet und eingeengt. Man erhält 77 g der Titelverbindung.
$^1$H-NMR ($D_6$-DMSO): 3,62 (s, 3H, $OCH_3$); 5,40 (s, 1H, CHOH); 6,30 (s, br, 1H, CHOH); 7,10-7,40 (m, 4H, Phenyl-H).
1.d Synthese von 2-Fluorphenylglyoxylsäuremethylester

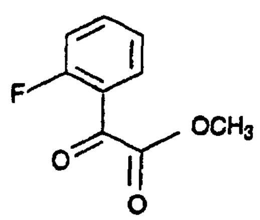

Zu 77,1 g des Produktes aus **1.c** in 700 ml $CH_2Cl_2$ gibt man 3,2 g KBr, 8,7 g $Na_2HPO_4$, 7,6 g $NaH_2PO_4$ x2$H_2O$, 2,8 g 2,2,6,6-Tetramethylpiperidin-N-oxid sowie 600 ml $H_2O$. Zu dieser Mischung werden unter Rühren bei 20°C innerhalb 1/2 h 343 g einer 12,5 proz. NaOCl-Lösung zugetropft. Man rührt noch 1 h weiter. Die organische Phase wird abgetrennt, die Wasserphase einmal mit $CH_2Cl_2$ nachextrahiert. Die vereinigten organischen Phasen werden dreimal mit $H_2O$ gewaschen, getrocknet und eingeengt. Man erhält 72,3 g der Titelverbindung.
$^1$H-NMR ($CDCl_3$): 3,97 (s, 3H, $OCH_3$), 7,08 (dd, 1H, Phenyl-H), 7,16 (dd, 1H, Phenyl-H), 7,33 (dd, 1H, Phenyl-H), 7,93 ppm (dd, 1H, Phenyl-H).
1.e Synthese von 2-Fluorphenylglyoxylsäuremethylamid

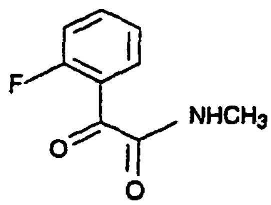

Eine Lösung aus 36g (0,2 mol) des Produktes aus **1.d** in 150 ml Tetrahydrofuran wurde bei ca. 25°C mit 17,1g (0,22 mol = 1,1 äq) Methylamin-Lösung (40%-ig in Wasser) versetzt. Nach 3h unter Rühren wurde das Lösungsmittel bei vermindertem Druck entfernt. Der so erhaltene Rückstand kristallisierte aus. Man erhielt so 30,0g der Titelverbindung (Fp.: 98-100°C).

$^1$H-NMR ($CDCl_3$) : 3,01 (d, 3H, $NCH_3$); 6,98 (d, 1H, NH); 7,05-7,18 (m, 2H, Phenyl-H); 7,60 (dd, 1H, Phenyl-H); 7,94 (t, 1H, Phenyl-H).

1.f Synthese von Butan-2,3-dion O-methyloxim

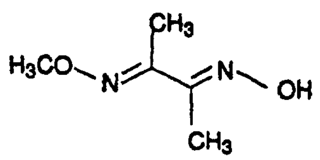

Zu 18,7 g (E) -Diacetylmonoxim in 100 ml Methanol gibt man 43,8 g Pyridin und tropft anschließend unter Rühren 17 g o-Methylhydroxylaminhydrochlorid, gelöst in 50 ml Methanol zu. Nach 3 Stunden wird bis zur Trockene eingeengt, der Rückstand in Ether aufgenommen und mit verd. Salzsäure gewaschen, sodann die Etherphase getrocknet und eingeengt. Man erhält 24 g der Titelverbindung.

$^1$H-NMR ($CDCl_3$): 2,01 + 2,09 (2 x s, 6H, $CH_3$); 3,98 (s, 3H, $OCH_3$); 8,59 (s, 1H, OH).

1.g Synthese von 3-Methyl-pent-3-en-2-on oxim

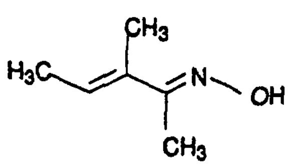

Eine Lösung aus 50g (0,5 mol) 3-Methylpent-3-en-2-on in 500 ml Methanol wurde mit 80g (1 mol) Pyridin und 35g (0,5 mol) Hydroxylamin-hydrochlorid versetzt. Nach 12h rühren bei Raumtemperatur (ca. 25°C) wurde die Reaktionsmischung bei vermindertem Druck vom Lösungsmittel befreit. Der Rückstand wurde in tert.-Butyl-methylether aufgenommen. Die organische Phase wurde mit Wasser und verdünnter Salzsäure gewaschen, getrocknet und bei vermindertem Druck eingeengt. Man erhielt 48,0g der Titelverbindung als weiße Kristalle (Fp.: 70-72°C).

$^1$H-NMR ($CDCl_3$): 1,78 (d, 3H, $CH_3$); 1,85 (s, 3H, $CH_3$); 2,04 (s, 3H, $CH_3$); 6,01 (q, 3H, =CH); 9,98 (s, 1H, OH).

1.h Synthese von 2-Oxo-[2-(1-meta-tolyl-ethylidenaminooxy)-phenyl]-essigsäuremethylester

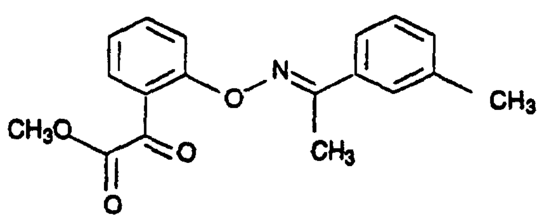

Zur Mischung von 4,0 g 3-Methylacetophenonoxim in 20 ml N,N-Dimethylacetamid (DMA) gibt man bei 20°C 3,0 g Kalium-tert.-butanolat, läßt 1/2 Stunde rühren und tropft dann bei 15°C ein Gemisch von 5,9 g 2-Fluorphenylglyoxalsäure-methylester in 20 ml DMA zu. Nach 3 Stunden bei 20°C wird genügend Methyl-tert.-butylether und wäßrige $NH_4Cl$-Lösung zugegeben, die Phasen getrennt und die org. Phase dreimal mit $NH_4Cl$-Lösung gewa-

schen, sodann getrocknet und eingeengt. Man erhält nach Säulenchromatographie an Kieselgel 4,1 g der Titelverbindung.

$^1$H-NMR ($CDCl_3$) : 2,40 + 2,41 (2 x s, 6H, $CH_3$); 3,85 (s, 3H, $OCH_3$); 7,14 (m, 1H, Phenyl-H); 7,22-7,63 (m, 6H, Phenyl-H); 7,80 (dd, 1H, Phenyl-H).

1.i Synthese von [2-(2-Methoxyimino-1-methylpropylidenaminooxy)-phenyl]-oxo-essigsäuremethylester

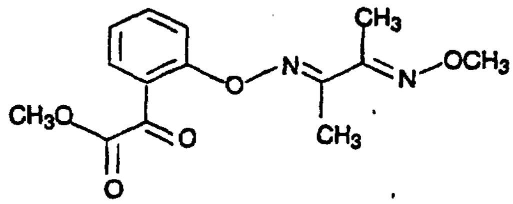

2,3 g des Produktes aus **1.f** in 15 ml DMA versetzt man mit 2,0 g Kalium-tert.-butanolat, rührt 1/2 Stunde und tropft dann bei 20°C 3 g des 2-Fluorphenyl-glyoxalsäuremethlesters aus **1.d**, gelöst in 6 ml DMA (Dimethylformamid) zu, wobei die Temperatur durch Eiskühlung bei 20°C gehalten wird. Danach wird nach 2 Stunden weiter gerührt. Zur Aufarbeitung wird das Reaktionsgemisch auf $NH_4Cl$-Lösung gegeben und die wäßrige Phase mehrmals mit Ether extrahiert. Die vereinigten organischen Phasen werden mit Ammoniumchloridlösung gewaschen, getrocknet und eingeengt. Man erhält 3,9 g der Titelverbindung.

$^1$H-NMR ($CDCl_3$): 2,09 + 2,22 (2 x s, 6H, $CH_3$); 3,88 + 4,01 (2 x s, 6H, $OCH_3$); 7,50 (dd, 2H, Phenyl-H); 7,82 (d, 1H, Phenyl-H).

1.j Synthese von 2-Oxo-[2-(2-Methoxyimino-1-methylpropylidenaminooxy)-phenyl]-essigsäure-N-methylamid

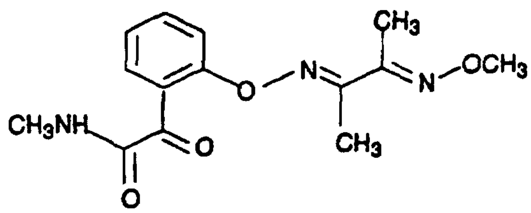

Eine Mischung aus 1,4g (10,5 mmol) des Produkts aus **1.f**, 1,2g (11 mmol) Kalium-tert.-butanolat und 1,8g (10 mmol) des Produkts aus **1.e** in 30 ml Dimethylacetamid wurde 1h bei 60°C gerührt. Die so erhaltene Reaktionsmischung wurde auf Eiswasser gegossen. Die wäßrige Mischung wurde zweimal mit $CH_2Cl_2$ extrahiert. Die vereinigten organischen Phasen wurden mit $Na_2SO_4$ getrocknet und anschließend bei vermindertem Drcuk eingeengt. Man erhielt 1,3g der Titelverbindung als braunes Öl.

$^1$H-NMR ($CDCl_3$): 2,02 + 2,20 (2 x s, 6H, $CH_3$); 2,91 (d, 3H, $NCH_3$); 3,98 (s, 3H, $OCH_3$); 6,89 (d, 1H, NCH); 7,09 (d, 1H, Phenyl-H); 7,31 (t, 1H, Phenyl-H); 7,49 (t, 1H, Phenyl-H); 7,65 (d, 1H, Phenyl-H).

1.k Synthese von 2-Oxo-[2-(1-ethoxyethylidenaminooxy)-phenyl]-essigsäuremethylester

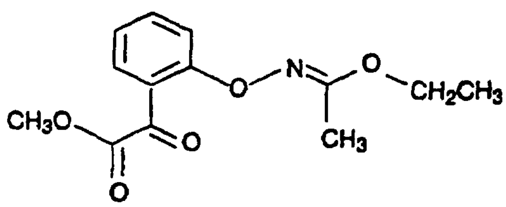

Eine Lösung aus 22,7g 1-Ethoxy-ethylaminoxim in 300 ml Toluol wurde mit 24,7g (0,22 mol) Kalium-tert.-butanolat versetzt und anschließend 2h gerührt. Die so erhaltene Mischung wurde bei 40°C und vermindertem Druck eingeengt und der so erhaltene Rückstand wurde in 500 ml Dimethylformamid aufgenommen.

Die so erhaltene Lösung wurde bei 15°C tropfenweise mit einer Lösung aus 40,1g (0,22 mol) in 250 ml Dimethylformamid versetzt. Nach 3h wurde die erhaltene Reaktionsmischung mit Eiswasser versetzt und dreimal mit ter. -Butyl-methylether extrahiert. Die vereinigten organischen Phasen wurden über $Na_2SO_4$ getrocknet und bei vermindertem Druck eingeengt. Das zurückbleibende Öl wurde säulenchromatographisch [an Kieselgel mit Cyclohexan/Essigsäureethylester (10:1)] gereinigt. Man erhielt 30g der Titelverbindung als Öl.

$^1$H-NMR ($CDCl_3$): 1,28 (t, 3H, $CH_3$); 2,08 (s, 3H, $CH_3$); 3,83 + 3,88 (2 x s, 6H, $OCH_3$); 4,18 (q, 2H, $OCH_2$); 7,05 (t, 1H, Phenyl-H); 7,38 (d, 1H, Phenyl-H); 7,54 (t, 1H, Phenyl-H); 7,76 (d, 1H, Phenyl-H).

1.l Synthese von 2-Oxo-[2-(3-methyl-pent-3-en-2-ylideniminooxy)-phenyl]-essigsäuremethylester

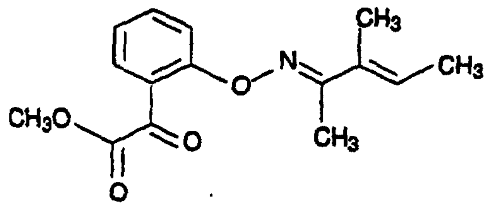

Eine Mischung aus 3,0g (20 mmol) des Produktes aus **1.g**, 2,2g (20 mmol) Kalium-tert.-butanolat und 20 ml Methanol wurde nach 10 min rühren bei ca. 25°C bei vermindertem Druck zur Trockene eingeengt.
Der Rückstand wurde in 20 ml Dimethylformamid aufgenommen, mit einer Lösung aus 3,5g (20 mmol) des Produkts aus **1.d** und 10 ml Dimethylformamid versetzt und die erhaltene Mischung wurde 2h bei 50°C gerührt. Die Reaktionsmischung wird mit Wasser versetzt und mit tert.-Butyl-methylether extrahiert. Die vereinigten organischen Phasen werden getrocknet und bei vermindertem Druck eingeengt. Der Rückstand (4,0g Öl) wurde säulenchromatographisch [Kieselgel, Cyclohexan/Essigsäureethylester (10->5/1)]gereinigt. Man erhielt so 2,0g der Titelverbindung als Öl.
$^1$H-NMR ($CDCl_3$): 1,82 (d, 3H, $CH_3$); 1,90 + 2,04 (2 x s, 6H, $CH_3$); 3,85 (s, 3H, $OCH_3$); 6,14 (q, 1H, =CH); 7,10 (t, 1H, Phenyl-H); 7,52 (m, 2H, Phenyl-H); 7,80 (d, 1H, Phenyl-H).

2. Herstellung der Verbindungen I:

2.a Synthese von Methoxyimino-[2-(1-meta-tolyl-ethylidenaminooxy)-phenyl]-essigsäuremethylester

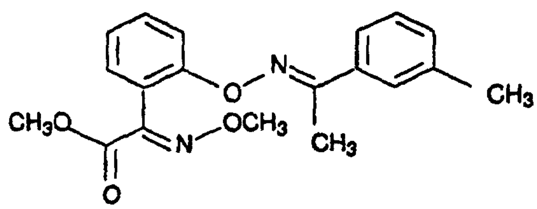

0,8 g des Ketoesters aus **1.h** werden in 7 ml $CH_3OH$ mit 0,24 g o-Methylhydroxylaminhydrochlorid und 0,23 g Pyridin bei 50°C 1 Stunde gerührt. Danach wird das Methanol im Vak. entfernt und der Rückstand mit Essigsäureethylester aufgenommen, mit verd. HCl sowie dann mit Wasser gewaschen, getrocknet und eingeengt. Rohausbeute 6,3 g, die allmählich kristallisieren. Nach säulenchromatographischer Reinigung isoliert man aus der zweiten Fraktion 2,2 g der Titelverbindung; Fp. 90°C.
$^1$H-NMR ($CDCl_3$): 2,32 + 2,34 (2 x s, 6H, $CH_3$); 3,82 + 4,09 (2 x s, 6H), $OCH_3$); 7,10 (t, 1H, Phenyl-H); 7,21-7,61 (m, 7H, Phenyl-H).

2.b Synthese von 2-Methoxyimino-N-Methyl-2-[2-(1-meta-tolylethylidenaminooxy)-phenyl]-essigsäuremethylamid

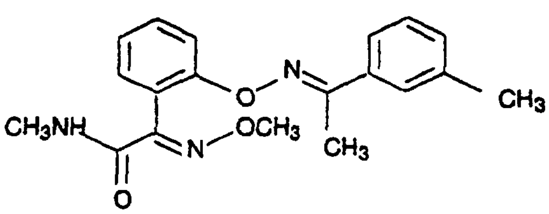

Zu 0,5 g des Oximethers aus **2.a** in 4 ml Tetrahydrofuran werden bei Rückflußtemperatur portionsweise 2,9 ml 40 proz. wäßrige Methylaminlösung gegeben. Über Nacht wird die Mischung weiter gerührt, dann bei vermindertem Druck eingeengt, in Essigester aufgenommen, mit NaCl-Lösung gewaschen, getrocknet und eingeengt. Man erhält

0,4 g der Titelverbindung; blaßgelbe Kristalle, Fp. 109-111°C.

$^1$H-NMR ($CDCl_3$): 2,35 + 2,40 (2 x s, 6H, $CH_3$); 2,92 (d, 3H, $NCH_3$); 4,00 (s, 3H, $OCH_3$); 6,68 (d, 1H, NH); 7,10 (t, 1H, Phenyl-H); 7,22-7,62 (m, 7H, Phenyl-H).

2.c Synthese von Methoxyimino-[2-(2-methoxyimono-1-methyl-propylidenaminooxy)-phenyl]-essigsäuremethylester

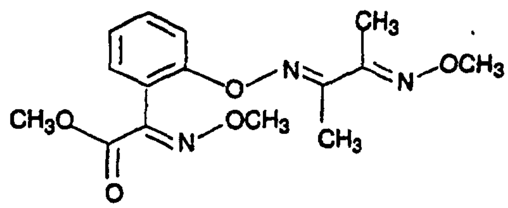

3 g des Rohproduktes aus **1.i** in 20 ml Methanol werden mit 0,83 g o-Methylhydroxylamin bei Raumtemperatur (ca. 25°C) umgesetzt. Nach 3 Stunden wird das Lösungsmittel i. Vak. angezogen und der Rückstand einer Säulenchromatographie an Kieselgel mit Laufmittel Cyclohexan/Ethylacetat 15 : 1 unterzogen. Als zweite Fraktion wurden 1,3 g der Titelverbindung vom Fp. 93-94°C isoliert.

$^1$H-NMR ($D_6$-DMSO): 2,01 + 2,04 (2 x s, 6H, $CH_3$); 3,71 (s, 3H, $OCH_3$); 3,95 (s, 6H, $OCH_3$); 7,12 (m, 1H, Phenyl-H); 7,31 (d, 1H, Phenyl-H); 7,43 (d, 2H, Phenyl-H).

2.d Synthese von Methoxyimino-[2-(2-methoxyimono-1-methyl-propylidenaminooxy)-phenyl]-essigsäuremethylamid

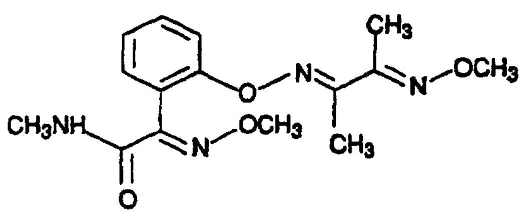

Verfahren A:

Das Produkt aus **2.c** (0,6 g) wurde in 10 ml Tetrahydrofuran wird mit 3,6 g 40 proz. wäßr. Methylaminlösung über Nacht bei Raumtemperatur (ca. 25°C) gerührt. Nach Abziehen des Lösungsmittels bei vermindertem Druck und Zugabe von Wasser,

Isolierung und Trocknen des entstandenen kristallinen Niederschlags erhielt man 0,3 g der Titelverbindung (Fp. 122-123°C).

$^1$H-NMR ($CDCl_3$): 2,05 + 2,12 (2 x s, 6H, $CH_3$); 2,90 (d, 3H, $NCH_3$); 3,92 + 3,98 (2 x s, 6H, $OCH_3$); 6,70 (m, 1H, NH); 7,07 (t, 1H, Phenyl-H); 7,22 (d, 1H, Phenyl-H); 7,38 (t, 1H, Phenyl-H); 7,49 (d, 1H, Phenyl-H).

Verfahren B:

Eine Lösung aus 1,1g (4 mmol) des Produkts aus **1.j** in 30 ml Methanol wurde mit 0,4g (4,2 mmol) O-Methylhydroxylamin-hydrochlorid und 0,5g (6 mmol) Pyridin versetzt. Nach 2h bei 50°C wurde das Lösungsmittel und die Base bei vermeindertem Druck entfernt. Der Rückstand wurde in Essigsäureethylester aufgenommen und die so erhaltenen Lösung wurde jeweils zweimal mit Wasser und verdünnter Salzsäure gewaschen. Die organische Phase wurde über $Na_2SO_4$ getrocknet und bei vermindertem Druck eingeengt. Man erhielt so 1,1g der Titelverbindung als gelbes Öl.

2.e Synthese von 3-Methoxy-[2-(2-methoxyimono-1-methyl-propylidenaminooxy)-phenyl]-acrylsäuremethylester

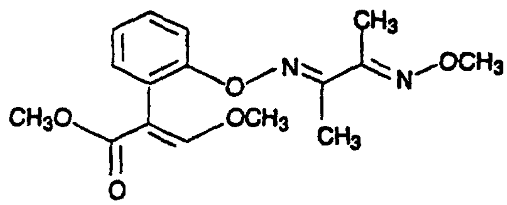

e) Das Rohprodukt von Herstellungsbeispiel **1.i**, und zwar 2,4 g davon, gelöst in 10 ml Dimethylformamid (DMF), wird zu einem Gemisch von 5,6 g Methoxymethyltriphenylphosphoniumchlorid und 2,5 g 30 proz. Natriummethylatlösung (in Methanol) in 20 ml DMF getropft. Nach 3 Stunden wird unter Eiskühlung 30 ml $H_2O$ zugetropft und der entstandene Niederschlag abgesaugt. nach Chromatographie über $SiO_2$ mit Cyclohexan/ Ethylacetat erhält man aus der zweiten Fraktion 1,8 g der Titelverbindung als blaßgelben Feststoff (Fp. 101°C).
$^1$H-NMR ($CDCl_3$): 2,09 + 2,11 (2 x s, 6H, $CH_3$); 3,65 + 3,82 (2 x s, 6H, $OCH_3$); 7,01 (t, 1H, Phenyl-H); 7,22 (m, 2H, Phenyl-H); 7,45 (d, 1H, Phenyl-H); 7,49 (s, 1H, CH).

2.f Synthese von 3-Methoxy-[2-(1-ethoxy-ethylidenaminooxy)-phenyl]-acrylsäuremethylester

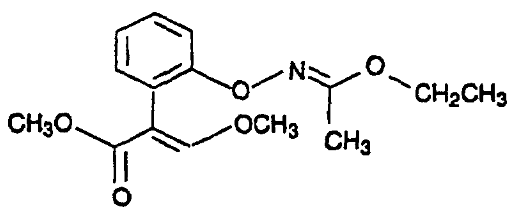

Eine Lösung aus 6,9g (0,02 mol) Methoxymethyltriphenylphosphonium-chlorid, 3,1g (0,017 mol) Natriummethanolat (30%-ig in Methanol) und 30 ml Dimethylformamid wurde tropfenweise mit einer Lösung aus 2,7g (0,01 mol) des Produkts aus **1.k** und 10 ml Dimethylformamid versetzt. Nach 3h wurde das Reaktionsgemisch auf Eiswasser gegeben und mit tert.-Butyl-methylether extrahiert. Die vereinigten organischen Phasen werden über $Na_2SO_4$ getrocknet und bei vermindertem Druck eingeengt. Der so erhaltene Rückstand wurde säulenchromatographisch [Kieselgel, Toluol/Essigsäureethylester (10/1)] gereinigt. Man erhielt so 1,9g der Titelverbindung als Öl.
$^1$H-NMR ($CDCl_3$): 1,26 (t, 3H, $CH_3$); 2,00 (s, 3H, $CH_3$); 3,65 (s, 3H, $OCH_3$); 3,80 (s, 3H, $OCH_3$); 4,18 (q, 2H, $OCH_2$); 6,95 (t, 1H, Phenyl-H); 7,20 (d, 1H, Phenyl-H); 7,30 (t, 1H, Phenyl-H); 7,43 (d, 1H, Phenyl-H); 7,50 (s, 1H, =CH·
2.g Synthese von 2-Methoxyimino-[2-(1-ethoxy-ethylidenaminooxy)-phenyl]-essigsäuremethylester

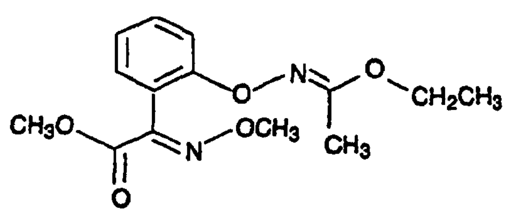

Eine Hischung aus 1,0g (4,0 mmol) des Produkts aus **1.k** und 5 ml Methanol wurde mit 0,25g O-Methylhydroxylamin versetzt und ca. 24h bei ca. 25°C gerührt. Die so erhaltene Reaktionsmischung wurde bei vermindertem Druck eingeengt uns der erhaltene Rückstand wurde in $CH_2Cl_2$ aufgenommen. Die organische Phase wurde mit Wasser gewaschen, getrocknet und bei vermindertem Druck eingeengt. Man erhielt 1,0g der Titelverbindung [2:1 Isomerengemisch].
$^1$H-NMR ($CDCl_3$): 1,26 (t, 3H, $CH_3$); 2,01 + 2,05 (2 x s, 6H, $CH_3$); 3,86 + 4,04 (2 x s, 6H, $OCH_3$); 4,10 (q, 2H, $OCH_2$); 6,94-7,50 (m, 4H, Phenyl-H).
2.h Synthese von 2-Methoxyimino-[2-phenylmethylidenaminooxy-phenyl]-essigsäuremethylester

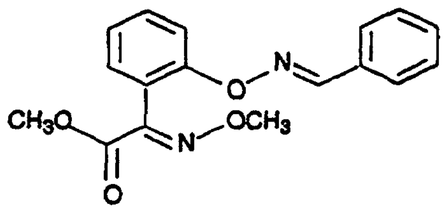

Eine Lösung aus 1,0g (3,4 mmol) des Produkts aus **1.k** und 5 ml Methanol wurde mit 1,8g (17 mmol) Benzaldehyd und 0,33g Methansulfonsäure versetzt und die so erhaltene Mischung wurde 1h gerührt. Die so erhaltene Lösung wurde bei vermindertem Druck eingeengt und der verbleibende Rückstand wurde in $CH_2Cl_2$ aufgenommen. Die organische Phase wurde mit Wasser gewaschen, getrocknet und eingeengt. Der dabei anfallende Rückstand wurde säulenchromatographisch [Toluol/Essigsäureethylester (10/1)] gereinigt. Man erhielt 400mg der Titelverbindung als Öl.

$^1$H-NMR ($CDCl_3$): 3,95 (s, 3H, $OCH_3$); 4,02 (s, 3H, $OCH_3$); 7,30-7,68 (m, 9H, Phenyl-H); 8,05 (s, 1H, =CH).

2.i Synthese von 2-[2-(3-methyl-pent-3-en-2-ylideniminooxy)-phenyl]-but-2-ensäuremethylester

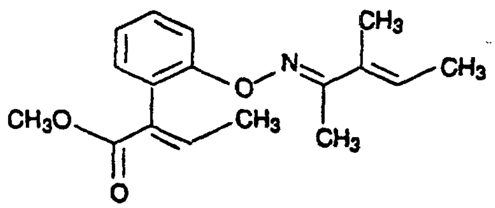

Eine Mischung aus 4,7g (14,5 mmol) Ethyl-triphenylphosphonium-chlorid in 20 ml Dimethylformamid wurde bei ca. 25°C mit 2,2g (12 mmol) Natriummethanolat-Lösung (30%-ig in Methanol) versetzt. Die so erhaltene Mischung wurde anschließend tropfenweise mit einer Lösung aus 2,0g des Produktes aus **1.l** in 10 ml Dimethylformamid versetzt und weitere 2h gerührt. Die Reaktionsmischung wurde danach mit Wasser versetzt und mit tert.-Butylmethylether extrahiert. Die vereinigten organischen Phasen wurden getrocknet und bei vermindertem Druck eingeengt. Der verbleibende Rückstand wurde säulenchromatographisch [Cyclohexan/Essigsäureethylester (10/1)] gereinigt. Man erhielt 700mg der Titelverbindung als Feststoff (Fp.: 90-93°C).

$^1$H-NMR ($CDCl_3$): 1,72 (d, 3H, $CH_3$); 1,80 (d, 3H, $CH_3$); 1,89 (s, 3H, $CH_3$); 2,03 (s, 3H, $CH_3$); 3,68 (s, 3H, $OCH_3$); 6,03 (pseudo-q, 1H, =CH); 6,98-7,35 (m, 5H, Phenyl-H, =CH); 7,50 (d, 1H, Phenyl-H).

**Tabelle I(1):**

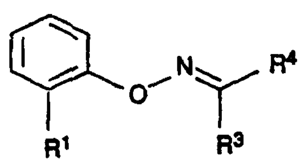

I(I)

| Nr. | $R^1$ | $R^3$ | $R^4$ | Physikalische Daten |
|---|---|---|---|---|
| I(1).1 | Ia | $CH_3$ | $3-CH_3-C_6H_4$ | 90°C |
| I(1).2 | Ib | $CH_3$ | $3-CH_3-C_6H_4$ | 109 - 111°C |
| I(1).3 | Ia | $CH_3$ | $4-CH_3-C_6H_4$ | 99°C |
| I(1).4 | Ib | $CH_3$ | $4-CH_3-C_6H_4$ | 109°C |
| I(1).5 | Id | $-CH_2-(CH_2)_3-CH_2-$ | | Öl |
| I(1).6 | Ia | $CH_3$ | $OC_2H_5$ | $^1$H: 1.32 (t,3H); 2.06 (s,3H); 3.92 (s,3H); 4.05 (s,3H); 4.08 (q,2H), 6.95-7.42 (m,4H) |

| Nr. | $R^1$ | $R^3$ | $R^4$ | Physikalische Daten |
|---|---|---|---|---|
| I(1).7 | Id | $CH_3$ | $OC_2H_5$ | $^1H$: 1.28 (t,3H); 2.03 (s,3H); 3.76 (s,3H); 3.84 (s,3H); 4.08 (q,2H); 6.98 (t,1H); 7.08 (d,1H); 7.21 (t,1H); 7.42 (d,1H); 7.50 (s,1H). |
| I(1).8 | Ia | 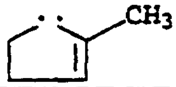 | | 128°C |
| I(1).9 | Ib | 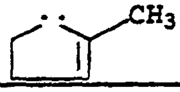 | | 135-137°C |

Tabelle I(2):

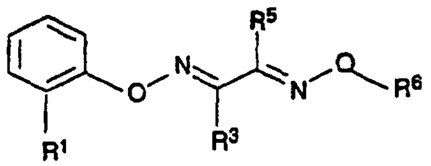

I(III)

| Nr. | $R^1$ | $R^3$ | $R^5$ | $R^6$ | Physikalische Daten |
|---|---|---|---|---|---|
| I(2).1 | Ia | $CH_3$ | 4-F-$C_6H_5$ | $CH_3$ | 112°C |
| I(2).2 | Ia | $CH_3$ | $CH_3$ | $CH_3$ | 93°C |
| I(2).3 | Ib | $CH_3$ | $CH_3$ | $CH_3$ | 122 - 123°C |
| I(2).4 | Id | $CH_3$ | 4-F-$C_6H_5$ | $CH_3$ | 141°C |
| I(2).5 | Ib | $CH_3$ | 4-F-$C_6H_5$ | $CH_3$ | 139°C |
| I(2).6 | Id | $CH_3$ | $CH_3$ | $CH(CH_3)_2$ | 103°C |
| I(2).7 | Id | $CH_3$ | $CH_3$ | $CH_3$ | 101°C |
| I(2).8 | Ib | $CH_3$ | $CH_3$ | $CH(CH_3)_2$ | 97°C |
| I(2).9 | Ia | $CH_3$ | $CH_3$ | $C_2H_5$ | 106 - 109°C |
| I(2).10 | Ib | $CH_3$ | $CH_3$ | $C_2H_5$ | 119 - 123°C |
| I(2).11 | Ia | $C_2H_5$ | $CH_3$ | $CH_3$ | 92 - 97°C |
| I(2).12 | Ib | $C_2H_5$ | $CH_3$ | $CH_3$ | 97 - 100°C |
| I(2).13 | Ia | $CH_3$ | $CH_3$ | $CH(CH_3)_2$ | 74°C |
| I(2).14 | Id | $C_2H_5$ | $CH_3$ | $CH_3$ | 1711, 1647, 1542, 1288, 1266, 1132 |
| I(2).15 | Id | $CH_3$ | $CH_3$ | $C_2H_5$ | 78 - 80°C |
| I(2).16 | Ia | $CH_3$ | $CH_3$ | $CH_2C\equiv CH$ | 51 - 53°C |
| I(2).17 | Id | $CH_3$ | $CH_3$ | $CH_2C\equiv CH$ | 59 - 63°C |
| I(2).18 | Ib | $CH_3$ | $CH_3$ | $CH_2C\equiv CH$ | 146 - 149°C |
| I(2).19 | Ib | $CH_3$ | $C_2H_5$ | $CH_3$ | 113°C |
| I(2).20 | Id | $CH_3$ | $C_2H_5$ | $CH_3$ | 1713, 1638, 1541, 1258, 1128, 1051. |

| Nr. | $R^1$ | $R^3$ | $R^5$ | $R^6$ | Physikalische Daten |
|---|---|---|---|---|---|
| I(2).21 | Ia | $CH_3$ | $C_2H_5$ | $CH_3$ | 1732, 1453, 1224, 1207, 1073, 1050. |
| I(2).22 | Ib | $C_2H_5$ | $CH_3$ | $C_2H_5$ | 101 - 103°C |
| I(2).23 | Id | $C_2H_5$ | $CH_3$ | $C_2H_5$ | 88 - 92°C |
| I(2).24 | Ia | $C_2H_5$ | $CH_3$ | $C_2H_5$ | 102°C |
| I(2).25 | Ib | $C_2H_5$ | $CH_3$ | $CH(CH_3)_2$ | 92 - 94°C |
| I(2).26 | Id | $C_2H_5$ | $CH_3$ | $CH(CH_3)_2$ | 74 - 78°C |
| I(2).27 | Ia | $C_2H_5$ | $CH_3$ | $CH(CH_3)_2$ | 93 - 96°C |
| I(2).28 | Ib | $C_2H_5$ | $CH_3$ | $CH_2C{\equiv}CH$ | 95 -100°C |
| I(2).29 | Id | $C_2H_5$ | $CH_3$ | $CH_2C{\equiv}CH$ | 1711, 1637, 1542, 1252, 1218, 1129. |
| I(2).30 | Ia | $C_2H_5$ | $CH_3$ | $CH_2C{\equiv}CH$ | 1731, 1480, 1453, 1227, 1207, 1072. |
| I(2).31 | Ib | $C_6H_5$ | $CH_3$ | $CH_3$ | 122 - 125°C |
| I(2).32 | Id | $C_6H_5$ | $CH_3$ | $CH_3$ | 116 - 120°C |
| I(2).33 | Ia | $C_6H_5$ | $CH_3$ | $CH_3$ | 107 - 113°C |
| I(2).34 | Ia | $CH_3$ | $OCH(CH_3)_2$ | $CH_3$ | 78°C |
| I(2).35 | Ib | $CH_3$ | $CH_2CH_3$ | $CH_2C{\equiv}CH$ | 100 - 103°C |
| I(2).36 | Ia | $CH_3$ | $CH_2CH_3$ | $CH_2C{\equiv}CH$ | 94 - 97°C |
| I(2).37 | Id | $CH_3$ | $CH_2CH_3$ | $CH_2C{\equiv}CH$ | Öl |
| I(2).38 | Ia | $CH_3$ | $CH_2CH_3$ | $CH_2CH_3$ | 91 - 95°C |
| I(2).39 | Ib | $CH_3$ | $CH_2CH_3$ | $CH_2CH_3$ | 91 - 93°C |
| I(2).40 | Id | $CH_3$ | $CH_2CH_3$ | $CH_2CH_3$ | Öl |
| I(2).41 | Ie | $CH_3$ | $CH_2CH_3$ | $CH_2CH_3$ | Öl |
| I(2).42 | Ia | $CH_3$ | $CH_2OCH_3$ | $CH_3$ | Öl |
| I(2).43 | Ib | $CH_3$ | $CH_2OCH_3$ | $CH_3$ | 95 -100°C |

Tabelle I(3):

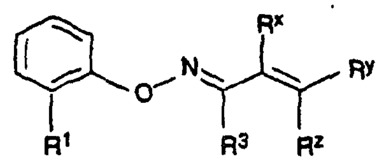

I(v)

| Nr. | $R^1$ | $R^3$ | $R^x$ | $R^y$ | $R^z$ | Physikalische Daten |
|---|---|---|---|---|---|---|
| I(3).1 | Ia | $CH_3$ | H | $C_6H_5$ | H | 102°C |
| I(3).2 | Ib | $CH_3$ | H | $C_6H_5$ | H | 155°C |
| I(3).3 | Ia | $CH_3$ | H | 4-F-$C_6H_5$ | H | 109°C |
| I(3).4 | Ib | $CH_3$ | H | 4-F-$C_6H_5$ | H | 72 - 75°C |
| I(3).5 | Ia | $CH_3$ | H | 4-Cl-$C_6H_5$ | H | 141°C |
| I(3).6 | Ib | $CH_3$ | H | 4-Cl-$C_6H_5$ | H | 147°C |
| I(3).7 | Ia | $CH_3$ | H | $CH_3$ | $CH_3$ | 58 - 60°C |

| Nr. | $R^1$ | $R^3$ | $R^x$ | $R^y$ | $R^z$ | Physikalische Daten |
|---|---|---|---|---|---|---|
| I(3).8 | Ib | $CH_3$ | H | $CH_3$ | $CH_3$ | 85 - 88°C |
| I(3).9 | Ib | $CH_3$ | $CH_3$ | $CH_3$ | H | 83 - 84°C |
| I(3).10 | Ib | $CH_3$ | $CH_3$ | $CH(CH_3)_2$ | H | 69 - 71°C |
| I(3).11 | Ia | $CH_3$ | $CH_3$ | $CH_3$ | H | 81 - 83°C |
| I(3).12 | Ia | $CH_3$ | $CH_3$ | $CH(CH_3)_2$ | H | 90 - 92°C |
| I(3).13 | Id | $CH_3$ | $CH_3$ | $CH_3$ | H | 90 - 93°C |
| I(3).14 | Id | $CH_3$ | $CH_3$ | $CH(CH_3)_2$ | H | 78 - 80°C |
| I(3).15 | Ie | $CH_3$ | $CH_3$ | $CH_3$ | H | 69 - 72°C |
| I(3).16 | Ie | $CH_3$ | $CH_3$ | $CH(CH_3)_2$ | H | 56 - 59°C |
| I(3).17 | Ia | $CH_3$ | H | H | $C_6H_5$ | 88°C |
| I(3).18 | Ia | $CH_3$ | $CH_3$ | $C_2H_5$ | H | 83 - 86°C |
| I(3).19 | Ib | $CH_3$ | $CH_3$ | $C_2H_5$ | H | 81 - 86°C |
| I(3).20 | Id | $CH_3$ | $CH_3$ | $C_2H_5$ | H | 77 - 79°C |
| I(3).21 | Ie | $CH_3$ | $CH_3$ | $C_2H_5$ | H | Öl |
| I(3).22 | Ia | $CH_3$ | $CH_3$ | $C_3H_7$ | H | 80 - 82°C |
| I(3).23 | Ib | $CH_3$ | $CH_3$ | $C_3H_7$ | H | 99 - 100°C |
| I(3).24 | Id | $CH_3$ | $CH_3$ | $C_3H_7$ | H | 74 - 76°C |
| I(3).25 | Ie | $CH_3$ | $CH_3$ | $C_3H_7$ | H | 54 - 56°C |
| I(3).26 | Ib | $CH_3$ | $CH_3$ | 4-F-$C_6H_4$ | H | 140 - 142°C |
| I(3).27 | Ia | $CH_3$ | $CH_3$ | $C_6H_5$ | H | 115 - 117°C |
| I(3).28 | Ia | $CH_3$ | $CH_3$ | 4-F-$C_6H_4$ | H | 103 - 105°C |
| I(3).29 | Id | $CH_3$ | $CH_3$ | $C_6H_5$ | H | 108°C |
| I(3).30 | Id | $CH_3$ | $CH_3$ | 4-F-$C_6H_4$ | H | 108°C |
| I(3).31 | Ie | $CH_3$ | $CH_3$ | $C_6H_5$ | H | 81 - 83°C |
| I(3).32 | Ie | $CH_3$ | $CH_3$ | 4-F-$C_6H_4$ | H | 82 - 84°C |

Tabelle VI:

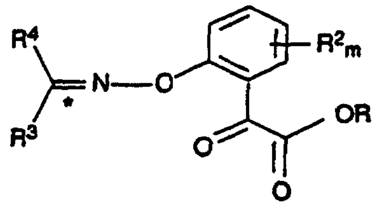

(VI)

| Nr. | $R^2_m$ | $R^3$ | $R^4$ | $^1$H-NMR ($CDCl_3$, ppm) | * |
|---|---|---|---|---|---|
| VI.1 | H | $CH_3$ | $3-CH_3-C_6H_4$ | 2,42 (3H,s), 2,44 (3H,s), 3,85 (3H,s), 7,1-7,9 (8H, vier Multipletts) | E |
| VI.2 | H | $CH_3$ | $C(CH_3)=NOCH_3$ | 2,1 (3H,s), 2,2 (3H,s), 3,75 (3H,s), 4,0 (3H,s), 7,1-7,9 (4H, vier Multipletts) | E,E |

* = Konfiguration der Doppelbindung

Beispiele zur Wirkung gegen Schadpilze

1. Wirkung der Verbindungen als Hemmstoffe der mitochondrialen Atmung

Um die Wirkung der erfindungsgemäßen Verbindungen auf die mitochondriale Atmung zu bestimmen, müssen zunächst die Mitochondrien aus den zu untersuchenden Organismen isoliert werden. Hierzu wird im Falle des Hefepilzes Saccharomyces cerevisiae eine 30- bis 40-%ige Zellsuspension des Pilzes in einer wäßrigen Lösung von 0,6 M Mannit + 0,01 M Tris-[hydroxymethyl]aminomethan + 0,002 M Ethylendiamintetraessigsäure-dinatriumsalz (pH 6,8) mit einer Glaskugelmühle aufgeschlossen und der Extrakt durch differentielle Zentrifugation bei 1000 x g und 12000 x g fraktioniert. Die Mitochondrien befinden sich im Niederschlag der 12000 x g-Zentrifugation.

Analog können auch Mitochondrien aus andern Pilzen isoliert werden, z.B. aus dem phytopathogenen Pilz Drechslera sorokiniana (synonym Helminthosphorium satioum), aus dem humanpathogenen Dermatophyten Trichophyton mentagrophytes sowie aus anderen Organismen, z.B. aus der Stubenfliege (musca domestica) als Insekt oder aus der Spinnmilbe Tetranychus urticae.

Die Wirkung der Verbindungen auf die Atmungskette wird bestimmt, indem zu einer Suspension von Mitochondrien in einer wäßrigen Lösung von 0,01 M Tris-[hydroxymethyl]aminomethan + 0,65 M Sorbit + 0,01 M $KH_2PO_4$ + 0,01 M KCl + 0,0004 M Ethylendiamintetraessigsäure-dinatriumsalz + 0,3 % Rinderserumalbumin + 0,0007 M KCN + 6 x $10^{-6}$ Ubichinon-50 + 0,01 M Na-Succinat + 0,15 % Cytochrom c (pH 7,5) eine Lösung der zu untersuchenden Verbindung in Dimethylsulfoxid gegeben wird und die Geschwindigkeit der Reduktion des Cytochrom c photometrisch anhand der Extinktionszunahme bei 546 nm bestimmt wird. Als Kontrolle dient ein Ansatz mit der entsprechenden Menge reinem DMSO. Der Hemmwert für die jeweils angegebene Wirkstoffkonzentration wird dann wie folgt berechnet.

$$\%\ \text{Hemmung} = 100 \cdot \frac{\left(\frac{\Delta E}{\Delta t}\right)_o - \left(\frac{\Delta E}{\Delta t}\right)_x}{\left(\frac{\Delta E}{\Delta t}\right)_o}$$

wobei ΔE die Extinktionsänderung, Δt die Zeitänderung,

$$\left(\frac{\Delta E}{\Delta t}\right)_o$$

die Umsatzgeschwindigkeit der Kontrolle und

$$\left(\frac{\Delta E}{\Delta t}\right)_x$$

die Umsatzgeschwindigkeit der Probe x bedeutet.

Die folgende Tabelle zeigt für eine Reihe von erfindungsgemäßen Verbindungen deren Wirkung als Atmungshemmer auf die Mitochondrien des Hefepilzes Saccharomyces cerevisiae, des phytopathogenen Pilzes Drechslera sorokiniana sowie der Stubenfliege musca domestica.

Die Hemmung der mitochondrialen Atmung ist dabei jeweils als $I_{50}$-Wert angegeben (dies ist die Konzentration an Testsubstanz, bei der 50 % Hemmung auftritt) und als F-Wert, dies ist ein relativer Wert, der wie folgt definiert ist:

$$F = \frac{I_{50}\ (\text{Testsubstanz})}{I_{50}\ (\text{Standardsubstanz})}$$

Als Standardsubstanz dient das sogenannte Enoletherstilben der folgenden Formel:

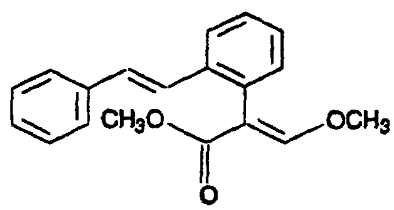

Tabelle II:

| Hemmung der mitochondrialen Atmung durch Verbindungen der Formel I aus den Tabellen I(1)-I(3) | | |
|---|---|---|
| Nr. | Saccharom. cerevisiae | |
| | $I_{50}$ (Mol/l) | F |
| I(1).1 | $2,7 \cdot 10^{-8}$ | 0,96 |
| I(1).2 | $3,2 \cdot 10^{-7}$ | 11 |
| I(2).7 | $3,3 \cdot 10^{-9}$ | 0,14 |
| I(2).2 | $1,2 \cdot 10^{-8}$ | 0,43 |
| I(2).3 | $2,7 \cdot 10^{-7}$ | 9,6 |

Die fungizide Wirkung der Verbindungen der allgemeinen Formel I gegen Schadpilze ließ sich durch folgende Gewächshausversuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

Wirkung gegen Erysiphe graminis var. tritici:

Blätter von Weizenkeimlingen (Sorte "Kanzler") wurden zunächst mit der wäßrigen Aufbereitung der Wirkstoffe (250 ppm-haltig) behandelt. Nach ca. 24h wurden die Pflanzen mit Sporen des Weizenmehltaus (Erysiphe graminis var. tritici) bestäubt. Die so behandelten Pflanzen wurden anschließend für 7 Tage bei 20-22°C und einer relativen Luftfeuchtigkeit von 75-80% inkubiert. Anschließend wurde das Ausmaß der Pilzentwicklung ermittelt.

In diesem Test zeigten die mit den erfindungsgemäßen Verbindungen behandelten Pflanzen einen Befall von 15% und weniger, die mit einem bekannten Wirkstoff (Verbindung Nr. 195, Tabelle 3, EP-A 463 488) behandelten Pflanzen 40% Befall und die unbehandelten Pflanzen 70% Befall.
In einem entsprechenden Test (Weizenkeimlinge der Sorte "Kanzler", Aufwandmenge 63 ppm), bei dem die Pflanzen zunächst infiziert und incubiert wurden und anschließend mit den Wirkstoffen behandelt wurden zeigten die mit den erfindungsgemäßen Verbindungen behandelten Pflanzen einen Befall von 5% und weniger, die mit einem bekannten Wirkstoff (Verbindung Nr. 195, Tabelle 3, EP-A 463 488) behandelten Pflanten 25% Befall und die unbehandelten Pflanzen 60% Befall.
In einem entsprechenden Test (Weizenkeimlinge der Sorte "Frühgold", Aufwandmenge 250 ppm), bei dem die Pflanzen zunächst mit den Wirkstoffen behandelt und anschließend infiziert und incubiert wurden zeigten die mit den erfindunsgemäßen Verbindungen behandelten Pflanzen einen Befall von 15% und weniger und die unbehandelten Pflanzen 75% Befall.

Wirkung gegen Plasmopara viticola *(Rebenperonospora)*

Topfreben (Sorte: "Müller Thurgau") wurden mit der Wirkstoffaufbereitung tropfnaß gespritzt (Aufwandmenge: 63 ppm). Nach 8 Tagen wurden die Pflanzen mit einer Zoosporenaufschwemmung des *Pilzes Plasmopara viticola* besprüht und 5 Tage bei 20-30°C bei hoher Luftfeuchtigkeit aufbewahrt. Vor der Beurteilung wurden die Pflanzen danach für 16h bei hoher Luftfeuchtigkeit aufbewahrt. Die Auswertung erfolgte visuell.
In diesem Test zeigten die mit den erfindungsgemäßen Wirkstoffen behandelten Pflanzen einen Befall von 15% und weniger, während die unbehandelten (Kontroll-) Pflanzen zu 70% befallen waren.

Beispiele zur Wirkung gegen tierische Schädlinge

Die Wirkung der Verbindungen der allgemeinen Formel I gegen Insekten, Spinnentiere und Nematoden ließ sich durch folgende Gewächshausversuche zeigen:
Die Wirkstoffe wurden

a) als 0,1%-ige Lösung in Aceton oder
b) als 10%-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL, Emulgator auf der Basis ethoxylierter Fettalkohole)

aufbereitet und entsprechend der gewünschten Konzentration mit Aceton im Fall von a) bzw. mit Wasser im Fall von b) verdünnt.
Nach Abschluß der versuche wurde die jeweils niedrigste Konzentration ermittelt, bei der die Verbindungen im Vergleich zu unbehandelten Kontrollversuchen noch eine 80 - 100%-ige Hemmung bzw. Mortalität hervorriefen (Wirkschwelle bzw. Minimalkonzentration).

Aphis fabae *(Schwarze Laus)*, Kontaktwirkung

Stark befallene Buschbohnen (Vicia faba) wurden mit der wässrigen Wirkstoffaufbereitung behandelt. Nach 24h wurde die Mortalitätsrate bestimmt.
In diesem Test zeigte die Verbindung I(2).9 eine Wirkschwelle von 200 ppm.

Heliothis virescens *(Baumwolleule)*, Kontakt-/Fraßwirkung

Ca. 10 cm große Tabakpflanzen wurden mit der wäßrigen Wirkstoffaufbereitung behandelt. Nach dem Abtrocknen wurden die Pflanzen mit jeweils 10 Larven des 3. Enwicklungsstadiums belegt. Nach 48h wurden Mortalität und Fraßverhinderung beurteilt.
In diesem Test zeigte die Verbindung I(2).17 eine Wirkschwelle von 40 ppm.

Nephotettix cincticeps *(Grüne Reiszikade)*, Kontaktwirkung

Rundfilter wurden mit der wäßrigen Wirkstoffaufbereitung behandelt und anschließend mit 5 adulten Zikaden belegt. Nach 24h wurde die Mortalität beurteilt.

In diesem Test zeigten die Verbindungen I(1).4, I(2).7, I(2).19, I(2).20, I(2).21, I(2).22, I(2).25, I(2).26, I(2).27, I(2).28 und I(2).29 Wirkschwellen von 0,2 mg und weniger.

Prodenia litura *(Ägypt. Baumwollwurm)*, Kontakt-/Fraßwirkung

Fünf Raupen des Entwicklungsstadiums L3 (10 - 12 mm), die keine feststellbare Schädigung im Kontaktversuch erlitten hatten, wurden auf Standardnährboden (3,1 l Wasser, 80 g Agar, 137 g Bierhefe, 515 g Maismehl, 130 g Weizenkeime sowie übliche Zusatzstoffe und Vitamine (20 g Wessonsalz, 5 g Nipagin, 5 g Sorbin, 10 g Zellulose, 18 g Ascorbinsäure, 1 g Lutavit® blend (Vitamin), 5 ml alkoholische Biotin-Lösung)) aufgebracht, der zuvor mit der wässrigen Wirkstoffaufbereitung benetzt worden war. Die Beobachtung erstreckte sich bis zum Schlüpfen der Falter in einem Kontrollversuch ohne Wirkstoff.

In diesem Test zeigten die Verbindungen I(2).2, I(2).16 und I(2).17 Wirkschwellen von 0,2 mg und weniger.

Tetranychus telarius *(Rote Spinne)*, Kontaktwirkung

Stark befallene getopfte Buschbohnen, die das zweite Folgeblattpaar zeigten, wurden mit wässrigen Wirkstoffaufbereitung behandelt. Nach 5 Tagen im Gewächshaus wurde der Bekämpfungserfolg mittels Binokular bestimmt.

In diesem Test zeigten die Verbindungen I(2).2, I(2).6, I(2).7, I(2).8, I(2).9, I(2).13, I(2).14, I(2).15, I(2).16, I (2).17, I(2).19, I(2).20 und I(2).21 Wirkschwellen von 200 ppm und weniger.

**Patentansprüche**

**1.** 2-Iminooxyphenylessigsäurederivate der allgemeinen Formel I

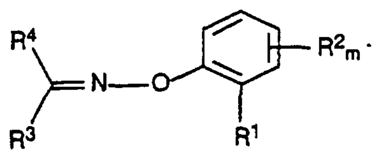

(I)

in der die Substituenten und der Index die folgende Bedeutung haben:

$R^1$ C $(CO_2CH_3)=NOCH_3$ (Ia), C $(CONHCH_3)=NOCH3$ (Ib), $C(CONH_2)=NOCH_3$ (Ic), $C(CO_2CH_3)=CHOCH_3$ (Id) oder $C(CO_2CH_3)=CHCH_3$ (Ie);

$R^2$ Cyano, Nitro, Halogen, $C_1$-$C_4$-Alkyl, $C_1$-$C_4$-Halogenalkyl oder $C_1$-$C_4$-Alkoxy;

m 0, 1 oder 2, wobei die Reste $R^2$ verschieden sein können, wenn m für 2 steht;

R3 Wasserstoff, Cyano, Hydroxy, Halogen,
$C_1$-$C_6$-Alkyl, $C_1$-$C_6$-Halogenalkyl, $C_1$-$C_6$-Alkoxy-$C_1$-$C_6$-alkyl, $C_1$-$C_6$-Alkoxy, $C_1$-$C_6$-Halogenalkoxy, $C_1$-$C_6$-Alkylthio, Cyclopropyl, $C_2$-$C_6$-Alkenyl,
Aryloxy-$C_1$-$C_6$-alkyl, Benzyl oder Benzyloxy, wobei die aromatischen Ringe in diesen Resten einen bis drei der folgenden Gruppen tragen können: Cyano, Nitro, Halogen, $C_1$-$C_6$-Alkyl, $C_1$-$C_6$-Halogenalkyl, $C_1$-$C_6$-Alkoxyl $C_1$-$C_6$-Halogenalkoxy, oder $C(CH_3)=N-Y-R^a$, wobei

$R^a$ $C_1$-$C_6$-Alkyl und

Y Sauerstoff oder Stickstoff, wobei das Stickstoffatom ein Wasserstoffatom oder eine $C_1$-$C_6$-Alkylgruppe trägt;

$R^4$ Wasserstoff, Cyano,

ggf. subst. Alkyl, Alkenyl, Alkinyl, Cycloalkyl, Heterocyclyl, Aryl und Hetaryl;
ggf. subst. Alkoxy, Alkenyloxy, Alkinyloxy, Cycloalkoxy, Heterocyclyloxy, Aryloxy und Hetaryloxy;
ggf. subst. Arylthio und Hetarylthio;

$$-Q_p-C(R^5)=N-Y^1-R^6$$

oder

$$-Q-O-N=CR^7R^8,$$

wobei

Q eine direkte Bindung, $CH_2$, $CH(CH_3)$, $CH(CH_2CH_3)$ oder 1,1-Cyclopropyl bedeutet;

p 0 oder 1 bedeutet;

$Y^1$ Sauerstoff oder Stickstoff, wobei das Stickstoffatom ein Wasserstoffatom oder eine $C_1$-$C_4$-Alkylgruppe trägt;

$R^5$ eine der für $R^3$ genannten Gruppen, oder
ggf. subst. Cycloalkoxy, Heterocyclyloxy, Aryloxy, Hetaryloxy, Arylthio und Hetarylthio;

$R^6$ ggf. subst. $C_1$-$C_{10}$-Alkyl, $C_3$-$C_6$-Cycloalkyl, $C_2$-$C_{10}$-Alkenyl, $C_2$-$C_{10}$-Alkinyl, $C_1$-$C_{10}$-Alkylcarbonyl, $C_2$-$C_{10}$-Alkenylcarbonyl, $C_2$-$C_{10}$-Alkinylcarbonyl oder $C_1$-$C_{10}$-Alkylsulfonyl;
ggf. subst. Aryl, Hetaryl, Arylcarbonyl, Hetarylcarbonyl, Arylsulfonyl oder Hetarylsulfonyl;

$R^7$, $R^8$ Methyl, Ethyl, Phenyl und Benzyl, wobei die aromatischen Ringe einen bis drei der folgenden Substituenten tragen können: Cyano, Nitro, Halogen, $C_1$-$C_6$-Alkyl, $C_1$-$C_6$-Halogenalkyl, $C_1$-$C_6$-Alkoxy und $C_1$-$C_6$-Halogenalkoxy;

$R^3$ und $R^4$ gemeinsam mit dem Kohlenstoffatom, an das sie gebunden sind, ein vier- bis achtgliedriger Ring, welcher neben Kohlenstoffatomen ein oder zwei Sauerstoff- und/ oder Schwefelatome und/oder NH- und/oder N($C_1$-$C_4$-Alkyl)-Gruppen enthalten kann, und dessen Kohlenstoffatome einen der folgenden Substituenten tragen können: Halogen, $C_1$-$C_6$-Alkyl oder $C_1$-$C_4$-Alkoxyimino; wobei

$R^3$ und $R^4$ nicht gleichzeitig über Heteroatome an das Kohlenstoffatom gebunden sind;

sowie deren Salze.

**2.** Verbindungen der Formel I gemäß Anspruch 1, in denen $R^4$ die folgende Bedeutung hat:

Alkyl, Alkenyl, Alkinyl, Alkoxy, Alkenyloxy oder Alkinyloxy, wobei die Kohlenwasserstoffgruppen ihrerseits partiell oder vollständig halogeniert sein können und/oder eine bis drei der folgenden Reste tragen können:

- Cyano, Nitro, Hydroxy, Mercapto, Amino, Carboxyl, Aminocarbonyl, Aminothiocarbonyl,
- $C_1$-$C_6$-Alkylsulfonyl, $C_1$-$C_6$-Alkylsulfoxyl, $C_1$-$C_6$-Alkylcarbonyl, $C_3$-$C_6$-Cycloalkyl, $C_1$-$C_6$-Alkoxy, $C_1$-$C_6$-Halogenalkoxy, $C_1$-$C_6$-Alkoxycarbonyl, $C_1$-$C_6$-Alkylthio, $C_1$-$C_6$-Alkylamino, Di-$C_1$-$C_6$-alkylamino, $C_1$-$C_6$-Alkylaminocarbonyl, Di-$C_1$-$C_6$-alkylaminocarbonyl, $C_1$-$C_6$-Alkylaminothiocarbonyl, Di-$C_1$-$C_6$-alkylaminothiocarbonyl, $C_2$-$C_6$-Alkenyloxy,
- Benzyloxy, Aryl, Aryloxy, Hetaryl und Hetaryloxy, wobei die aromatischen Ringe durch übliche Gruppen substituiert sein können;

Cycloalkyl, Cycloalkoxy, Heterocyclyl oder Heterocyclyloxy, wobei die cyclischen Gruppen ihrerseits partiell oder vollständig halogeniert sein können und/oder eine bis drei der folgenden Reste tragen können:

- Cyano, Nitro, Hydroxy, Mercapto, Amino, Carboxyl, Aminocarbonyl, Aminothiocarbonyl,

- $C_1$-$C_6$-Alkyl, $C_1$-$C_6$-Halogenalkyl, $C_1$-$C_6$-Alkylsulfonyl, $C_1$-$C_6$-Alkylsulfoxyl, $C_1$-$C_6$-Alkylcarbonyl, $C_3$-$C_6$-Cycloalkyl, $C_1$-$C_6$-Alkoxy, $C_1$-$C_6$-Halogenalkoxy, $C_1$-$C_6$-Alkoxycarbonyl, $C_1$-$C_6$-Alkylthio, $C_1$-$C_6$-Alkylamino, Di-$C_1$-$C_6$-alkylamino, $C_1$-$C_6$-Alkylaminocarbonyl, Di-$C_1$-$C_6$-alkylaminocarbonyl, $C_1$-$C_6$-Alkylaminothiocarbonyl, Di-$C_1$-$C_6$-alkylaminothiocarbonyl, $C_2$-$C_6$-Alkenyl, $C_2$-$C_6$-Alkenyloxy,
- Benzyl, Benzyloxy, Aryl, Aryloxy, Hetaryl und Hetaryloxy, wobei die aromatischen Ringe durch übliche Gruppen substituiert sein können;

Aryl, Hetaryl, Aryloxy oder Hetaryloxy, wobei die cyclischen Gruppen ihrerseits partiell oder vollständig halogeniert sein können und/oder eine bis drei der folgenden Reste tragen können:

- Cyano, Nitro, Hydroxy, Mercapto, Amino, Carboxyl, Aminocarbonyl, Aminothiocarbonyl,
- $C_1$-$C_6$-Alkyl, $C_1$-$C_6$-Halogenalkyl, $C_1$-$C_6$-Alkylsulfonyl, $C_1$-$C_6$-Alkylsulfoxyl, $C_1$-$C_6$-Alkylcarbonyl, $C_3$-$C_6$-Cycloalkyl, $C_1$-$C_6$-Alkoxy, $C_1$-$C_6$-Halogenalkoxy, $C_1$-$C_6$-Alkoxycarbonyl, $C_1$-$C_6$-Alkylthio, $C_1$-$C_6$-Alkylamino, Di-$C_1$-$C_6$-alkylamino, $C_1$-$C_6$-Alkylaminocarbonyl, Di-$C_1$-$C_6$-alkylaminocarbonyl, $C_1$-$C_6$-Alkylaminothiocarbonyl, Di-$C_1$-$C_6$-alkylaminothiocarbonyl, $C_2$-$C_6$-Alkenyl, $C_2$-$C_6$-Alkenyloxy,
- Benzyl, Benzyloxy, Aryl, Aryloxy, Hetaryl, Hetaryloxy, wobei die aromatischen Ringe durch übliche Gruppen substituiert sein können,
- $C(=NOR^i)$-$A_n$-$R^{ii}$ oder $NR^{iii}$-CO-D-$R^{iv}$;

A Sauerstoff, Schwefel oder Stickstoff, wobei der Stickstoff Wasserstoff oder $C_1$-$C_6$-Alkyl trägt;

n 0 oder 1;

$R^i$ Wasserstoff, $C_1$-$C_6$-Alkyl, $C_3$-$C_6$-Alkenyl oder $C_3$-$C_6$-Alkinyl;

$R^{ii}$ Wasserstoff oder $C_1$-$C_6$-Alkyl;

D eine direkte Bindung, Sauerstoff oder $NR^b$ ($R^b$ = Wasserstoff, $C_1$-$C_6$-Alkyl, $C_2$-$C_6$-Alkenyl, $C_2$-$C_6$-Alkinyl, $C_3$-$C_6$-Cycloalkyl, $C_3$-$C_6$-Cycloalkenyl, Aryl, Aryl-$C_1$-$C_6$-alkyl, Hetaryl und Hetaryl-$C_1$-$C_6$-alkyl);

$R^{iii}$ Wasserstoff, Hydroxy, $C_1$-$C_6$-Alkyl, $C_2$-$C_6$-Alkenyl, $C_2$-$C_6$-Alkinyl, $C_1$-$C_6$-Alkoxy, $C_2$-$C_6$-Alkenyloxy, $C_2$-$C_6$-Alkinyloxy, $C_1$-$C_6$-Alkoxy-$C_1$-$C_6$-alkyl, $C_1$-$C_6$-Alkoxy-$C_1$-$C_6$-alkoxy und $C_1$-$C_6$-Alkoxycarbonyl;

$R^{iv}$ Wasserstoff, $C_1$-$C_6$-Alkyl, $C_2$-$C_6$-Alkenyl, $C_2$-$C_6$-Alkinyl, $C_3$-$C_6$-Cycloalkyl, $C_3$-$C_6$-Cycloalkenyl, Aryl, Aryl-$C_1$-$C_6$-alkyl, Hetaryl und Hetaryl-$C_1$-$C_6$-alkyl.

**3.** Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

**4.** Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.

**5.** Verfahren zur Bekämpfung von tierischen Schädlingen, **dadurch gekennzeichnet, daß** man die Schädlinge oder die vor ihnen zu schützenden Materialien, Pflanzen, den Boden oder Saatgüter mit einer wirksamen Menge einer Verbindung der allgemeinen Formel I gemäß Anspruch 1 behandelt.

**6.** Verwendung der Verbindungen I gemäß Anspruch 1 zur Herstellung eines zur Bekämpfung von tierischen Schädlingen oder Schadpilzen geeigneten Mittels.

**7.** Verwendung der Verbindungen I gemäß Anspruch 1 zur Bekämpfung von tierischen Schädlingen oder Schadpilzen geeigneten Mittels.

**8.** Verfahren zur Herstellung der Verbindungen I gemäß Anspruch 1, in denen $R^1$ für $C(CO_2CH_3)=NOCH_3$ (Ia), $C(CO_2CH_3)=CHOCH_3$ (Id) oder $C(CO_2CH_3)=CHCH_3$ (Ie) bedeutet, **dadurch gekennzeichnet, daß** man einen Benzoesäureester der Formel II

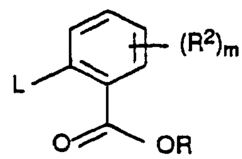

(II)

in der L für eine nucleophil austauschbare Abgangsgruppe und R für eine $C_1$-$C_4$-Alkylgruppe steht, in Gegenwart einer Base mit einem Oxim der Formel III

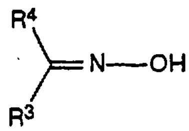

(III)

in das entsprechende Derivat der Formel IV

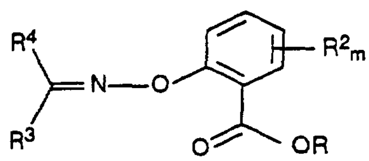

(IV)

überführt, IV zur entsprechenden Carbonsäure IVa

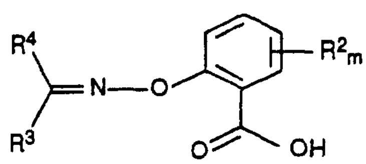

(IVa)

verseift und IVa anschließend zunächst zum Chlorid Va

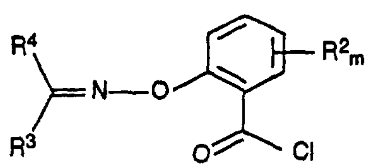

(Va)

und danach zum Cyanid Vb

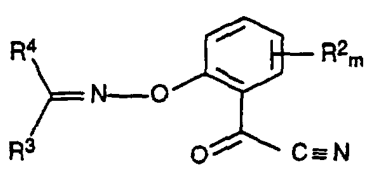

(Vb)

umsetzt, Vb im Wege der Pinner Reaktion in den entsprechenden $\alpha$-Ketoester VI

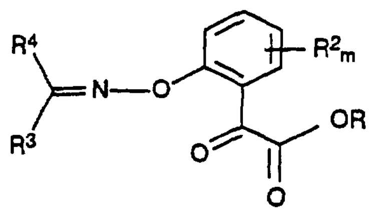

(VI)

überführt und VI anschließend entweder

a) mit O-Methylhydroxylamin oder dessen Salz (VIIa)

$$CH_3\text{-O-}NH_2 \qquad CH_3\text{-O-}NH_3^{\oplus}\,Z^{\ominus} \qquad \text{(VIIa)}$$

in der $Z^-$ für ein Halogenidanion steht, in die entsprechende Verbindung Ia [R = $C(CO_2CH_3)=NOCH_3$]

b) mit einem Wittig- bzw. Wittig-Horner-Reagens der Formel VIIb

$$CH_3\text{-O-}CH_2\text{-}P^* \qquad \text{(VIIb)}$$

in der P* für einen Phosphonat oder einen Phosphonium-halogenid Rest steht, in die entsprechende Verbindung Id [R = $C(CO_2CH_3)=CHOCH_3$], oder

b) mit einem Wittig- bzw. Wittig-Horner-Reagens der Formel VIIc

$$CH_3\text{-}CH_2\text{-}P^* \qquad \text{(VIIc)}$$

in die entsprechende Verbindung Ie [R = $C(CO_2CH_3)=CHCH_3$],

überführt.

**9.** Verfahren zur Herstellung der Verbindungen I gemäß Anspruch 1, in denen $R^1$ für $C(CONHCH_3)=NOCH_3$ (Ib) oder $C(CONH_2)=NOCH_3$ (Ic) steht, **dadurch gekennzeichnet, daß** man eine Verbindung der Formel Ia gemäß Anspruch 8 in an sich bekannter Weise mit Methylamin oder dessen Salz (XIII)

$$CH_3\text{-}NH_2 \qquad CH_3\text{-}NH_3^{\oplus}\,Z^{\ominus} \qquad \text{(XIII)}$$

in der $Z^-$ für ein Halogenidanion steht, in die entsprechende Verbindung Ib [R = $C(CONHCH_3)=NOCH_3$] bzw. mit Ammoniak oder einem Ammonium-Salz in die entsprechende Verbindung Ic [R = $C(CONH_2)=NOCH_3$] überführt.

**10.** Zwischenprodukte der Formel Vb gemäß Anspruch 8.

**11.** Zwischenprodukte der Formel X

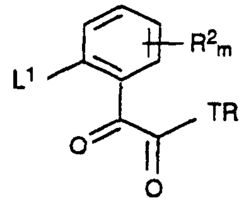

(X)

in der $R^2$ und m die in Anspruch 1 gegebene Bedeutung haben und

T für Sauerstoff oder Stickstoff steht, wobei das Stickstoffatom ein Wasserstoffatom oder eine $C_1$-$C_6$-Alkylgruppe tragen kann;

R für eine $C_1$-$C_4$-Alkylgruppe steht, und

$L^1$ für einen Rest -O-N=$CR^3R^4$ steht, wobei $R^3$ und $R^4$ die in Anspruch 1 gegebene Bedeutung haben.

## Claims

**1.** 2-Iminooxyphenylacetic acid derivatives of the formula I

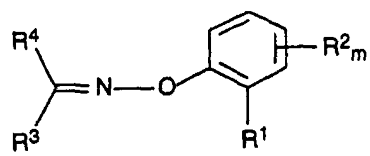

(I)

where the substituents and the index have the following meanings:

$R^1$ is $C(CO_2CH_3)=NOCH_3$ (Ia), $C(CONHCH_3)=NOCH_3$ (Ib), $C(CONH_2)=NOCH_3$ (Ic), $C(CO_2CH_3)=CHOCH_3$ (Id) or $C(CO_2CH_3)=CHCH_3$ (Ie);

$R^2$ is cyano, nitro, halogen, $C_1$-$C_4$-alkyl, $C_1$-$C_4$-haloalkyl or $C_1$-$C_4$-alkoxy;

m is 0, 1 or 2, it being possible for the radicals $R^2$ to be different when m is 2;

$R^3$ is hydrogen, cyano, hydroxyl, halogen,
$C_1$-$C_6$-alkyl, $C_1$-$C_6$-haloalkyl, $C_1$-$C_6$-alkoxy-$C_1$-$C_6$-alkyl, $C_1$-$C_6$-alkoxy, $C_1$-$C_6$-haloalkoxy, $C_1$-$C_6$-alkylthio, cyclopropyl, $C_2$-$C_6$-alkenyl,
aryloxy-$C_1$-$C_6$-alkyl, benzyl or benzyloxy, it being possible for the aromatic rings in these radicals to have attached to them one to three of the following groups: cyano, nitro, halogen, $C_1$-$C_6$-alkyl, $C_1$-$C_6$-haloalkyl, $C_1$-$C_6$-alkoxy, $C_1$-$C_6$-haloalkoxy, or $C(CH_3)=N-Y-R^a$ where

$R^a$ is $C_1$-$C_6$-alkyl and

Y is oxygen or nitrogen, the nitrogen atom having attached to it a hydrogen atom or a $C_1$-$C_6$-alkyl group;

$R^4$ is hydrogen, cyano,
unsubstituted or substituted alkyl, alkenyl, alkynyl, cycloalkyl, heterocyclyl, aryl and hetaryl;
unsubstituted or substituted alkoxy, alkenyloxy, alkynyloxy, cycloalkoxy, heterocyclyloxy, aryloxy and hetaryloxy;
unsubstituted or substituted arylthio and hetarylthio;

$$-Q_p-C(R^5)=N-Y^1-R^6$$

or

$$-Q-O-N=CR^7R^8$$

where

Q is a direct bond, $CH_2$, $CH(CH_3)$, $CH(CH_2CH_3)$ or 1,1-cyclopropyl;

p is 0 or 1;

$Y^1$ is oxygen or nitrogen, the nitrogen atom having attached to it a hydrogen atom or a $C_1$-$C_4$-alkyl group;

$R^5$ is one of the groups mentioned for $R^3$, or
unsubstituted or substituted cycloalkoxy, hetero-cyclyloxy, aryloxy, hetaryloxy, arylthio and hetarylthio;

$R^6$ is unsubstituted or substituted $C_1$-$C_{10}$-alkyl, $C_3$-$C_6$-cycloalkyl, $C_2$-$C_{10}$-alkenyl, $C_2$-$C_{10}$-alkynyl, $C_1$-$C_{10}$-alkylcarbonyl, $C_2$-$C_{10}$-alkenylcarbonyl, $C_2$-$C_{10}$-alkynylcarbonyl or $C_1$-$C_{10}$-alkylsulfonyl;
unsubstituted or substituted aryl, hetaryl, arylcarbonyl, hetarylcarbonyl, arylsulfonyl or hetarylsulfonyl;

$R^7$, $R^8$ are methyl, ethyl, phenyl and benzyl, it being possible for the aromatic rings to have attached to them one to three of the following substituents: cyano, nitro, halogen, $C_1$-$C_6$-alkyl, $C_1$-$C_6$-haloalkyl, $C_1$-$C_6$-alkoxy and $C_1$-$C_6$-haloalkoxy;

$R^3$ and $R^4$ together with the carbon atom to which they are bonded are a four- to eight-membered ring which, besides carbon atoms, may contain one or two oxygen and/or sulfur atoms and/or NH and/or N($C_1$-$C_4$-alkyl) groups and whose carbon atoms can have attached to them one of the following substituents: halogen, $C_1$-$C_6$-alkyl or $C_1$-$C_4$-alkoxyimino;

$R^3$ and $R^4$ not simultaneously being bonded to the carbon atom via hetero atoms;

or a salt thereof.

**2.** A compound of the formula I as claimed in claim 1 where $R^4$ has the following meanings:

alkyl, alkenyl, alkynyl, alkoxy, alkenyloxy or alkynyloxy, it being possible for the hydrocarbon groups, in turn, to be partially or fully halogenated and/or to have attached to them one to three of the following radicals:

- cyano, nitro, hydroxyl, mercapto, amino, carboxyl, aminocarbonyl, aminothiocarbonyl,
- $C_1$-$C_6$-alkylsulfonyl, $C_1$-$C_6$-alkylsulfoxyl, $C_1$-$C_6$-alkylcarbonyl, $C_3$-$C_6$-cycloalkyl, $C_1$-$C_6$-alkoxy, $C_1$-$C_6$-haloalkoxy, $C_1$-$C_6$-alkoxycarbonyl, $C_1$-$C_6$-alkylthio, $C_1$-$C_6$-alkylamino, di-$C_1$-$C_6$-alkylamino, $C_1$-$C_6$-alkylaminocarbonyl, di-$C_1$-$C_6$-alkylaminocarbonyl, $C_1$-$C_6$-alkylaminothiocarbonyl, di-$C_1$-$C_6$-alkylaminothiocarbonyl, $C_2$-$C_6$-alkenyloxy,
- benzyloxy, aryl, aryloxy, hetaryl and hetaryloxy, it being possible for the aromatic rings to be substituted by customary groups;

cycloalkyl, cycloalkoxy, heterocyclyl or heterocyclyloxy, it being possible for the cyclic groups, in turn, to be partially or fully halogenated and/or to have attached to them one to three of the following radicals:

- cyano, nitro, hydroxyl, mercapto, amino, carboxyl, aminocarbonyl, aminothiocarbonyl,
- $C_1$-$C_6$-alkyl, $C_1$-$C_6$-haloalkyl, $C_1$-$C_6$-alkylsulfonyl, $C_1$-$C_6$-alkylsulfoxyl, $C_1$-$C_6$-alkylcarbonyl, $C_3$-$C_6$-cycloalkyl, $C_1$-$C_6$-alkoxy, $C_1$-$C_6$-haloalkoxy, $C_1$-$C_6$-alkoxycarbonyl, $C_1$-$C_6$-alkylthio, $C_1$-$C_6$-alkylamino, di-$C_1$-$C_6$-alkylamino, $C_1$-$C_6$-alkylaminocarbonyl, di-$C_1$-$C_6$-alkylaminocarbonyl, $C_1$-$C_6$-alkylaminothiocarbonyl, di-$C_1$-$C_6$-alkylaminothiocarbonyl, $C_2$-$C_6$-alkenyl, $C_2$-$C_6$-alkenyloxy,
- benzyl, benzyloxy, aryl, aryloxy, hetaryl and hetaryloxy, it being possible for the aromatic rings to be substituted by customary groups; aryl, hetaryl, aryloxy or hetaryloxy, it being possible for the cyclic groups, in turn, to be partially or fully halogenated and/or to have attached to them one to three of the following radicals:
- cyano, nitro, hydroxyl, mercapto, amino, carboxyl, aminocarbonyl, aminothiocarbonyl,
- $C_1$-$C_6$-alkyl, $C_1$-$C_6$-haloalkyl, $C_1$-$C_6$-alkylsulfonyl, $C_1$-$C_6$-alkylsulfoxyl, $C_1$-$C_6$-alkylcarbonyl, $C_3$-$C_6$-cycloalkyl, $C_1$-$C_6$-alkoxy, $C_1$-$C_6$-haloalkoxy, $C_1$-$C_6$-alkoxycarbonyl, $C_1$-$C_6$-alkylthio, $C_1$-$C_6$-alkylamino,

di-$C_1$-$C_6$-alkylamino, $C_1$-$C_6$-alkylaminocarbonyl, di-$C_1$-$C_6$-alkylaminocarbonyl, $C_1$-$C_6$-alkylaminothiocarbonyl, di-$C_1$-$C_6$-alkylaminothiocarbonyl, $C_2$-$C_6$-alkenyl, $C_2$-$C_6$-alkenyloxy,

- benzyl, benzyloxy, aryl, aryloxy, hetaryl, hetaryloxy, it being possible for the aromatic rings to be substituted by customary groups,
- $C(=NOR^i)$-$A_n$-$R^{ii}$ or $NR^{iii}$-CO-D-$R^{iv}$;

A is oxygen, sulfur or nitrogen, the nitrogen having attached to it hydrogen or $C_1$-$C_6$-alkyl;

n is 0 or 1;

$R^i$ is hydrogen, $C_1$-$C_6$-alkyl, $C_3$-$C_6$-alkenyl or $C_3$-$C_6$-alkynyl;

$R^{ii}$ is hydrogen or $C_1$-$C_6$-alkyl;

D is a direct bond, oxygen or $NR^b$ ($R^b$ = hydrogen, $C_1$-$C_6$-alkyl, $C_2$-$C_6$-alkenyl, $C_2$-$C_6$-alkynyl, $C_3$-$C_6$-cycloalkyl, $C_3$-$C_6$-cycloalkenyl, aryl, aryl-$C_1$-$C_6$-alkyl, hetaryl and hetaryl-$C_1$-$C_6$-alkyl);

$R^{iii}$ is hydrogen, hydroxyl, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy, $C_1$-$C_6$-alkoxy-$C_1$-$C_6$-alkyl, $C_1$-$C_6$-alkoxy-$C_1$-$C_6$-alkoxy and $C_1$-$C_6$-alkoxycarbonyl;

$R^{iv}$ is hydrogen, $C_1$-$C_6$-alkyl, $C_2$-$C_6$-alkenyl, $C_2$-$C_6$-alkynyl, $C_3$-$C_6$-cycloalkyl, $C_3$-$C_6$-cycloalkenyl, aryl, aryl-$C_1$-$C_6$-alkyl, hetaryl and hetaryl-$C_1$-$C_6$-alkyl.

**3.** A composition which is suitable for controlling animal pests or harmful fungi, comprising a solid or liquid carrier and a compound of the formula I as claimed in claim 1.

**4.** A method of controlling harmful fungi, which comprises treating the fungi, or the materials, plants, the soil or seed to be protected against fungal infection, with an effective amount of a compound of the formula I as claimed in claim 1.

**5.** A method of controlling animal pests, which comprises treating the pests, or the materials, plants, the soil or seed to be protected against them, with an effective amount of a compound of the formula I as claimed in claim 1.

**6.** The use of a compound I as claimed in claim 1 for the preparation of a composition which is suitable for controlling animal pests or harmful fungi.

**7.** The use of a compound I as claimed in claim 1 for controlling animal pests or harmful fungi.

**8.** A process for the preparation of a compound I as claimed in claim 1 where $R^1$ is $C(CO_2CH_3)=NOCH_3$ (Ia), $C(CO_2CH_3)=CHOCH_3$ (Id) or $C(CO_2CH_3)=CHCH_3$ (Ie), which comprises converting, in the presence of a base, a benzoic ester of the formula II

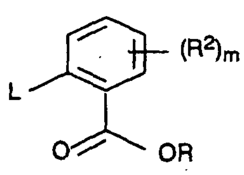

(II)

where L is a nucleophilically exchangeable leaving group and R is a $C_1$-$C_4$-alkyl group, in the presence of a base, with an oxime of the formula III

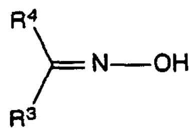

(III)

to give the corresponding derivative of the formula IV

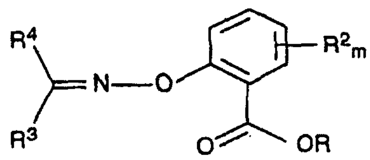

(IV)

hydrolyzing IV to give the corresponding carboxylic acid IVa

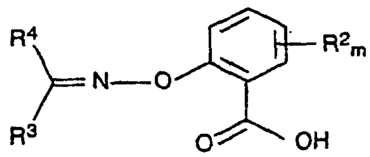

(IVa)

and subsequently first reacting IVa to the chloride Va

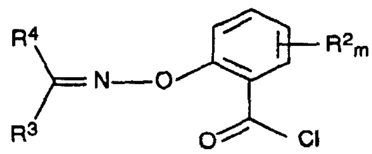

(Va)

and then to the cyanide Vb

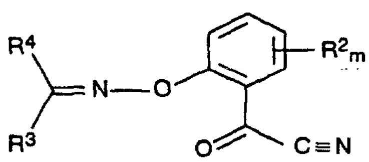

(Vb)

converting Vb, via a Pinner reaction, into the corresponding α-keto ester VI

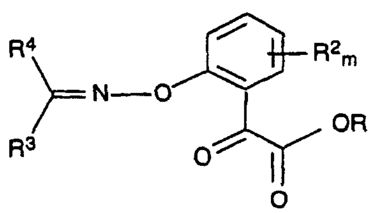

(VI)

and subsequently converting VI either

a) with O-methylhydroxylamine or a salt thereof (VIIa)

$$CH_3\text{-O-}NH_2 \qquad CH_3\text{-O-}NH_3^{\oplus} Z^{\ominus} \qquad \text{(VIIa)}$$

where $Z^-$ is a halide ion,
to give the corresponding compound Ia
[R = $C(CO_2CH_3)=NOCH_3$],

b) with a Wittig, or Wittig-Horner reagent, of the formula VIIb

$$CH_3\text{-O-}CH_2\text{-P*} \qquad \text{(VIIb)}$$

where P* is a phosphonate or a phosphonium halide radical
to give the corresponding compound Id
[R = $C(CO_2CH_3)=CHOCH_3$], or

b) with a Wittig, or Wittig-Horner, reagent of the formula VIIc

$$CH_3\text{-}CH_2\text{-P*} \qquad \text{(VIIc)}$$

to give the corresponding compound Ie [R = $C(CO_2CH_3)=CHCH_3$].

**9.** A process for the preparation of a compound I as claimed in claim 1 where $R^1$ is $C(CONHCH_3)=NOCH_3$ (Ib) or $C(CONH_2)=NOCH_3$ (Ic), which comprises converting a compound of the formula Ia as claimed in claim 8 in a manner known per se with methylamine or a salt thereof (XIII)

$$CH_3\text{-}NH_2 \qquad CH_3\text{-}NH_3^{\oplus} Z^{\ominus} \qquad \text{(XIII)}$$

where $Z^-$ is a halide anion
to give the corresponding compound Ib [R = $C(CONHCH_3)=NOCH_3$], or with ammonia or an ammonium salt to give the corresponding compound Ic [R = $C(CONH_2)=NOCH_3$].

**10.** An intermediate of the formula Vb as claimed in claim 8.

**11.** An intermediate of the formula X

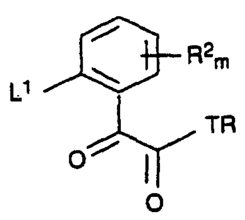

(X)

where $R^2$ and m have the meanings given in claim 1 and

T is an oxygen or nitrogen, it being possible for the nitrogen atom to have attached to it a hydrogen atom or a $C_1$-$C_6$-alkyl group;

R is a $C_1$-$C_4$-alkyl group, and

$L^1$ is a radical $-O-N=CR^3R^4$, where $R^3$ and $R^4$ have the meanings given in claim 1.

## Revendications

**1.** Dérivés de l'acide 2-iminooxyphénylacétique de formule générale I

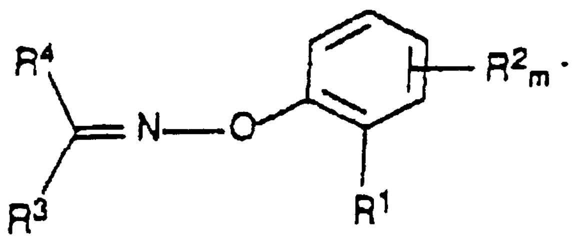

(I)

dans laquelle les substituants et l'indice ont les significations suivantes :

$R^1$ représente $C(CO_2CH_3)=NOCH_3$ (Ia), $C(CONHCH_3)=NOCH_3$ (Ib), $C(CONH_2)=NOCH_3$ (Ic), $C(CO_2CH_3)=CHOCH_3$ (Id) ou $C(CO_2CH_3)=CHCH_3$ (Ie) ;

$R^2$ représente un cyano, un nitro, un halogène, un alkyle en $C_1$-$C_4$, un halogénoalkyle en $C_1$-$C_4$ ou un alcoxy en $C_1$-$C_4$ ;

m vaut 0, 1 ou 2, les radicaux $R^2$ pouvant être différents, lorsque m vaut 2 ;

$R^3$ représente un hydrogène, un cyano, un hydroxy, un halogène,
un alkyle en $C_1$-$C_6$, un halogénoalkyle en $C_1$-$C_6$, un $C_1$-$C_6$-alcoxy-$C_1$-$C_6$-alkyle, un alcoxy en $C_1$-$C_6$, un halogénoalcoxy en $C_1$-$C_6$, un alkylthio en $C_1$-$C_6$, un cyclopropyle, un alcényle en $C_2$-$C_6$,
un aryloxy-$C_1$-$C_6$-alkyle, un benzyle ou un benzyloxy, les noyaux aromatiques dans ces radicaux pouvant porter un à trois des groupes suivants : un cyano, un nitro, un halogène, un alkyle en $C_1$-$C_6$, un halogénoalkyle en $C_1$-$C_6$, un alcoxy en $C_1$-$C_6$, un halogénoalcoxy en $C_1$-$C_6$, ou $C(CH_3)=N-Y-R^a$, où

$R^a$ représente un alkyle en $C_1$-$C_6$ et

Y représente un oxygène ou un azote, l'atome d'azote portant un atome d'hydrogène ou un groupe alkyle en $C_1$-$C_6$ ;

$R^4$ représente un hydrogène, un cyano,
un alkyle, un alcényle, un alcynyle, un cycloalkyle, un hétérocyclyle, un aryle et un hétaryle, éventuellement substitués ;
un alcoxy, un alcényloxy, un alcynyloxy, un cycloalcoxy, un hétérocyclyloxy, un aryloxy et un hétaryloxy, éventuellement substitués ;
un arylthio et un hétarylthio, éventuellement substitués ;

$$-Q_p-C(R^5)=N-Y^1-R^6$$

ou

$$-Q-O-N=CR^7R^8,$$

où

Q représente une liaison directe, $CH_2$, $CH(CH_3)$, $CH(CH_2CH_3)$ ou un 1,1-cyclopropyle ;

p vaut 0 ou 1 ;

$Y^1$ représente un oxygène ou un azote, l'atome d'azote portant un atome d'hydrogène ou un

groupe alkyle en $C_1$-$C_4$ ;

$R^5$ représente l'un des groupes $R^3$ mentionnés, ou un cycloalcoxy, un hétérocyclyloxy, un aryloxy, un hétaryloxy, un arylthio et un étharylthio, éventuellement substitués ;

$R^6$ représente un alkyle en $C_1$-$C_{10}$, un cycloalkyle en $C_3$-$C_6$, un alcényle en $C_2$-$C_{10}$, un alcinyle en $C_2$-$C_{10}$, un alkylcarbonyle en $C_1$-$C_{10}$, un alcénylcarbonyle en $C_2$-$C_{10}$, un alcynylcarbonyle en $C_2$-$C_{10}$ ou un alkylsulfonyle en $C_1$-$C_{10}$, éventuellement substitués ; un aryle, un hétaryle, un arylcarbonyle, un hétarylcarbonyle, un arylsulfonyle ou un hétarylsulfonyle, éventuellement substitués ;

$R^7$, $R^8$ représentent un méthyle, un éthyle, un phényle et un benzyle, les noyaux aromatiques pouvant porter un à trois des substituants suivants : un cyano, un nitro, un halogène, un alkyle en $C_1$-$C_6$, un halogénoalkyle en $C_1$-$C_6$, un alcoxy en $C_1$-$C_6$, et un halogénoalcoxy en $C_1$-$C_6$

$R^3$ et $R^4$ représentent, conjointement avec l'atome de carbone auquel ils sont liés, un noyau à quatre à huit chaînons qui peut renfermer, en plus d'atomes de carbone, un ou deux atomes d'oxygène et/ou de soufre et/ou groupes NH et/ou N($C_1$-$C_4$-alkyle), et dont les atomes de carbone peuvent porter l'un des substituants suivants : un halogène, un alkyle en $C_1$-$C_6$ ou un alcoxyimino en $C_1$-$C_4$ ;

$R^3$ et $R^4$ n'étant pas liés simultanément à l'atome de carbone par l'intermédiaire d'hétéroatomes ;

ainsi que leurs sels.

**2.** Composés de formule I selon la revendication 1, dans lesquels $R^4$ a la signification suivante :

un alkyle, un alcényle, un alcynyle, un alcoxy, un alcényloxy ou un alcynyloxy, les groupes hydrocarbonés pouvant à leur tour être partiellement ou totalement halogénés et/ou pouvant porter un à trois des radicaux suivants :

- un cyano, un nitro, un hydroxy, un mercapto, un amino, un carboxy, un aminocarbonyle, un aminothiocarbonyle,
- un $C_1$-$C_6$-alkylsulfonyle, un $C_1$-$C_6$-alkylsulfoxyle, un $C_1$-$C_6$-alkylcarbonyle, un cycloalkyle en $C_3$-$C_6$, un alcoxy en $C_1$-$C_6$, un halogénoalcoxy en $C_1$-$C_6$, un $C_1$-$C_6$-alcoxycarbonyle, un alkylthio en $C_1$-$C_6$, un alkylamino en $C_1$-$C_6$, un di-$C_1$-$C_6$-alkylamino, un $C_1$-$C_6$-alkylaminocarbonyle, un di-$C_1$-$C_6$-alkylaminocarbonyle, un $C_1$-$C_6$-alkylaminothiocarbonyle, un di-$C_1$-$C_6$-alkylaminothiocarbonyle, un alcényloxy en $C_2$-$C_6$,
- un benzyloxy, un aryle, un aryloxy, un hétaryle et un hétaryloxy, les noyaux aromatiques pouvant être substitués par des groupes habituels ;

un cycloalkyle, un cycloalcoxy, un hétérocyclyle ou un hétérocyclyloxy, les groupes cycliques pouvant à leur tour être partiellement ou totalement halogénés et/ou pouvant porter un à trois des radicaux suivants :

- un cyano, un nitro, un hydroxy, un mercapto, un amino, un carboxy, un aminocarbonyle, un aminothiocarbonyle,
- un alkyle en $C_1$-$C_6$, un halogénoalkyle en $C_1$-$C_6$, un $C_1$-$C_6$-alkylsulfonyle, un $C_1$-$C_6$-alkylsulfoxyle, un $C_1$-$C_6$-alkylcarbonyle, un cycloalkyle en $C_3$-$C_6$, un alcoxy en $C_1$-$C_6$, un halogénoalcoxy en $C_1$-$C_6$, un $C_1$-$C_6$-alcoxycarbonyle, un alkylthio en $C_1$-$C_6$, un alkylamino en $C_1$-$C_6$, un di-$C_1$-$C_6$-alkylamino, un $C_1$-$C_6$-alkylaminocarbonyle, un di-$C_1$-$C_6$-alkylaminocarbonyle, un $C_1$-$C_6$-alkylaminothiocarbonyle, un di-$C_1$-$C_6$-alkylaminothiocarbonyle, un alcényle en $C_2$-$C_6$, un alcényloxy en $C_2$-$C_6$,
- un benzyle, un benzyloxy, un aryle, un aryloxy, un hétaryle et un hétaryloxy, les noyaux aromatiques pouvant être substitués par des groupes habituels ;

un aryle, un hétaryle, un aryloxy ou un hétaryloxy, les groupes cycliques pouvant à leur tour être partiellement ou totalement halogénés et/ou pouvant porter un à trois des radicaux suivants :

- un cyano, un nitro, un hydroxy, un mercapto, un amino, un carboxy, un aminocarbonyle, un aminothiocarbonyle,

- un alkyle en $C_1$-$C_6$, un halogénoalkyle en $C_1$-$C_6$, un $C_1$-$C_6$-alkylsulfonyle, un $C_1$-$C_6$-alkylsulfoxyle, un $C_1$-$C_6$-alkylcarbonyle, un cycloalkyle en $C_3$-$C_6$, un alcoxy en $C_1$-$C_6$, un halogénoalcoxy en $C_1$-$C_6$, un $C_1$-$C_6$-alcoxycarbonyle, un alkylthio en $C_1$-$C_6$, un alkylamino en $C_1$-$C_6$, un di-$C_1$-$C_6$-alkylamino, un $C_1$-$C_6$-alkylaminocarbonyle, un di-$C_1$-$C_6$-alkylaminocarbonyle, un $C_1$-$C_6$-alkylaminothiocarbonyle, un di-$C_1$-$C_6$-alkylaminothiocarbonyle, un alcényle en $C_2$-$C_6$, un alcényloxy en $C_2$-$C_6$,
- un benzyle, un benzyloxy, un aryle, un aryloxy, un hétaryle et un hétaryloxy, les noyaux aromatiques pouvant être substitués par des groupes habituels,

$$- C(=NOR^{i})-A_n-R^{ii}$$

ou

$$Nr^{iii}-CO-D-R^{iv} ;$$

A représente un oxygène, un soufre ou un azote, l'azote portant un hydrogène ou un alkyle en $C_1$-$C_6$ ;

n vaut 0 ou 1 ;

$R^i$ représente un hydrogène, un alkyle en $C_1$-$C_6$, un alcényle en $C_3$-$C_6$ ou un alcynyle en $C_3$-$C_6$ ;

$R^{ii}$ représente un hydrogène ou un alkyle en $C_1$-$C_6$ ;

D représente une liaison directe, un oxygène ou $NR^b$ ($R^b$ = un hydrogène, un alkyle en $C_1$-$C_6$, un alcényle en $C_2$-$C_6$, un alcynyle en $C_2$-$C_6$, un cycloalkyle en $C_3$-$C_6$, un cycloalcényle en $C_3$-$C_6$, un aryle, un aryl-$C_1$-$C_6$-alkyle, un hétaryle et un hétaryl-$C_1$-$C_6$-alkyle) ;

$R^{iii}$ représente un hydrogène, un hydroxy, un alkyle en $C_1$-$C_6$, un alcényle en $C_2$-$C_6$, un alcynyle en $C_2$-$C_6$, un alcoxy en $C_1$-$C_6$, un alcényloxy en $C_2$-$C_6$, un alcynyloxy en $C_2$-$C_6$, un $C_1$-$C_6$-alcoxy-$C_1$-$C_6$-alkyle, un $C_1$-$C_6$-alcoxy-$C_1$-$C_6$-alcoxy et un $C_1$-$C_6$-alcoxycarbonyle

$R^{iv}$ représente un hydrogène, un alkyle en $C_1$-$C_6$, un alcényle en $C_2$-$C_6$, un alcynyle en $C_2$-$C_6$, un cycloalkyle en $C_3$-$C_6$, un cycloalcényle en $C_3$-$C_6$, un aryle, un aryl-$C_1$-$C_6$-alkyle, un hétaryle et un hétaryl-$C_1$-$C_6$-alkyle.

**3.** Composition appropriée pour la lutte contre les parasites animaux ou les champignons nuisibles, comprenant un support solide ou liquide et un composé de formule générale I selon la revendication 1.

**4.** Procédé de contrôle contre les champignons nuisibles, **caractérisé en ce que** les champignons ou les matières, les plantes, les sols ou les semences à protéger vis-à-vis d'une infection fongique sont traités par une quantité efficace d'un composé de formule générale I selon la revendication 1.

**5.** Procédé de lutte contre les parasites animaux, **caractérisé en ce que** les parasites ou les matières, les plantes, les sols ou les semences à protéger vis-à-vis de ceux-ci sont traités par une quantité efficace d'un composé de formule générale I selon la revendication 1.

**6.** Utilisation des composés I selon la revendication 1 pour la préparation d'une composition appropriée pour la lutte contre les parasites animaux ou les champignons nuisibles.

**7.** Utilisation des composés I selon la revendication 1 pour la lutte contre les parasites animaux ou les champignons nuisibles.

**8.** Procédé de préparation des composés I selon la revendication 1, dans lesquels $R^1$ représente $C(CO_2CH_3)=NOCH_3$ (Ia), $C(CO_2-CH_3)=CHOCH_3$ (Id) ou $C(CO_2CH_3)=CHCH_3$ (Ie), **caractérisé en ce qu'**un ester benzoïque de formule II

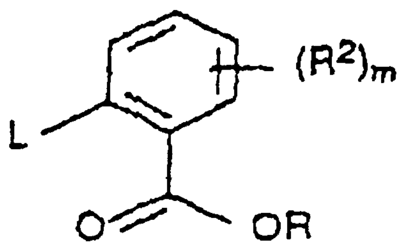

(II)

dans laquelle L désigne un groupe partant échangeable par voie nucléophile et R désigne un groupe alkyle en $C_1$-$C_4$, est transformé en présence d'une base avec un oxim de formule III

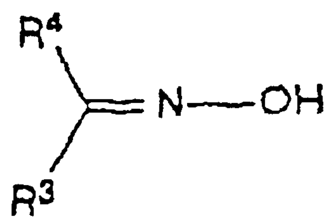

(III)

en le dérivé correspondant de formule IV

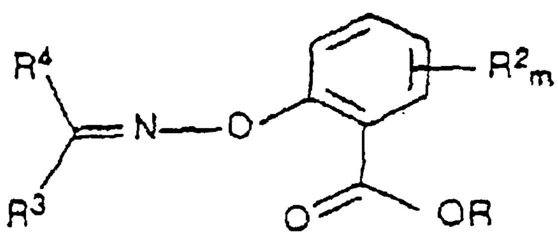

(IV)

IV est hydrolysé en acide carboxylique IVa correspondant

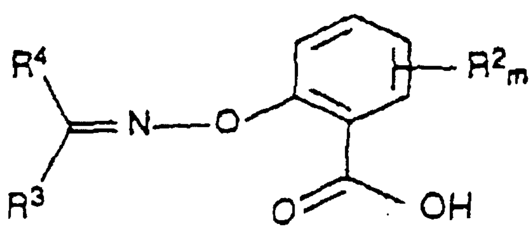

(IVa)

et IVa est ensuite mis à réagir d'abord pour donner lieu au chlorure Va

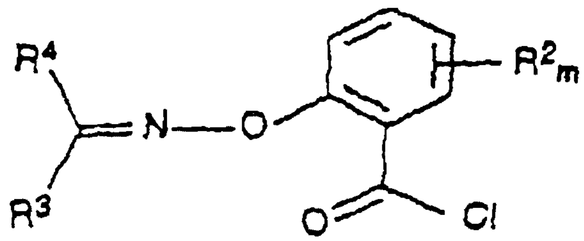

(Va)

et ensuite pour donner lieu au cyanure Vb

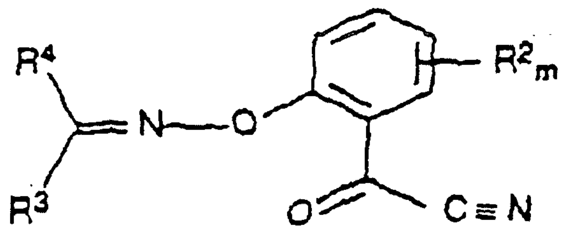

(Vb)

Vb est transformé, par l'intermédiaire de la réaction de Pinner, en l'α-cétoester VI correspondant

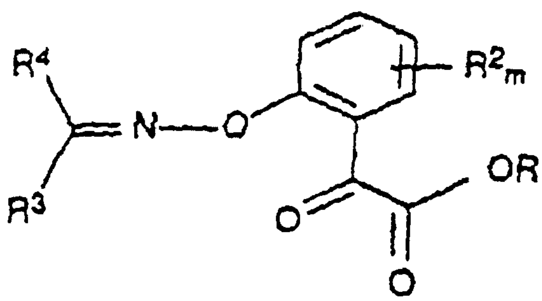

(VI)

et VI est ensuite transformé

a) soit en le composé la correspondant [R=C($CO_2CH_3$)=$NOCH_3$], avec de la O-méthylhydroxylamine ou son sel (VIIa)

$CH_3$-O-$NH_2$ $CH_3$-O-$NH_3^{\oplus} Z^{\ominus}$ (VIIa)

dans laquelle $Z^-$ désigne un anion halogénure,

b) soit en le composé Id correspondant [R=C($CO_2CH_3$)=$CHOCH_3$] avec un réactif de Wittig ou de Wittig-Horner de formule VIIb

$CH_3$-O-$CH_2$-P* (VIIb)

dans laquelle P* désigne un phosphonate ou un radical halogénure de phosphonium,

b) soit en le composé le correspondant [R=C($CO_2CH_3$)=$CHCH_3$] avec un réactif de Wittig ou de Wittig-Horner de formule VIIc

$$CH_3\text{-}CH_2\text{-}P^* \quad \text{(VIIc)}$$

**9.** Procédé de préparation des composés I selon la revendication 1, dans lesquels $R^1$ désigne $C(CONHCH_3)=NOCH_3$ (Ib) ou $C(CONH_2)=NOCH_3$ (Ic), **caractérisé en ce qu'**un composé de formule Ia selon la revendication 8 est transformé, de manière connue en soi, en le composé Ib correspondant $[R=C(CONHCH_3)=NOCH_3]$ avec de la méthylamine ou son sel (XIII)

$$CH_3\text{-}NH_2 \qquad CH_3\text{-}NH_3^{\oplus}Z^- \quad \text{(XIII)}$$

dans lesquels $Z^-$ désigne un anion halogénure, ou en le composé Ic correspondant $[R=C(CONH_2)=NOCH_3]$ avec de l'ammoniac ou un sel d'ammonium.

**10.** Intermédiaires de formule Vb selon la revendication 8.

**11.** Intermédiaires de formule X

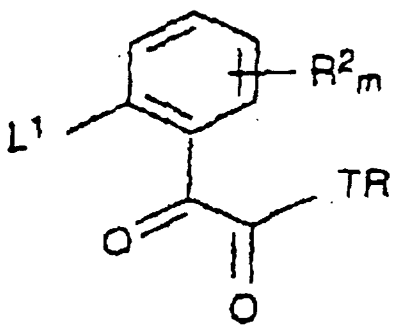

(X)

dans laquelle $R^2$ et m ont la signification indiquée à la revendication 1 et

T désigne un oxygène ou un azote, l'atome d'azote pouvant porter un atome d'hydrogène ou un groupe alkyle en $C_1$-$C_6$ ;

R désigne un groupe alkyle en $C_1$-$C_4$, et

$L^1$ désigne un radical $-O-N=CR^3R^4$, dans lequel $R^3$ et $R^4$ ont la signification indiquée à la revendication 1.